(19) Europäisches Patentamt
European Patent Office
Office européen des brevets

(11) Publication number: **0 451 764 A1**

# (12) EUROPEAN PATENT APPLICATION

(21) Application number: **91105550.7**

(22) Date of filing: **08.04.91**

(51) Int. Cl.5: **C07D 519/00**, C07D 477/00, A61K 31/435, //(C07D519/00, 498:00,477:00),(C07D519/00, 477:00,471:00),(C07D519/00, 495:00,477:00),(C07D519/00, 513:00,477:00)

(30) Priority: **09.04.90 US 506135**

(43) Date of publication of application:
**16.10.91 Bulletin 91/42**

(84) Designated Contracting States:
**AT BE CH DE DK ES FR GB GR IT LI LU NL SE**

(71) Applicant: **F. HOFFMANN-LA ROCHE AG**
**Postfach 3255**
**CH-4002 Basel(CH)**

(72) Inventor: **Dax, Scott Louis**
**47 3rd Street**
**Clifton, N.J. 07011(US)**
Inventor: **Keith, Dennis Dalton**
**8 Mendl Terrace**
**Montclair, N.J. 07042(US)**
Inventor: **Rossman, Pamela Loreen**
**82 Oakley Terrace**
**Nutley, N.J. 07110(US)**

(74) Representative: **Lederer, Franz, Dr. et al**
**Lederer, Keller & Riederer, Patentanwälte,**
**Lucile-Grahn-Strasse 22**
**W-8000 München 80(DE)**

## (54) Carbapenem compounds.

(57) Carbapenem compounds of the general formula

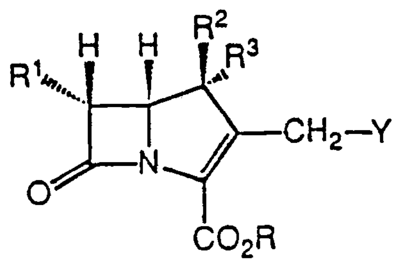

I

wherein R is hydrogen or a carboxylic acid protecting group; $R^1$ is selected from the group consisting of hydrogen, lower alkyl, lower alkoxy and hydroxyalkyl; $R^2$ and $R^3$ are each independently hydrogen, lower alkyl, lower alkoxy or lower alkylthio; and Y is

(i) an ester group;
(ii) a thioester group;
(iii) a piperazinyl or pyrrolidinyl derived carbamate group;
(iv) a piperazinyl derived amine group;
(v) a piperazinyl derived quaternary ammonium group; or
(vi) a group comprising a covalent bond;

EP 0 451 764 A1

the ester group and the thioester group being bonded to a substituted quinolonyl or naphthyridonyl group; the piperazinyl or pyrrolidinyl derived carbamate group, the piperazinyl derived amine group and the piperazinyl derived quaternary ammonium group containing a piperazinyl nucleus or a pyrrolidinyl nucleus having a nitrogen atom bonded directly to a substituted quinolonyl or naphthyridonyl group, the piperazinyl and the pyrrolidinyl nuclei being unsubstituted or substituted with one or more lower alkyl groups; and the covalent bond of the corresponding group being connected to a substituted isothiazolo or isoxazolo quinolonyl or naphthyridonyl group via a nitrogen atom in an isothiazolo or isoxazolo nucleus;
as well as the corresponding readily hydrolyzable esters, pharmaceutically acceptable salts and hydrates of any of the foregoing compounds.

The products have antibacterial activity.

The present invention relates to carbapenem compounds of the general formula

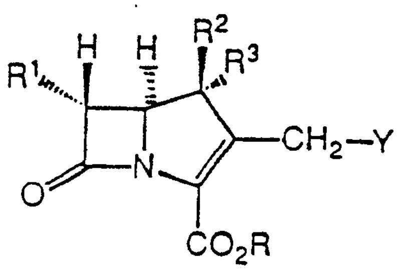

I

wherein R is hydrogen or a carboxylic acid protecting group; $R^1$ is selected from the group consisting of hydrogen, lower alkyl, lower alkoxy and hydroxyalkyl; $R^2$ and $R^3$ are each independently hydrogen, lower alkyl, lower alkoxy or lower alkylthio; and Y is

(i) an ester group;
(ii) a thioester group;
(iii) a piperazinyl or pyrrolidinyl derived carbamate group;
(iv) a piperazinyl derived amine group;
(v) a piperazinyl derived quaternary ammonium group; or
(vi) a group comprising a covalent bond;

the ester group and the thioester group being bonded to a substituted quinolonyl or naphthyridonyl group; the piperazinyl or pyrrolidinyl derived carbamate group, the piperazinyl derived amine group and the piperazinyl derived quaternary ammonium group containing a piperazinyl nucleus or a pyrrolidinyl nucleus having a nitrogen atom bonded directly to a substituted quinolonyl or naphthyridonyl group, the piperazinyl and the pyrrolidinyl nuclei being unsubstituted or substituted with one or more lower alkyl groups; and the covalent bond of the corresponding group being connected to a substituted isothiazolo or isoxazolo quinolonyl or naphthyridonyl group via a nitrogen atom in an isothiazolo or isoxazolo nucleus; as well as the corresponding readily hydrolyzable esters, pharmaceutically acceptable salts and hydrates of any of the foregoing compounds.

The compounds of formula I and the corresponding esters, salts and hydrates are useful as antibacterial compounds in mammalian species. These compounds may be administered orally or parentally and exhibit activity against a broad range of both Gram-negative and Gram-positive bacteria.

Also contemplated within the present invention are the intermediates and processes used to manufacture the compounds, as well as the pharmaceutical compositions.

The term "alkyl" refers to both straight and branched-chain saturated hydrocarbon groups containing 1 to 10 carbon atoms, for example, methyl, ethyl, propyl, isopropyl, tertiary butyl, and the like. The term "lower alkyl" denotes alkyl as above having 1-5 carbon atoms.

As used herein, the term "$C_3$-$C_7$ cycloalkyl" denotes a 3-7 carbon atoms group such as cyclopropyl, cyclobutyl, and the like.

The term "alkenyl" refers to a straight or branched chain alkenyl having 2 to 10 carbon atoms such as ethylenyl, propenyl and the like. The term "lower alkenyl" refers to alkenyl as above having 2-5 carbon atoms.

The term "lower alkylene mono-oxy group of 1-3 carbon atoms" refers to a lower alkylene having a carbon atom replaced by an oxygen group. The term "lower alkylene di-oxy group" refers to a lower alkylene having two carbon atoms replaced by two oxygen groups. Examples include, among others, methyleneoxy, ethyleneoxy, methylenedioxy, ethylenedioxy, etc.

The term "alkoxy" refers to a straight or branched chain alkoxy group containing 1 to 10 carbon atoms. The term "lower alkoxy" refers to an alkoxy group as described above containing 1-5 carbon atoms, for example, ethoxy, propoxy and the like.

The term "amino protecting groups" refers to protecting groups conventionally used to replace an acidic proton of an amino group. Examples of such groups are described in Green, T., Protective Groups in Organic Synthesis, Chapter 7, John Wiley and Sons, Inc. (1981), pp. 218-287, herein incorporated by reference.

The term "carboxylic acid protecting group" refers to protecting groups conventionally used to replace the acidic proton of a carboxylic acid. Examples of such groups are described in Greene, T., Protective Groups in Organic Synthesis, Chapter 5, pp. 152-192 (John Wiley and Sons, Inc. 1981), incorporated herein

by reference.

The term "halogen" or "halo" refers to chloro, fluoro, bromo or iodo.

The term "halo-lower-alkyl" refers to an alkyl having up to 7 carbon atoms and one or more of whose hydrogens is replaced by halogen.

The term "mono-, di- or trihalophenyl" refers to a phenyl group which is substituted with one, two, or three halogens, respectively.

The term "hydroxyalkyl" refers to an alkyl group having a hydrogen atom replaced with a hydroxy group.

The term "substituted quinolonyl" refers generally to a group having the ring structure

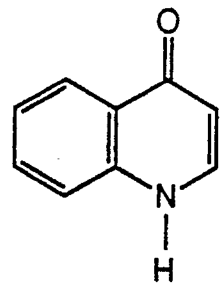

substituted on the rings with hydrogen, halogen, lower alkyl, lower alkenyl, $C_3$-$C_7$ cycloalkyl, halo-lower-alkyl, amino, phenyl, halophenyl, a piperazinyl group, a methyleneoxy ($-CH_2O-$) bridge to the piperazine nucleus to form a fused six-membered ring, a 5- or 6-membered heterocyclic ring containing one or two heteroatoms selected from the group consisting of N, O and S, or two substituents taken together to form a heterocyclic ring containing one or more heteroatoms selected from the group consisting of N, O and S.

Examples of quinolonyl groups used as antibacterials are illustrated in Wolfson, J.S. et al, Quinolone Antimicrobial Agents, American Society for Microbiology (1989), herein incorporated by reference.

The term "substituted naphthyridonyl" refers to a group having the ring structure

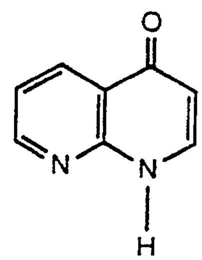

substituted as described above for the term "substituted quinolonyl".

Examples of naphthyridonyl groups used as antibacterials are illustrated in Wolfson, J.S. et al, Quinolone Antimicrobial Agents, American Society for Microbiology (1989), herein incorporated by reference.

The term "readily hydrolysable esters" refers to carboxyl group(s) of formula I which are in the form of readily hydroxyzable ester groups. Examples of such esters include, among others, lower alkanoyloxyalkyl esters, lower alkoxycarbonyloxyalkyl esters, lactonyl esters, lower alkoxymethyl esters and lower alkanoylamino methyl esters.

The term "methyleneoxy ($-CH_2O-$) bridge to the piperazine nucleus to form a fused six-membered ring" refers to a methyleneoxy bridge connecting a ring of the substituted quinolonyl or naphthyridonyl group to a piperazinyl ring of the quinolonyl or naphthyridonyl group.

The term "5- or 6-membered heterocyclic ring containing one or more heteroatoms selected from the group consisting of O, N and S" means a ring having at least one carbon atom, 1-3 nitrogen atoms and optionally one or more S or O atom as ring members. The heterocyclic ring may be substituted at one of its carbon atoms or at a ring nitrogen atom by alkyl, etc. Examples of such rings include, among others, piperazinyl, pyrrolidinyl, morpholinyl, pyridinyl, pyrrolyl, imidazolyl, etc.

The term "piperazinyl or pyrrolidinyl nucleus unsubstituted or substituted with one or more lower alkyl groups" refers to piperazinyl or pyrrolidinyl nucleus having one or more alkyl groups bonded to one or

more carbon or nitrogen atoms in either the piperazinyl or pyrrolidinyl nucleus of the Y linkage groups, such as, for example, N-methylpiperazine, dimethylpiperazine, methylpyrrolidine, dimethylpyrrolidine, etc.

In a preferred embodiment Y is an ester of formula

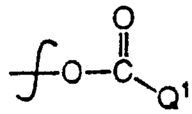

or a thioester of formula

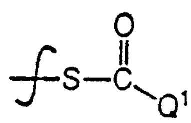

or a piperazinyl or pyrrolidinyl derived carbamate of formula

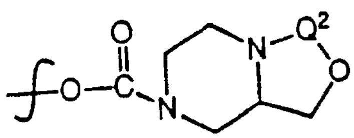

or

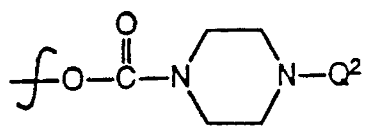

or

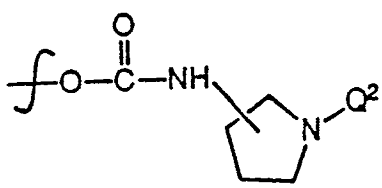

or

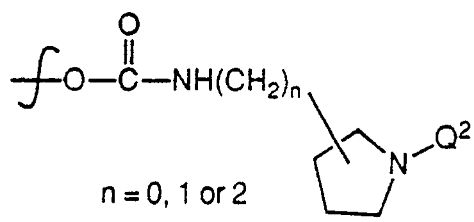

n = 0, 1 or 2

or a piperazinyl derived amine of formula

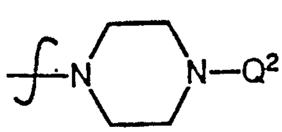

or

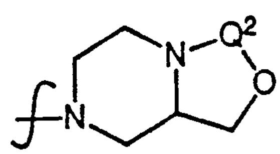

or a piperazinyl derived quaternary ammonium group of formula

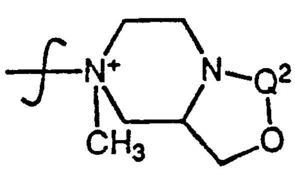

or

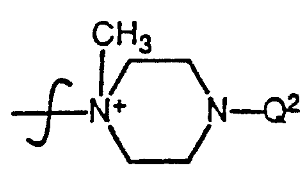

or a group comprising a covalent bond connected to $Q^3$, i.e. a group of formula

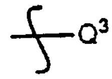

wherein the piperazine or pyrrolidine nucleus may be unsubstituted or substituted with one or more alkyl groups; and wherein $Q^1$, $Q^2$ and $Q^3$ are each independently a substituted quinolonyl group, i.e. having the ring structure

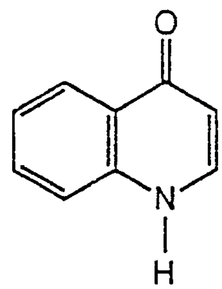

or a substituted naphthyridonyl group, i.e. having the ring structure

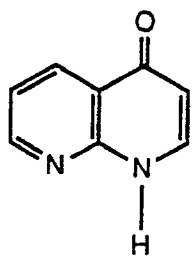

the quinolonyl or naphthyridonyl group being substituted with hydrogen, halogen, lower alkyl, lower alkenyl, $C_3$-$C_7$ cycloalkyl, halo-lower-alkyl, amino, phenyl, mono-, di-, or tri-halophenyl, a methyleneoxy ($-CH_2O-$) bridge to the piperazine nucleus to form a fused six-membered ring, a 5- or 6-membered heterocyclic ring containing one or two heteroatoms selected from a group consisting of O, N and S, or two substituents taken together form a heterocyclic ring containing one or more hetero atoms selected from the group consisting of N, O and S.

$Q^3$ Is preferably a substituted isothiazolo or isoxazolo quinolonyl or naphthyridonyl group.

In especially preferred embodiments Y is of the formula

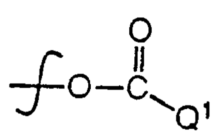

or

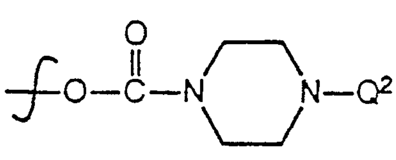

or

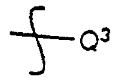

wherein $Q^1$, $Q^2$ and $Q^3$ are as described above.

In compounds of formula I the substituted quinolonyl or naphthyridonyl group of formula $Q^1$ is preferably of formula

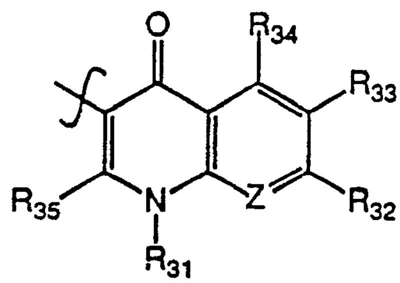

wherein Z represents

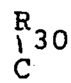

or N;

$R_{30}$ is hydrogen or halogen;

$R_{31}$ is hydrogen, lower alkyl, lower alkenyl, $C_3$-$C_7$ cycloalkyl, halo-lower-alkyl, phenyl, mono-, di- and tri-halophenyl or

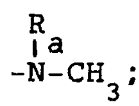

$R_a$ is hydrogen or $R_a$ and $R_{30}$ when taken together represent a lower alkylene mono-oxy group of 1-2 carbon atoms;

$R_{30}$ and $R_{31}$ when taken together represent a lower alkylene mono-oxy group of 2-3 carbon atoms;

$R_{32}$ is hydrogen, halogen, lower alkyl, a 5- or 6-membered heterocyclic ring containing one or two heteroatoms selected from a group consisting of O, N and S;

or when $R_{32}$ is piperazine $R_{30}$ is hydrogen, halogen or a methyleneoxy ($-CH_2O-$) bridge to the piperazine of $R_{32}$ to form a fused six-membered ring;

$R_{33}$ is hydrogen or halogen;

$R_{32}$ and $R_{33}$ when taken together represent a $C_1$-$C_2$ lower alkylene dioxy group;

$R_{34}$ is hydrogen or amino; and

$R_{35}$ is hydrogen, lower alkoxy or lower alkylthio.

Preferred quinolonyl or naphthyridinyl groups of $Q^1$ are those wherein Z is

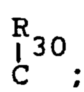

$R_{30}$ is hydrogen or halogen; $R_{31}$ is lower alkyl, $C_3$-$C_7$ cycloalkyl, halo-lower-alkyl, mono-, di- or tri-halophenyl;

$R_{32}$ is a piperazinyl group or a pyrrolidinyl group;

$R_{33}$ is halogen; $R_{34}$ is hydrogen or amino; and $R_{35}$ is hydrogen, lower alkoxy or lower alkylthio.

Especially preferred groups of $Q^1$ are those wherein Z is

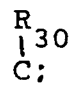

$R_{30}$ is hydrogen, fluorine or chlorine;
$R_{31}$ is ethyl, fluoroethyl, cyclopropyl or fluorophenyl;
$R_{32}$ is a piperazinyl, N-methyl piperazinyl or aminopyrrolidinyl group;
$R_{33}$ is fluoro; $R_{34}$ is hydrogen; and $R_{35}$ is hydrogen.

In compounds of formula I the substituted quinolonyl or naphthyridinyl group of $Q^2$ is of formula

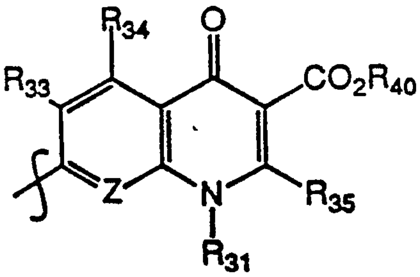

or

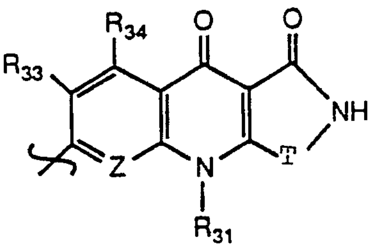

wherein Z represents

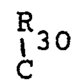

or N;
$R_{30}$ is hydrogen, halogen or a methyleneoxy ($-CH_2O-$) bridge to the piperazine nucleus of Y to form a fused six-membered ring;
$R_{31}$ represents hydrogen, lower alkyl, lower alkenyl, $c_3$ -$c_7$ cycloalkyl, halo-lower-alkyl, phenyl, mono-, di- or tri-halophenyl or

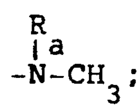

$R_a$ is hydrogen or $R_a$ and $R_{31}$ when taken together represent a lower alkylene mono-oxy group of 1-2 carbon atoms;
$R_{30}$ and $R_{31}$ when taken together represent a lower alkylene mono-oxy group of 2-3 carbon atoms;
$R_{33}$ is hydrogen or halogen;
$R_{34}$ is hydrogen or amino;
$R_{35}$ is hydrogen, lower alkoxy or lower alkylthio;
$R_{40}$ is hydrogen or a carboxylic acid protecting group; and
T is O or S.

Preferred $Q^2$ groups include those in which Z is

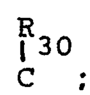

$R_{30}$ is hydrogen or halogen; $R^{31}$ is lower alkyl, $c_3$-$c_7$ cycloalkyl, halo-lower-alkyl, mono-, di- or tri-halophenyl;
$R^{32}$ is a piperazinyl or pyrrolidinyl group;
$R_{33}$ is halogen; $R_{34}$ is hydrogen or amino;
$R_{35}$ is hydrogen; $R_{40}$ is a hydrogen or a carboxylic acid protecting group; and T is O or S.

Especially preferred $Q^2$ groups include those in which Z is

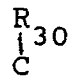 ;

$R_{30}$ is hydrogen, fluoro or chloro;
$R_{31}$ is ethyl, fluoroethyl, cyclopropyl or fluorophenyl;
$R^{32}$ is a piperazinyl group, an N-methyl piperazinyl group or an aminopyrrolidinyl group;
$R_{33}$ is fluoro; $R_{34}$ is hydrogen; $R_{35}$ is hydrogen; $R_{40}$ is hydrogen; and T is O or S.

$Q^3$ in compounds of formula I is of formula

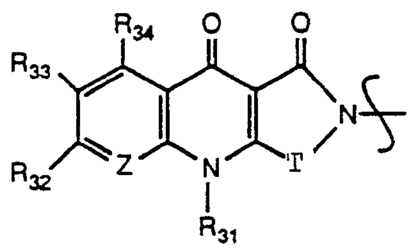

wherein Z represents

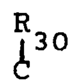

or N;
$R_{30}$ is hydrogen or halogen;
$R_{31}$ is hydrogen, lower alkyl, lower alkenyl, $C_3$-$C_7$ cycloalkyl, halo-lower-alkyl, phenyl, mono-, di-, or tri-halophenyl, or

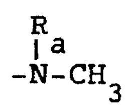 ;

$R_a$ and $R_{30}$ when taken together represent a lower alkylene mono-oxy group of 1-2 carbon atoms;
$R_{30}$ and $R_{31}$ when taken together represent a lower alkylene mono-oxy group of 2-3 carbon atoms;
$R_{32}$ represents hydrogen, halogen, lower alkyl, a 5- or 6-membered heterocyclic ring containing one or two heteroatoms selected from the group consisting of O, N and S; when $R_{32}$ is a piperazinyl group, $R_{30}$ is hydrogen, halogen or a methyleneoxy ($-CH_2O-$) bridge to a piperazinyl nucleus of $R_{32}$ to form a fused six-membered ring;
$R_{33}$ represents hydrogen and halogen;
$R_{32}$ and $R_{33}$ when taken together represent a lower alkylene dioxy group having 1-2 carbon atoms;
$R_{34}$ is hydrogen or amino; and T is O or S.

Preferred $Q^3$ groups include those in which Z is

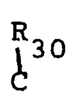 ;

$R_{30}$ is hydrogen or halogen; $R_{31}$ is lower alkyl, $C_3$-$C_7$ cycloalkyl, halo-lower-alkyl, mono-, di- or tri-halophenyl;
$R_{32}$ is a piperazinyl or a pyrrolidinyl group;

$R_{33}$ is halogen; $R_{34}$ is hydrogen; and
T is O or S.

Especially preferred $Q^3$ groups include those in which Z is

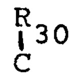

wherein $R_{30}$ is hydrogen, fluoro or chloro; $R_{31}$ is ethyl, fluoroethyl, cyclopropyl or fluorophenyl; $R_{32}$ is a piperazinyl, N-methyl-piperazinyl, or amino pyrrolidinyl group;
$R_{33}$ is fluoro; $R_{34}$ is hydrogen; and T is O or S.

The following compounds, among others, are included in the quinolonyl or naphthyridonyl groups of $Q^1$:

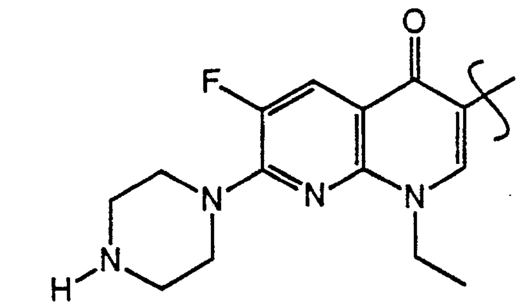

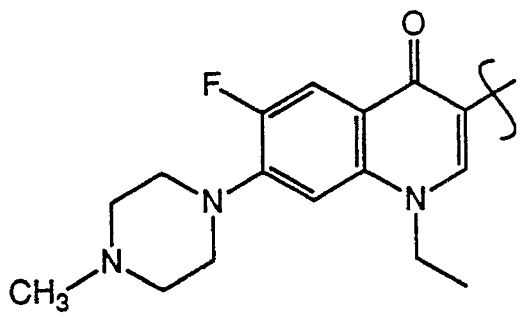

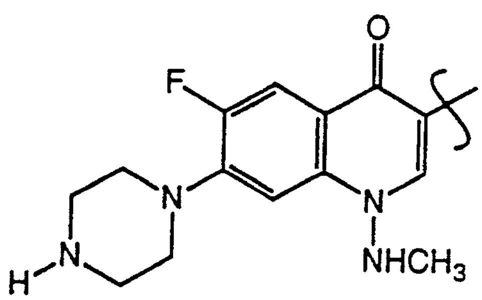

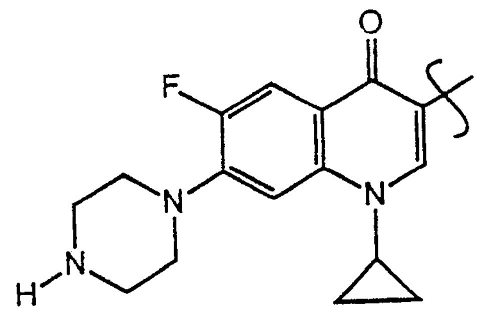

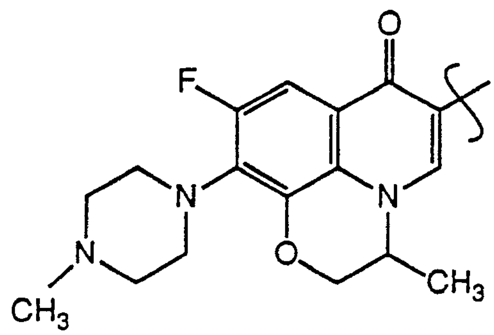

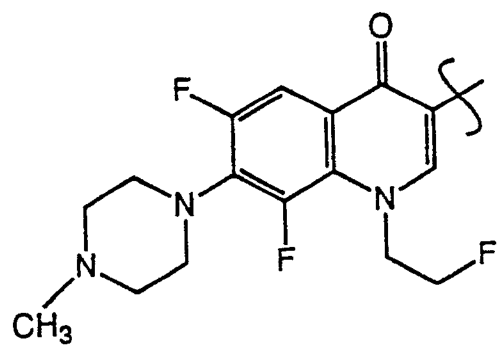

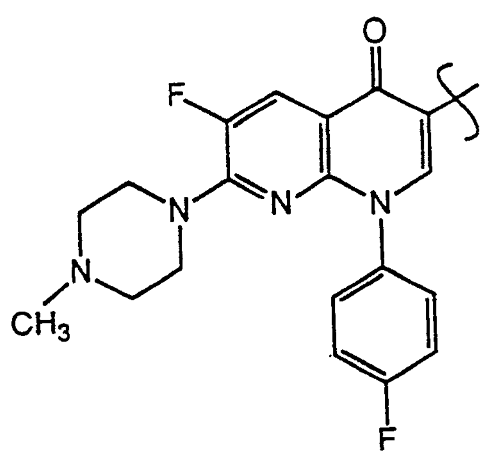

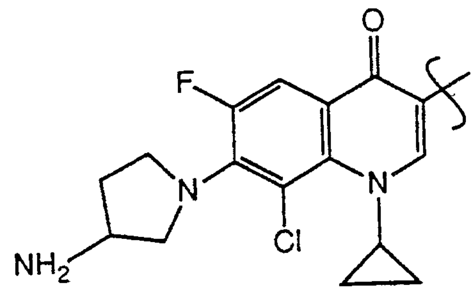

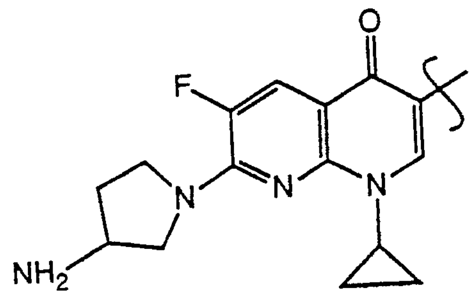

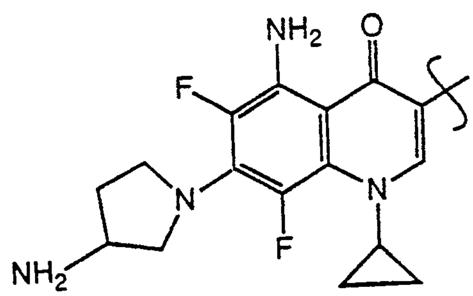

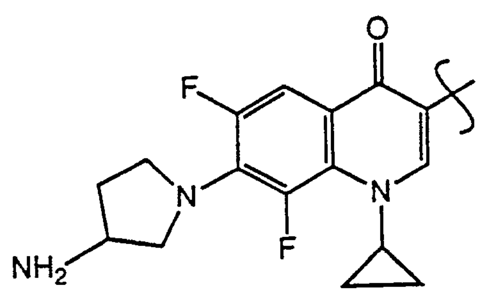

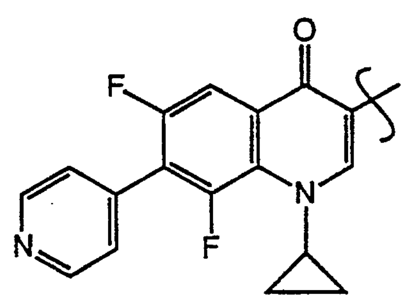

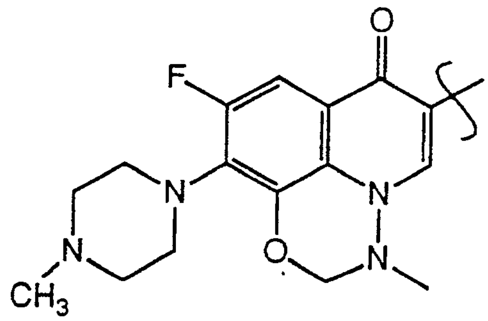

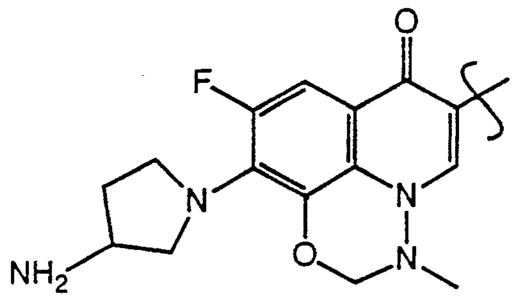

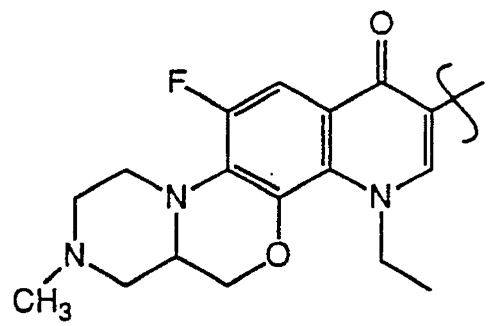

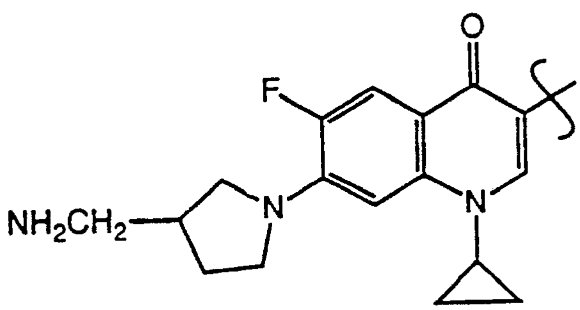

The following compounds, among others, are included in the quinolonyl or naphthyridonyl groups of $Q^2$:

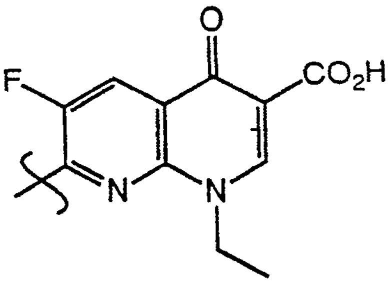

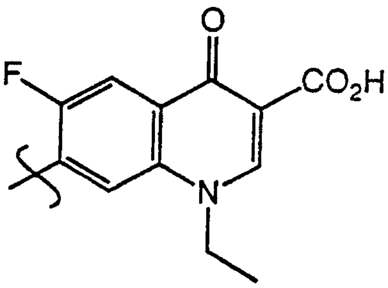

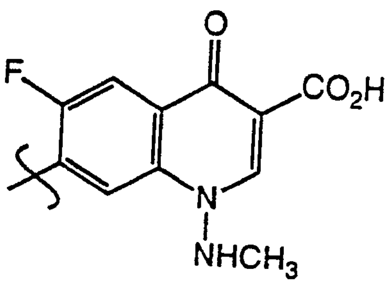

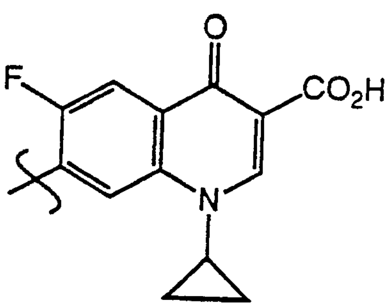

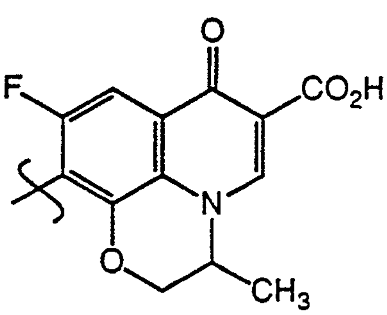

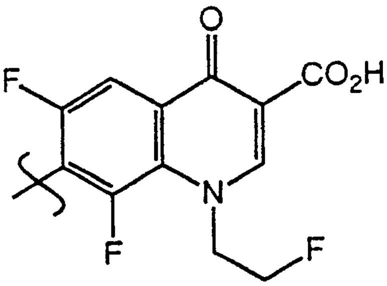

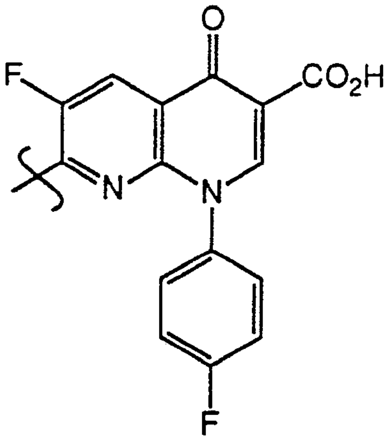

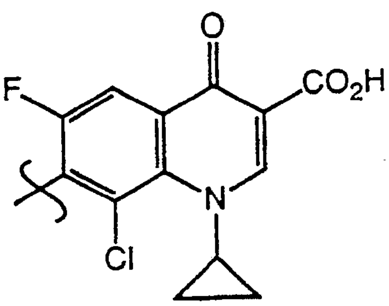

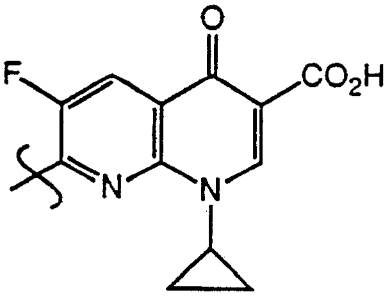

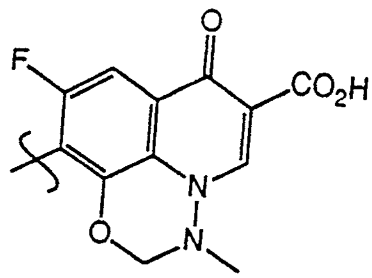

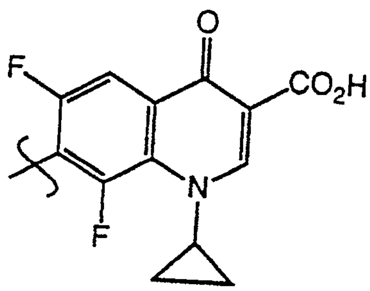

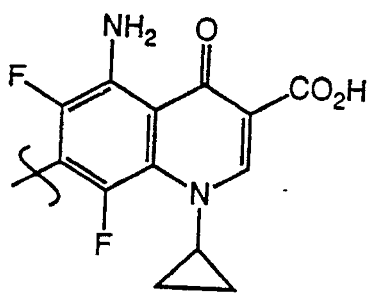

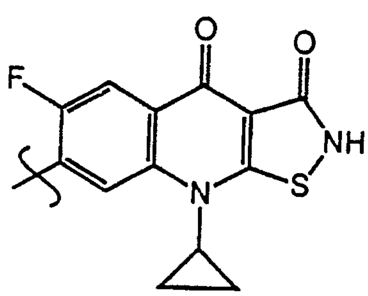

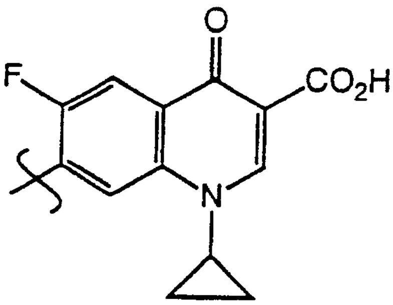

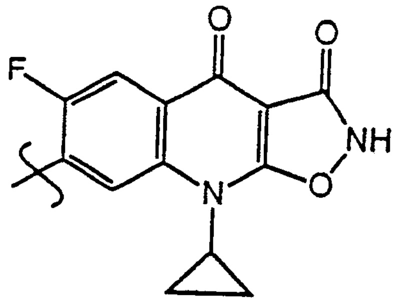

The following compounds, among others, are included in the quinolonyl or naphthyridonyl groups of $Q^3$:

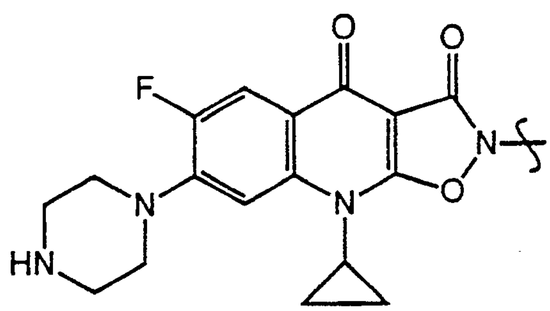

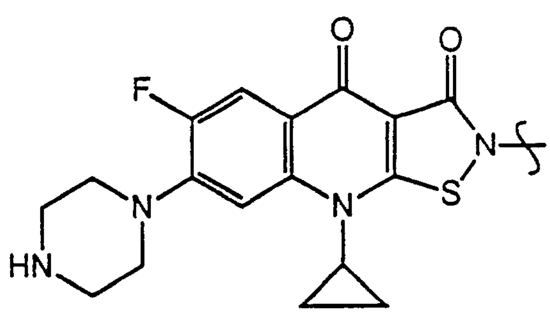

$R^1$ in compounds of formula I are hydrogen, lower alkyl lower alkoxy or hydroxyalkyl; and $R^2$ and $R^3$ are each independently hydrogen, lower alkyl, lower alkoxy or lower alkylthio.

Preferably $R^1$ represents hydroxyalkyl or lower alkyl; and more preferably hydroxyalkyl.

Preferably $R^2$ and $R^3$ are each independently hydrogen or lower alkyl. More preferably, $R^2$ and $R^3$ are each hydrogen, or $R^2$ is lower alkyl and $R^3$ is hydrogen.

Preferred compounds of the present invention are those of formula

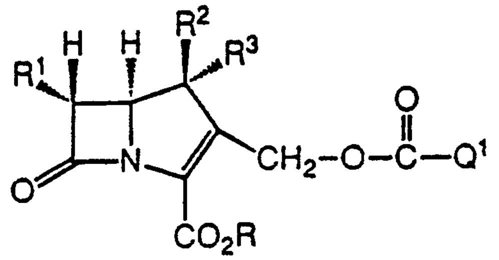

IA

wherein R, $R^1$, $R^2$, $R^3$ and $Q^1$ are as described above,
as well as corresponding readily hydrolyzable esters, pharmaceutically acceptable salts and hydrates of the compounds.

More preferably, compounds of formula 1A include such where R is hydrogen, $R^1$ is hydroxyalkyl, $R^2$ and $R^3$ each independently represent hydrogen or lower alkyl and $Q^1$ is formula

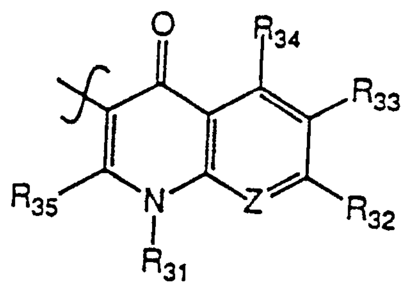

wherein Z represents

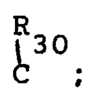

$R_{30}$ is hydrogen or halogen; $R_{31}$ is lower alkyl, $c_3$-$c_7$ cycloalkyl, mono-, di- or tri-halo- phenyl or halo-lower-alkyl; $R_{32}$ is a piperazinyl group or a pyrrolidinyl group; $R_{33}$ is halogen; $R_{34}$ is hydrogen or amino; and $R_{35}$ is hydrogen.

An especially preferred compound of formula IA is:
rac-[5R,6S(R)]-3-[[[[6,8-Difluoro-1-(2-fluoroethyl)-1,4-dihydro-4-oxo-7-(4-methyl-1-piperazinyl-3-quinolonyl]-car-bonyl]oxy]methyl]-6-(1-hydroxyethyl)-7-oxo-1-azabicyclo-[3.2.0]hept-2-ene-2-carboxylic acid.

Other preferable compounds of formula I include compounds of formula

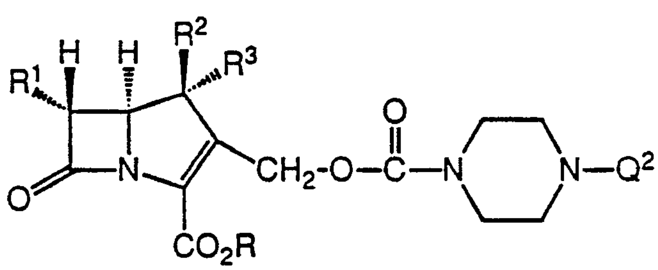

IB

wherein R, $R^1$, $R^2$, $R^3$ and $Q^2$ are as described above.

Preferred compounds of formula IB are those in which R is hydrogen or a carboxylic acid protecting

group; $R^1$ is selected from the group consisting of hydrogen, lower alkyl, lower alkoxy and hydroxyalkyl; $R^2$ and $R^3$ are each independently hydrogen, lower alkyl, lower alkoxy or lower alkylthio and $Q^2$ is of formula

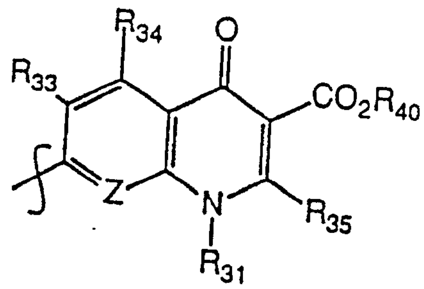

or

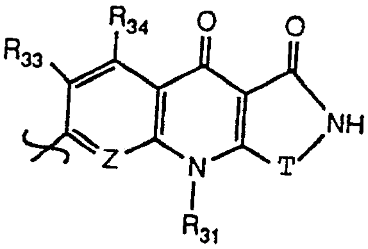

wherein Z represents

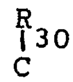

or N;

$R_{30}$ is hydrogen, halogen or a methyleneoxy ($-CH_2O-$) bridge to the piperazine nucleus of Y to form a fused six-membered ring;

$R_{31}$ represents hydrogen, lower alkyl, lower alkenyl, $c_3$-$c_7$ cycloalkyl, halo-lower-alkyl, phenyl, mono-, di-and tri-halophenyl or

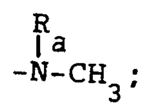

$R_a$ is hydrogen or $R_a$ and $R_{31}$ when taken together represent a lower alkylene mono-oxy group of 1-2 carbon atoms;

$R_{30}$ and $R_{31}$ when taken together represent a lower alkylene mono-oxy group of 2-3 carbon atoms;

$R_{33}$ is hydrogen or halogen;

$R_{34}$ is hydrogen or amino;

$R_{35}$ is hydrogen, lower alkoxy or lower alkylthio;

$R_{40}$ is hydrogen or a carboxylic acid protecting group; and

T is O or S.

An especially preferred compound of formula IB is:

rac-[5R,6S(R)]-3-[[[[(3-carboxylate-1-cyclopropyl-6-fluoro-1,4-dihydro-4-oxo-7-quinolonyl)-1-piperazinyl]-carbonyl]oxy]-methyl]-6-(1-hydroxyethyl)-7-oxo-1-azabicyclo[3.2.O]hept-2-ene-2-carboxylate disodium salt,

Also contemplated within the scope of the invention are compounds of formula

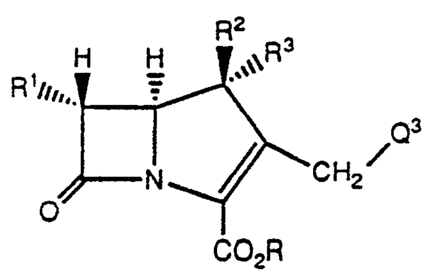

IC

wherein R, $R^1$, $R^2$, $R^3$ and $Q^3$ are as described above.

Preferable compounds of formula IC are those compounds in which R is hydrogen, $R^1$ is hydroxyalkyl, $R^2$ and $R^3$ each independently represent hydrogen or lower alkyl and $Q^3$ is of the formula

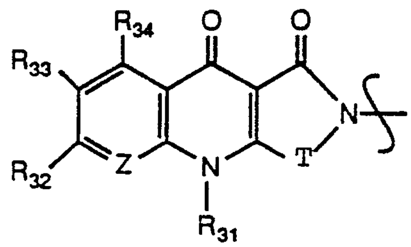

wherein Z represents

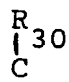 ,

$R_{30}$ is hydrogen or halogen, $R_{31}$ is lower alkyl, $C_3$-$C_7$ cycloalkyl, halo-lower-alkyl or mono-, di- or trihalophenyl, $R_{32}$ represents a piperazinyl or pyrrolidinyl group; $R_{33}$ is halogen; $R_{34}$ is hydrogen and T is O or S.

An especially preferred compound of formula IC includes rac-[4S,5R,6S(R)]-3-[[9-Cyclopropyl-6-fluoro-7-(1-piperazinyl)-2,3,4,9-tetrahydro-3,4-dioxoisothiazolo[5,4-b]quinolin-2-yl]methyl]-6-(1-hydroxyethyl)-4-methyl-7-oxo-1-azabicyclo-[3.2.0] hept-2-ene-2-carboxylic acid.

As readily hydrolyzable esters of the compounds of formula I there are to be understood compounds of formula I, the carboxyl group(s) of which (i.e., the 2-carboxy group) is/are present in the form of readily hydrolyzable ester groups. Examples of such esters, which can be of the conventional type, are the lower alkanoyloxyalkyl esters (e.g., the acetoxymethyl, pivaloyloxymethyl,1-acetoxyethyl and 1-pivaloyloxyethyl ester), the lower alkoxycarbonyl-oxyalkyl esters (e.g., the methoxycarbonyloxymethyl, 1-ethoxycarbonyloxyethyl and 1-isopropoxycarbonyloxyethyl ester), the lactonyl esters (e.g., the phthalidyl and thiophthalidyl ester), the lower alkoxymethyl esters (e.g., the methoxymethyl ester) and the lower alkanoylaminomethyl esters (e.g., the acetamidomethyl ester). Other esters (e.g., the benzyl and cyanomethyl esters) can also be used.

Examples of salts of the compounds of formula I are alkali metal salts such as the sodium and potassium salt, the ammonium salt, alkaline earth metal salts such as calcium salt, salts with organic bases such as salts with amines (e.g., salts with N-ethylpiperidine, procaine, dibenzylamine, N,N'-dibenzylethylenediamine, alkylamines or dialkylamine) as well as salts with amino acids such as, for example, salts with arginine or lysine.

The terms "salts" and "pharmaceutically acceptable salts" as employed throughout this disclosure refer to compounds of formula I which also encompass zwitterions and other types of internal salts in which a carboxy group is negatively charged (i.e. lacks a hydrogen atom) and is neutralized by an accompanying positive charge within the molecule such as the positive charge on the quarternary nitrogen atom.

The compounds of formula I when they contain a basic functional group such as an amine, also form additional salts with organic or inorganic acids. Examples of such salts are hydrohalides (e.g. hydrochlorides, hydrobromides and hydroiodides) as well as other mineral acid salts such as sulphates, nitrates, phosphates and the like, alkylsulphonates and monoarysulphonates such as ethane sulphonates, toluenesulphonates, benzenesulphonates and the like and also other organic acid salts such as acetates, tartrates, maleates, citrates, benzoates, salicylates, ascorbates and the like.

The compounds of Formula I, their pharmaceutically acceptable salts and esters and hydrates of those compounds can be used as agents to combat bacterial infections (including urinary tract infections and respiratory infections) in mammalian species, for example, dogs, cats, horses, etc., and humans. These compounds exhibit activity against a broad range of both Gram-negative and Gram-positive bacteria.

The in vitro activity of the compounds of the present invention as measured by the Minimum Inhibitory Concentration (MIC) in micrograms/ml utilizing the Agar Dilution Method * against a variety of Gram-positive and Gram-negative organisms, is as follows:

Compound A: rac-[5R,6S(R)]-3-[[[[6,8-Difluoro-1-(2-fluoroethyl)-1,4-dihydro-4-oxo-7-(4-methyl-1-piperazinyl)-3-quinolonyl]carbonyl]oxy]methyl]-6-(1-hydroxyethyl)-7-oxo-1-azabicyclo-[3.2.0]hept-2-ene-2-

* See Reiner, R., Antibiotics, An Introduction, p. 23 (F. Hoffmann-La Roche Ltd., 1982)

carboxylic acid monosodium salt.

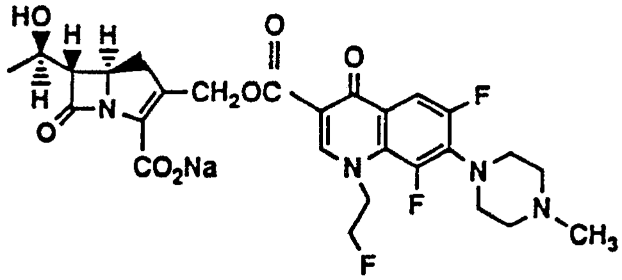

Table 1

In Vitro MIC (mg/ml), Agar Dilution Method

| Culture | Compound A |
|---|---|
| E. coli 257 | 0.125 |
| E. coli ATCC 25922 | 0.125 |
| E. coli TEM-1 | 0.125 |
| Cit. freundii BS-16 | 0.25 |
| K. pneumoniae A | 0.25 |
| Enter. cloacae 5699 | 0.25 |
| Enter. cloacae P99 | 0.125 |
| Ser. marcescens SM | 0.25 |
| Ser. marcescens 1071 | 0.25 |
| Prot. vulgaris ATCC 6380 | 0.25 |
| Prot. vulgaris 1028 BC | 0.25 |
| Prot. mirabilis 90 | 1.00 |
| Ps. aeruginosa ATCC 27853 | 8.0 |
| PS. aeruginosa 5712 | 16.00 |
| Ps. aeruginosa 8780 | 4.00 |
| Ps. aeruginosa 765 | 8.00 |
| Ps. aeruginosa 18SH | 2.00 |
| Staph. aureus Smith | 0.125 |
| Staph. aureus ATCC 29213 | 0.25 |
| Staph. aureus 67 | 1.00 |
| Staph. aureus 753 | 1.00 |
| Str. pneumoniae 6301 | 0.0157 |
| Str. pyogenes 4 | 0.0157 |
| Str. faecalis ATCC 29212 | 2.00 |

For combatting bacterial infections in mammals, a therapeutically active compound of formula I can be administered to a mammal in an amount of about 5 mg/kg/day to about 500 mg/kg/day, preferably about 10 mg/kg/day to 100 mg/kg/day, and most especially about 10 mg/kg/day to about 55 mg/kg/day.

All modes of administration which have been used in the past to deliver penicillins and cephalosporins to the site of the infection are also contemplated for use with this novel family of the compounds of formula I of this invention. Such methods of administration include intravenous, intramuscular and enterally e.g. as a suppository.

The compounds of formula I and their corresponding esters, salts and hydrates can be made according to the invention by a process which process comprises

(a) for the manufacture of a readily hydrolyzable ester of a carboxylic acid of formula I in which Y is one of groups (i), (ii), (iv), (v) and (vi) reacting a compound of the formula

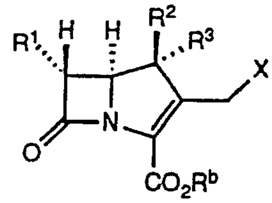

X

wherein $R^1$-$R^3$ are as above, $R^b$ is the residue of a readily hydrolyzable ester, X is hydrogen, and amino, hydroxy and carboxy groups present may be protected;
with a salt of a carboxylic or carbothioic acid bonded to a substituted quinolonyl or naphthyridonyl group;
with a piperazine which is unsubstituted or substituted with one or more lower alkyl groups and has a nitrogen atom bonded directly to a substituted quinolonyl or naphthyridonyl group; or
with a salt of a substituted isothiazolo or isoxazolo quinolone or naphthyridone in which the salt formation occurs at a nitrogen atom in the isothiazolo or isoxazolo nucleus;
followed by cleavage of any protecting group present;
or
(b) for the manufacture of a readily hydrolyzable ester of a carboxylic acid of formula I in which Y is group (iii) reacting a compound of the formula

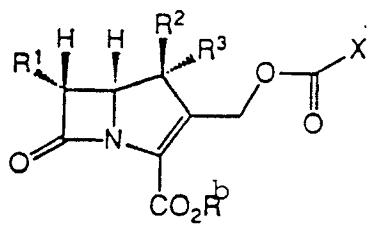

XII

wherein $R^1$-$R^3$, $R^b$ and X are as above, and amino, hydroxy and carboxy groups present may be protected;
with a piperazine or pyrrolidine which is unsubstituted or substituted with one or more lower alkyl groups and has a nitrogen atom bonded directly to a substituted quinolonyl or naphthyridonyl group;
followed by cleavage of any protecting group present;
or
(c) for the manufacture of a readily hydrolyzable ester of a carboxylic acid or formula I in which Y is one of groups (i), (ii) and (iii) subjecting a compound of the formula

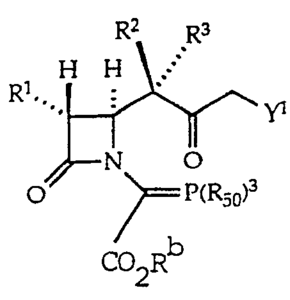

XXII

wherein $R^1$-$R^3$ and $R^b$ are as above, $Y^1$ is an ester or thioester group bonded to a substituted quinolonyl or naphthyridonyl group or a piperazinyl or pyrrolidinyl derived carbamate group which is unsubstituted or substituted with one or more lower alkyl groups and contains a piperazinyl nucleus or a pyrrolidinyl nucleus having a nitrogen atom bonded directly to a substituted quinolonyl or naphthyridonyl group, $R_{50}$ is an aryl or alkyl group and amino, hydroxy and carboxy groups present may be protected,

to thermolysis to effect ring closure;
followed by cleavage of any protecting group present;
or
(d)for the manufacture of a carboxylic acid of formula I converting an ester of the formula

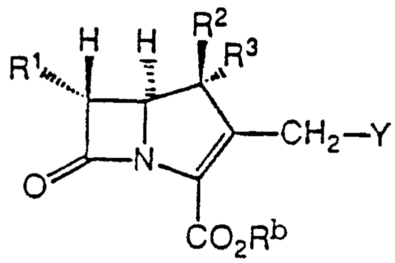

IE

wherein $R^1$-$R^3$ and Y are as above and $R^b$ is a carboxylic acid protecting group,
to the carboxylic acid of formula I,
or
(e) for the manufacture of a readily hydrolyzable ester of a compound of formula I subjecting a carboxylic acid of formula I to a corresponding esterification,
or
(f) for the manufacture of salts or hydrates of a compound of formula I or hydrates of said salts converting a compound of formula I into a salt or hydrate or into a hydrate of said salt.

The following reaction schemes illustrate the above process for producing the compounds of formula I and their corresponding esters, salts and hydrates.

In the following reaction sequences where a substituent group is present which may be attacked during the reaction it should be in protected form utilizing well known protecting groups.

For example, amino groups e.g., on $Q^1$, $Q^2$ or $Q^3$ may be protected with easily removable protective groups employed in peptide chemistry, such as an alkoxycarbonyl group, e.g., t-butoxycarbonyl, etc., a substituted alkoxycarbonyl group, e.g., trichloroethoxycarbonyl, etc., a substituted alkylcarbonyl, e.g., monochloromethylcarbonyl, a substituted aralkyloxycarbonyl group, e.g. p-nitrobenzyloxycarbonyl or triarylalkyl group, such as triphenylmethyl group.

A preferred amino protecting group is tert.-butyloxycarbonyl (t-BOC) or triphenylmethyl.

As carboxylic acid, protecting group (viz. carboxy protecting group or ester protecting group) one may utilize an ester form which can be easily converted into a free carboxy group under mild conditions, the ester protecting group being exemplified by, for example, t-butyl, p-nitrobenzyl, benzhydryl, allyl, etc.

As hydroxy protecting group one may utilize an ester form which can be easily converted into a free hydroxy group under mild conditions, the hydroxy protecting group being exemplified, for example, by 2-trichloroethyl and trimethylsilyl.

Scheme 1

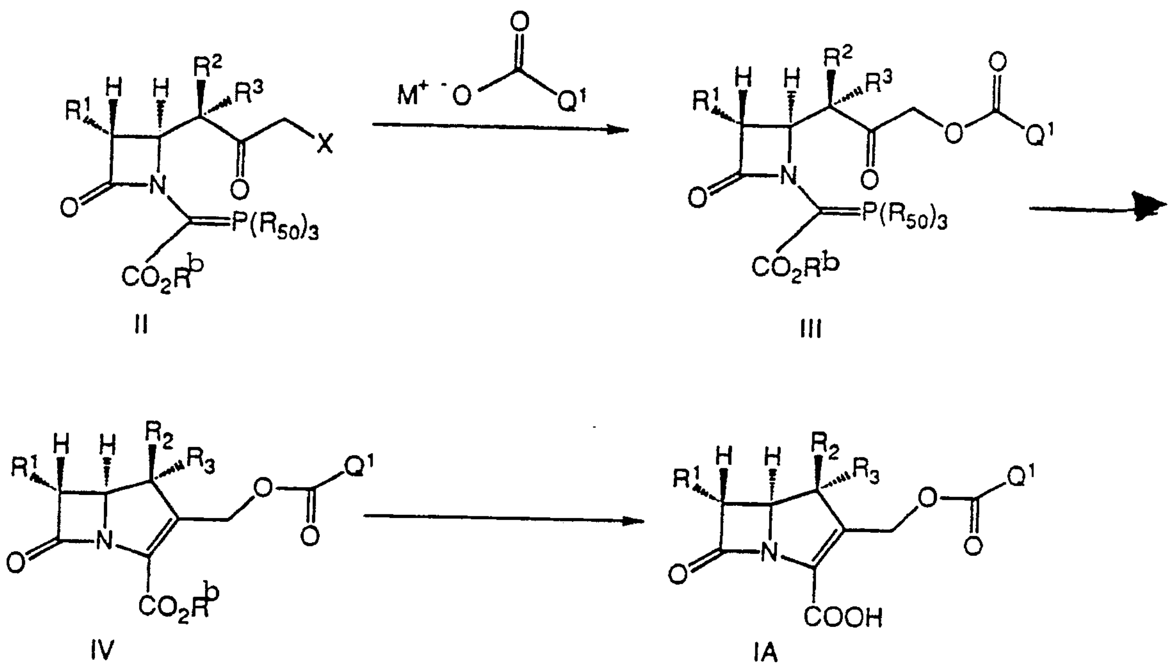

II III

IV IA

wherein $R^b$, $R^1$, $R^2$, $R^3$ and $Q^1$ are as defined above; M is Na, K or Li: X is halogen, such as bromo, iodo, chloro or fluoro; and $R^{50}$ is an aryl or an alkyl group.

Examples of $P(R_{50})_3$ include, among others, triphenylphosphorylidene, tritoluenylphosphorylidene, trimethylphosphorylidene, triethylphosphorylidene and the like. Especially preferred is triphenylphosphorylidene.

Scheme 1

II→III:

The quinolone or corresponding carboxylate salt of formula

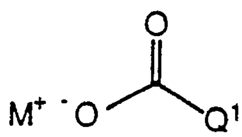

is dissolved in an aprotic organic solvent such as N,N-dimethylformamide, dimethylsulfoxide, acetonitrile or tetrahydrofuran containing a base such as triethylamine, pyridine or diisopropylethylamine. The α-halo-azetidinone of formula II is added and the resultant mixture is stirred under an inert argon atmosphere. The reaction is preferably performed from about 0°C to about 100°C, with about room temperature as particularly preferred, to obtain a compound of formula III.

III→IV:

A suitable compound of the formula III is dissolved in an inert organic solvent such as toluene, benzene or cyclohexane and heated under an inert argon atmosphere. Reaction may occur from approximately 50°C to about 150°C with the preferred temperature of about 100°C to obtain a compound of formula IV.

IV→IA:

A compound of formula IV may contain a carboxylic acid protected as an ester and also an alcohol protected as an ether, silyl ether, carbonate or ester. Protecting group removal may be performed according

to methods known in the art, either sequentially or simultaneously to obtain a compound of formula IA.

Scheme 2

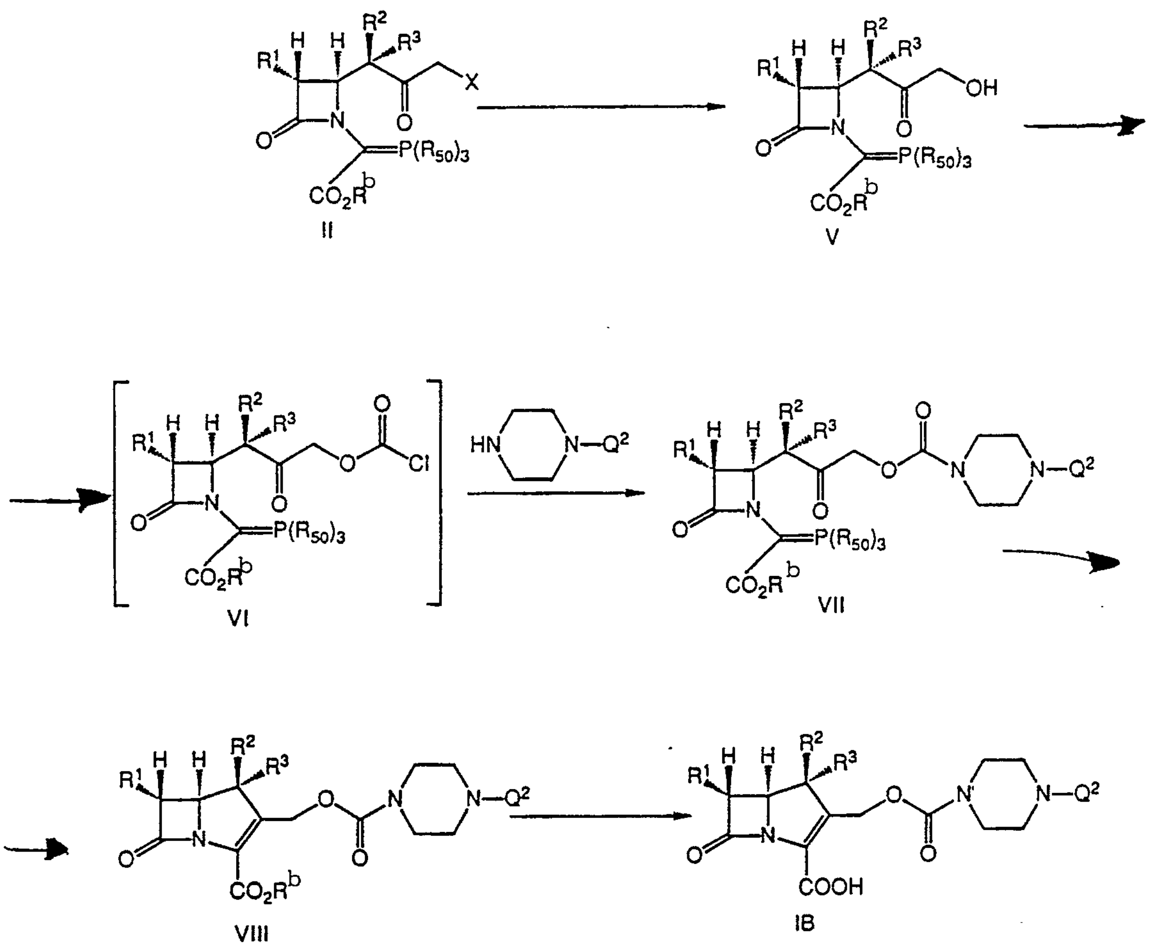

II V VI VII VIII IB

wherein $R^b$, $R^1$, $R^2$, $R^3$, $Q^2$, X and $R_{50}$ are as defined above.

Scheme 2

II→V:

The α-halo-azetidinone of formula II is dissolved in an aprotic organic such as N,N-dimethylformamide, dimethylsulfoxide, acetonitrile or tetrahydrofuran containing a base such as triethylamine, pyridine or diisopropylethylamine. A chloroacetate salt such as the sodium salt of chloroacetic acid is added and the mixture is stirred under an inert argon atmosphere. Reaction is performed from about 0° C to about 100° C with about room temperature as preferred to obtain a product having a corresponding chloroacetate ester.

The isolated ester is dissolved in a solvent such as methanol, ethanol or propanol and cooled to about 0° C to about 5° C. To this solution is added an aqueous bicarbonate solution. The mixture is stirred at about 0° C to about 50° C with the range of about 0° C to about 5° C preferred. The α-hydroxy-azetidinone of formula V is obtained.

V→VI:

A phosgene solution or suitable phosgene equivalent is dissolved in an aprotic organic solvent which is preferably halogenated such as methylene chloride, chloroform or dichloroethane. The α-hydroxy-azetidinone of formula V is added as a solution in a separate but simultaneous manner with an organic base such as N,N-diisopropylethylamine, triethylamine or pyridine. The reaction is performed under an inert

argon atmosphere with the preferred temperature range of about 0° C to about room temperature to obtain a chloroformate of formula VI which may be purified via extraction procedures but is preferably used immediately as follows.

VI→VII:

The chloroformate VI is added to a solution of quinolone of formula

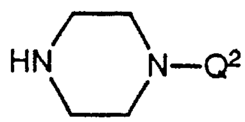

which has been pretreated with a silylating agent in an aprotic organic solvent. The organic solvent is preferably halogenated, such as methylene chloride and the like. The silylating agent is preferably N-methyl-N-(trimethylsilyl)-trifluoroacetamidebut other silylating agents such as (N,O)-bis-(trimethylsilyl) acetamide may be used. The mixture is stirred under an inert argon atmosphere with the preferred temperature of about 0° C to about room temperature to yield compounds of formula VII.

VII→VIII:

A suitable compound of formula VII is dissolved in an inert organic solvent such as toluene, benzene or cyclohexane and heated under an inert argon atmosphere. The reaction may be carried out from about 50° C to about 150° C with the preferred temperature of about 100° C.

VIII→IB:

A compound of formula VIII may contain a carboxylic acid protected as an ester and also an alcohol protected as an ether, silyl ether, carbonate, ester, etc. Protecting group removal may be performed according to methods known in the art either sequentially or simultaneously to obtain the compounds of IB. One can also remove the alcohol protecting group from compound VII.

Scheme 3

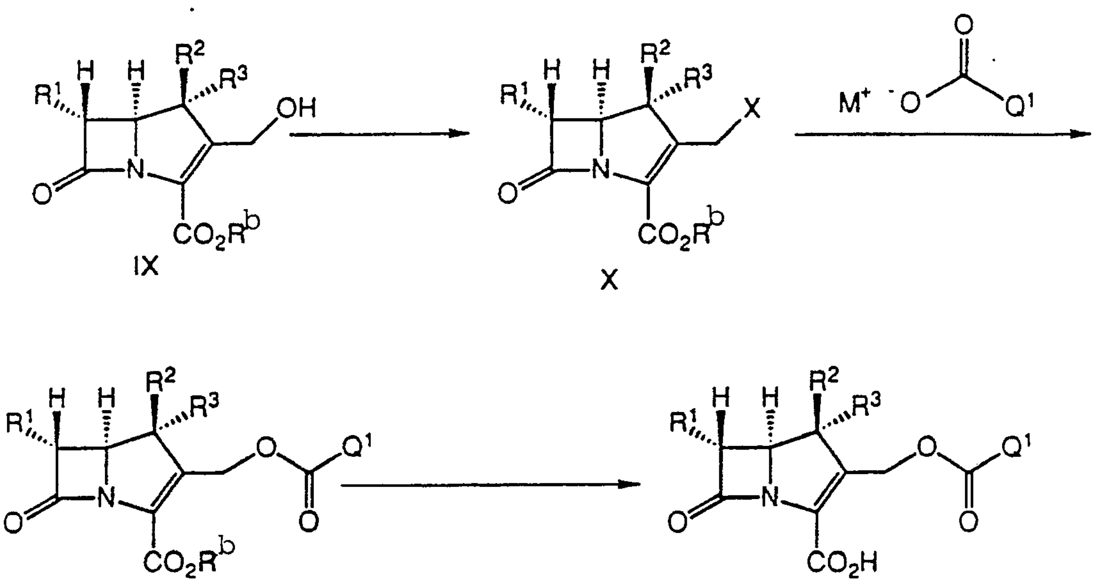

IX X IV IA

wherein $R^b$, $R^1$, $R^2$, $R^3$, $Q^1$, M and $R_{50}$ are as defined above; X is halogen or $OR_L$ where $R_L$ is para-toluenesulfonyl, methanesulfonyl or trifluoromethanesulfonyl.

Scheme 3

IX→X:

A carbapenem alcohol of formula IX is dissolved in an aprotic organic solvent such as methylene chloride, tetrahydrofuran or acetonitrile containing a base such as triethylamine, pyridine or diisopropylethylamine. A halogenating agent such as trimethylsilyl iodide or phosphorous tribromide is added. The resultant mixture is stirred under an inert argon atmosphere at temperatures of between about -20° C to about 100° C to yield the corresponding halogenated carbapenem of formula X.

Alternatively, a sulfonyl halide may be used in place of a halogenating agent to obtain the corresponding carbapenem sulfonate of formula X. Thus, a carbapenem alcohol IX is dissolved in a solvent containing an organic base as described in the above paragraph. Para-toluenesulfonyl chloride, methanesulfonyl chloride or trifluoromethanesulfonyl chloride is added to the reaction and the resultant mixture is stirred under an inert argon atmosphere at temperatures of between about -20° C to about 100° C to yield the corresponding carbapenem sulfonate of formula X.

X→IV:

The quinolone or corresponding carboxylate salt of formula

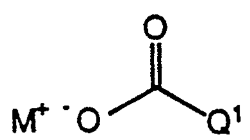

is dissolved in an aprotic organic solvent containing an organic base such as triethylamine, pyridine or diisopropylethylamine. The halogenated carbapenem X or the carbapenem sulfonate X is added and the resultant mixture is stirred under an inert argon atmosphere. Reaction is performed from about 0° C to about 100° C, with about room temperature as preferred to obtain the compound of formula IV.

IV→IA:

A compound of formula IV may contain a carboxylic acid protected as an ester and/or an alcohol protected as an ether, silyl ether, carbonate or ester. Protecting group removal may be performed according to methods known in the art either sequentially or simultaneously to obtain a compound of formula IA.

Scheme 4

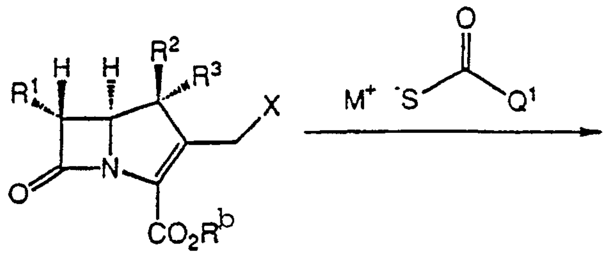

X

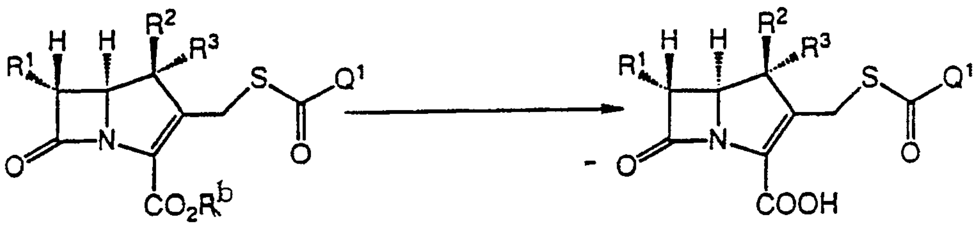

XI ID

wherein $R^b$, $R^1$, $R^2$, $R^3$, $Q^1$, M and $R_{50}$ are as defined above; X is halogen or $OR_L$ wherein $R_L$ is p-toluenesulfonyl, methanesulfonyl, or trifluoromethanesulfonyl.

Scheme 4

X→XI:

The quinolonecarbothioic acid or corresponding thiocarboxylate salt of formula

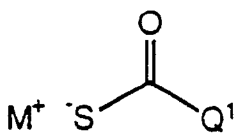

is dissolved in an aprotic organic solvent such as N,N-dimethylformamide, dimethylsulfoxide, acetonitrile, tetrahydrofuran containing a base such as triethylamine, pyridine or diisopropylethylamine. The halogenated carbapenem X or the carbapenem sulfonate X is added and the resultant mixture is stirred under an inert argon atmosphere. Reaction is performed from about 0° C to about 100° C, with about room temperature as preferred to obtain compounds of formula XI.

XI→ID:

As in schemes 1-3 above, a compound of formula XI may contain a carboxylic acid protected as an ester and possibly also an alcohol protected as an ether, silyl ether, carbonate or ester. Protecting group removal may be performed according to methods known in the art either sequentially or simultaneously to obtain compounds of formula ID.

Scheme 5

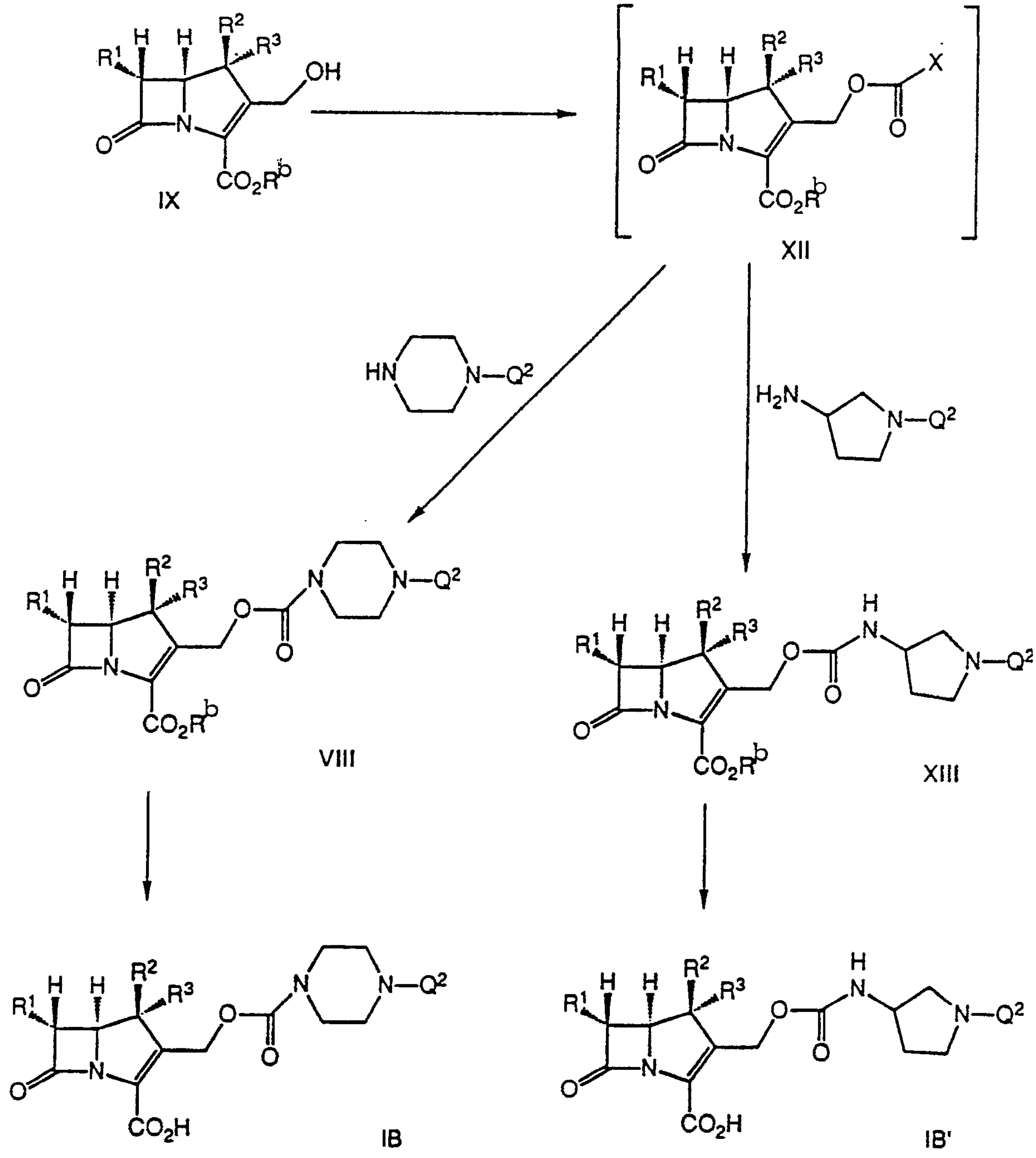

IX

XII

VIII

XIII

IB

IB'

wherein $R^b$, $R^1$, $R^2$, $R^3$, X and $Q^2$ are as defined above.

Scheme 5

IX→XII:

A phosgene solution (or suitable phosgene equivalent) is dissolved in an aprotic organic solvent which is preferably halogenated, such as methylene chloride, chloroform or dichloroethane. Carbapenem alcohol of formula IX, dissolved in the aforementioned solvent, is added in a separate but simultaneous manner with a base such as N,N-diisopropylamine, triethylamine or pyridine. The reaction is performed under an inert argon atmosphere with a preferred temperature range of about 0° C to about room temperature. The resultant chloroformate XII is used immediately as follows:

XII→VIII:

Chloroformate XII is added to a solution of quinolone with a piperazine nucleus of formula

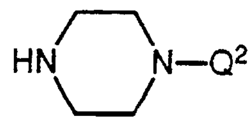

which has been pretreated with a silylating agent in an aprotic organic solvent which is preferably halogenated, such as methylene chloride and the like. The preferred silylating agent is N-methyl-N-(trimethylsilyl)trifluoroacetamide but N,O-bis(trimethylsilyl)acetamide, etc. may be used. The mixture is stirred under an inert argon atmosphere with the preferred temperatures of about 0 °C to about room temperature thus yielding compounds of formula VIII.

<u>VIII→IB:</u>

A compound of formula VIII may contain a carboxylic acid protected as an ester and/or an alcohol protected as an ether, silyl ether, carbonate or ester. Protecting group removal may be performed according to methods known in the art either sequentially or simultaneously to obtain compounds of formula IB.

<u>XII→XIII:</u>

Alternatively, chloroformate XII is added to a solution of quinolone with a pyrrolidine nucleus of formula

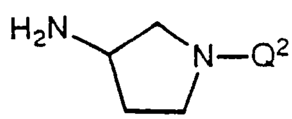

which has been pretreated with a silylating agent in an aprotic organic solvent which is preferably halogenated, such as methylene chloride and the like. The preferred silylating agent is N-methyl-N-(trimethylsilyl)-trifluoroacetamide but N,O-bis(trimethylsilyl)acetamide etc., may be used. The mixture is stirred with an inert argon atmosphere with the preferred temperatures of about 0 °C to about room temperature to yield compounds of formula XIII.

<u>XIII→IB':</u>

A compound of formula XIII may contain a carboxylic acid protected as an ester and/or an alcohol protected as an ether, silyl ether, carbonate or ester. Protecting group removal may be performed according to methods known in the art either sequentially or simultaneously to obtain a compound of formula IB'.

<u>Scheme 6</u>

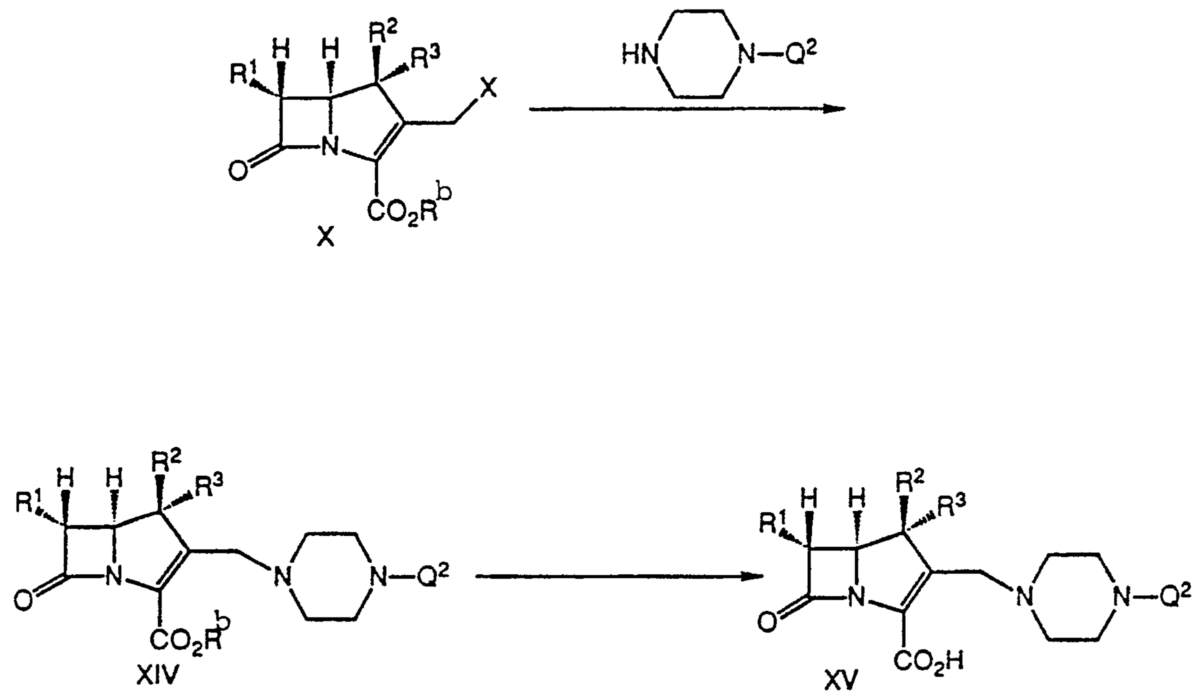

X

XIV

XV

wherein $R^b$, $R^1$, $R^2$, $R^3$ and $Q^2$ are as defined above; X is halogen or $OR_L$ where $R_L$ is p-toluenesulfonyl, methanesulfonyl and trifluoromethanesulfonyl.

Scheme 6

X→XIV:

Halogenated carbapenem X or carbapenem sulfonate X is added to a solution of quinolone of formula

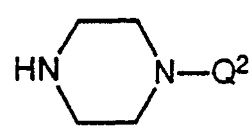

which is pretreated with a silylating agent in an aprotic organic solvent which is preferably halogenated, such as methylene chloride and the like. The preferred silylating agent of choice is N-methyl-N-(trimethysilyl)trifluoroacetamide and (N,O)-bis(trimethylsilyl)acetamide may also be used. The mixture is stirred under an inert argon atmosphere with the preferred temperature of about 0°C to about room temperature thus yielding compounds of formula XIV.

XIV→XV:

A compound of formula XIV may contain a carboxylic acid protected as an ester and/or an alcohol protected as an ether, silyl ether, carbonate or ester, etc. Protecting group removal may be performed according to methods known in the art either sequentially or simultaneously with the process of yielding compounds of XIV to obtain compounds of formula XV.

Scheme 7

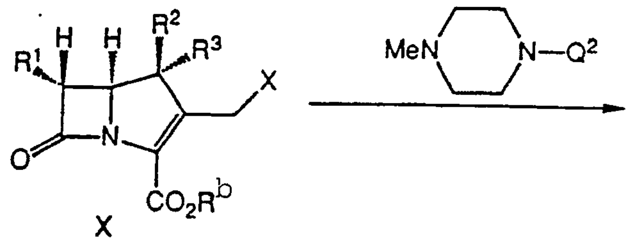

X

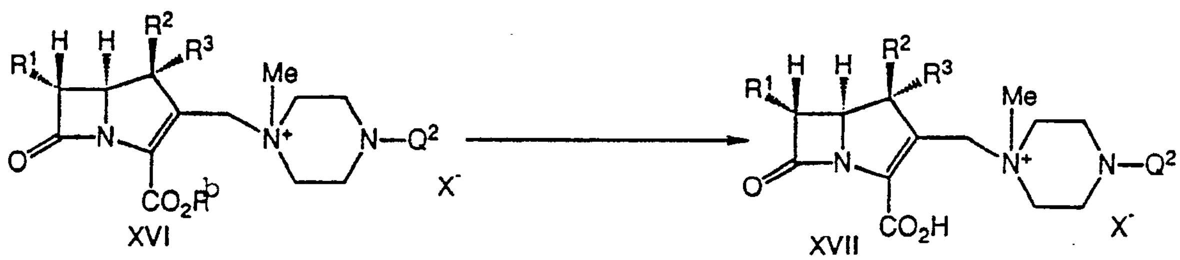

XVI

XVII

wherein $R^b$, $R^1$, $R^2$, $R^3$ and $Q^2$ are as defined above; and X is halogen or $OR_L$ where $R_L$ is $COCH_3$.

Scheme 7

X→XVI:

Carbapenem X is added to a solution of quinolone of formula

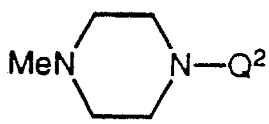

which is pretreated with a silylating agent in an aprotic organic solvent, preferebly a halogenated solvent, such as methylene chloride and the like. The preferred silylating agent is N-methyl-N-(trimethylsilyl)-trifluoroacetamide but (N,O)-bis(trimethylsilyl)acetamide may also be used. The mixture is stirred under an inert argon atmosphere, with the preferred temperature of about 0 °C to about room temperature, thus yielding compounds of formula XVI.

XVI→XVII:

A compound of formula XVII may contain a carboxylic acid protected as an ester and/or an alcohol protected as an ether, silyl ether, carbonate or ester. Protecting group removal may be performed according to methods known in the art either sequentially or simultaneously to yield compounds of XVII.

The following Examples illustrate the present invention but are not intended to limit its extent in any manner. While the examples describe what are at present considered to be the preferred embodiments of this invention, it will be obvious to those skilled in the art that various changes and modifications may be made therein without departing from the invention, and it is, therefore, aimed to cover all such changes and modifications as fall within the true spirit and scope of the invention.

Unless otherwise stated, percentages and ratios relating to solvent mixtures are expressed in volume, purity data determined by liquid chromatography and/or spectroscopic methods and are expressed in area %, and the remaining percentages and ratios are expressed in weight. Temperatures are in degrees celsius (°C), normal pressure is about 1 atmosphere, and room temperature is about 23 °C.

Scheme 8

Step 1

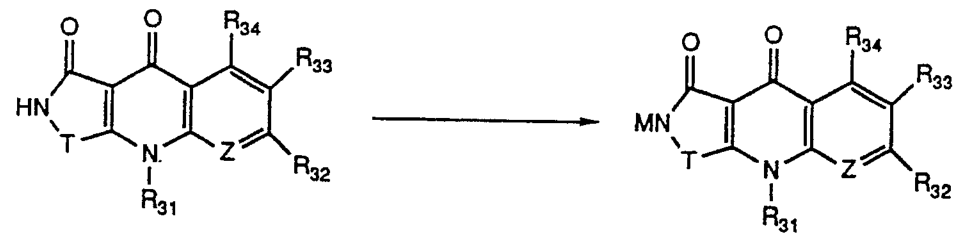

XVIII

XIX

Step 2

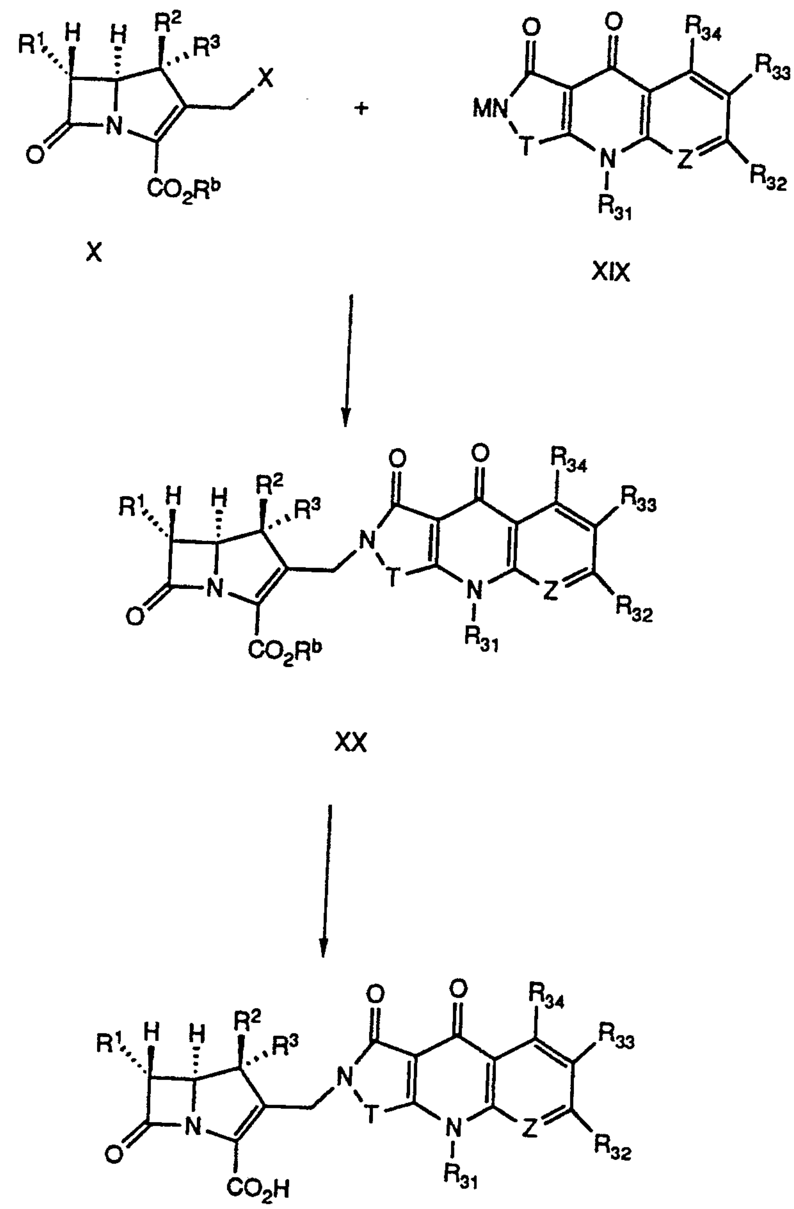

X

XIX

XX

XXI

wherein $R^b$, $R^1$, $R^2$, $R^3$, $R_{31}$, $R_{32}$, $R_{33}$, $R_{34}$, T and X are as defined above, and M is a cation, e.g. Na, K or Li.

Step 1: Compound XVIII is known or made by analogy. See U.S. Patent No. 4,767,762. Compound XVIII

is treated with a salt forming entity, e.g. sodium hydroxide, lithium hydroxide, potassium hydroxide, potassium carbonate, sodium hydride or potassium hydride, so as to form compound XIX. The treatment is carried out in an aqueous or organic solvent, or a mixture thereof. Use of compound XIX as potassium salt rather than sodium salt avoids the formation of the unwanted isomer $\Delta^2$ and forms the desired isomer $\Delta^3$ of Compound XX. Suitable bases include potassium hydroxide and potassium hydride.

Polar organic solvents are preferred e.g., DMF, THF, DMSO, hexamethylphosphoramide, dimethylacetamide, acetonitrile, or mixtures thereof. Step 1 may be performed at ambient temperatures (e.g. at about 23 °C).

Step 2: Compound XIX is reacted with Compound X. The reaction is carried out in a solvent, as described in Step 1, and proceeds readily at ambient temperature or lower. The resulting compound XX, if it contains protecting groups, may be deprotected by employing conventional techniques to form compound XXI.

Example 1

rac-[2R,3S(R)]-2-(3-bromo-2,2-dimethoxypropyl)-3-(1-hydroxyethyl)-4-oxoazetidine-1-(triphenylphosphoranylidene)acetic acid (2,2-dimethyl-1-oxopropoxy)methyl ester

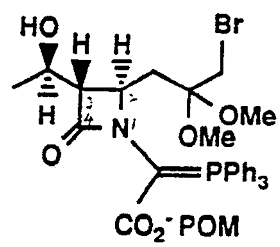

$$POM = CH_2O_2CC(CH_3)_3$$

To a solution of rac-[2R,3S(R)]-2-(3-bromo-2,2-dimethoxypropyl)-3-[1-[[2-trichloroethoxy)carbonyl]oxy]-ethyl]-4-oxoazetidine-1-triphenylphosphoranylidene)acetic acid (2,2-dimethyl-1-oxopropoxy)methyl ester *(7.99 g, 8.84 mmole) in 54 ml of 90% aqueous acetic acid was added zinc dust (18 g) and the mixture was stirred at room temperature for 20 minutes. The reaction was then filtered. The filtrate was extracted with saturated sodium carbonate solution (2 x 270 ml) and with water (2 x 400 ml), and the aqueous layers were back-extracted with methylene chloride (2 x 400 ml). The combined organic layer was dried over sodium sulfate and evaporated in vacuo to yield 6.76 g (100%) of the crude product as a colorless oil.

Example 2

rac-[2R,3S(R)-2-(3-bromo-2-oxopropyl)-3-(1-hydroxyethyl)-4-oxoazetidine-1-(triphenylphosphoranylidene)-acetic acid (2,2-dimethyl-1-oxopropoxy)methyl ester

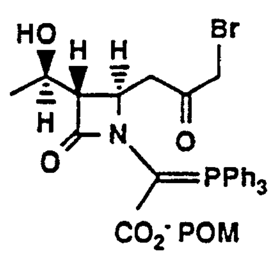

A solution of rac-[2R,3S(R)]-2-(3-bromo-2,2-dimethoxypropyl)-3-(1-hydroxyethyl)-4-oxoazetidine-1-

* - E. Götschi, French Patent FR 2,499,081, British Patent GB 2,092,147 and Canadian Patent 1,117,965

triphenylphosphoranylidene)acetic acid (2,2-dimethyl-1-oxopropoxy)methyl ester (6.76 g, 8.84 mmole) in acetone (230 ml), was cooled to -20° C. With stirring, cooled (-20° C) hydrobromic acid (74 ml of 48% solution in water) was added. The temperature was observed to rise to 12° C during this addition. The mixture was cooled to 0° C within 2 minutes and stirred at this temperature for an additional 20 minutes. The colorless solution was then poured into a mixture of sodium carbonate (52 g), pH 7 buffer (460 ml, 1M in phosphate) and ice (400 g). The mixture was extracted with methylene chloride (2 x 400 ml). The organic phase was washed with pH 7 buffer (300 ml, 1M in phosphate), dried over sodium sulfate and concentrated completely. Chromatography of the crude product on silica gel using ethyl acetate as the eluent, followed by crystallization of the product from ethyl acetate/hexane afforded the title compound as pale yellow crystals (3.13 g, 52%) [m.p. 122 - 123° C (dec.)].

Example 3

6,8-Difluoro-1-(2-fluoroethyl)-1,4-dihydro-7-(4-methyl-1-piperazinyl)-4-oxo-3-quinolinecarboxylate potassium salt

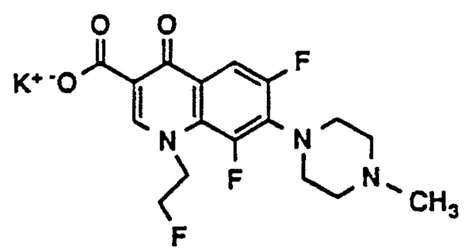

Fleroxacin [6,8-Difluoro-1-(2-fluoroethyl)-1,4-dihydro-7-(4-methyl-1-piperazinyl)-4-oxo-3-quinolinecarboxylic acid] (10 g, 27 mmole) was suspended in water (100 ml). Potassium hydroxide (27 ml of 1.0 N solution, 27 mmole) was added. Most solid material dissolved with stirring. After 5 h, the solution was filtered to remove insoluble material and the filtrate was freeze-dried to yield the desired product as a white solid (11 g, 100 %).

Example 4

rac-[2R,3S(R)]-6,8-Difluoro-1-(2-fluoroethyl)-1,4-dihydro-7-(4-methyl-1-piperazinyl)-4-oxo-3-quinolinecarboxylic acid [[3-(1-hydroxyethyl)-1-[2-[(2,2-dimethyl-1-oxopropoxy)methoxy]-2-oxo-1-(triphenylphosphoranylidene)ethyl]-4-oxo-2-azetidinyl]acetyl] methyl ester

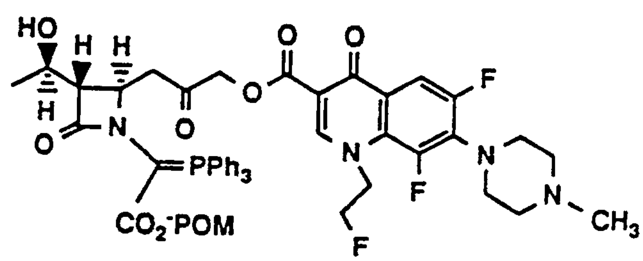

A solution of 6,8-difluoro-1-(2-fluoroethyl)-1,4-di-hydro-7-(4-methyl-1-piperazinyl)-4-oxo-3-quinolinecarboxylate potassium salt (0.115 g, 0.282 mmole) in dry DMF (4 ml) was stirred under an argon atmosphere. Triethylamine (40 µL, 0.287 mmole) was added followed by the addition of rac-[2R,3S(R)]-2-(3-bromo-2-oxopropyl)-3-(1-hydroxyethyl)-4-oxoazetidine-1-(triphenylphosphoranylidene) acetic acid (2,2-dimethyl-1-oxopropoxy)methyl ester (0.200 g, 0.293 mmole). The mixture was stirred at ambient temperature for 17 hours. After this time, the mixture was diluted with 25 ml of ethyl acetate and washed with water (4 x 5 ml) and then with brine (1 x 5 ml). Each aqueous washing was back-extracted using the same 20 ml of ethyl acetate. The combined organic extracts were dried (sodium sulfate) and concentrated. The residue was purified on a chromatotron model 7924 centrifugally-accelerated, radial TLC apparatus (2 mm silica gel plate) using methylene chloride-methanol (8:1 to 4:1) as eluent to afford 0.172 g (63%) of the title compound as a yellow solid.

Example 5

rac-[2R,3S(R)]-6,8-Difluoro-1-(2-fluoroethyl)-1,4-dihydro-7-(4-methyl-1-piperazinyl)-4-oxo-3-quinolinecarboxylic acid [[3-(1-(trimethylsilyloxy)ethyl)-1-[2-[(2,2-dimethyl-1-oxopropoxy)methoxy]-2-oxo-1-(triphenylphosphoranylidene)ethyl]-4-oxo-2-azetidinyl]acetyl]methyl ester

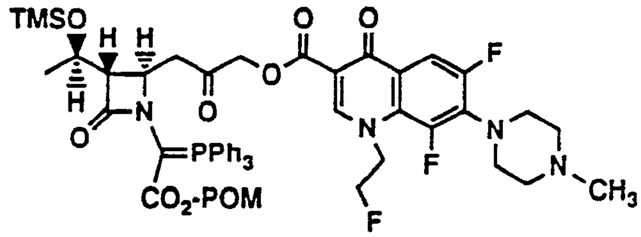

Chlorotrimethylsilane (1.4 ml, 11.03 mmole) was added to a stirred solution of the lactam-quinolone (Example 4) (0.713 g, 0.734 mmole) in dry pyridine (6.0 ml) under argon. The reaction flask was sealed and stirred at ambient temperature for 22 hours. The reaction mixture was diluted with 125 ml of ethyl acetate and extracted with cold 0.25M aqueous sodium bicarbonate solution (2 x 20 ml) and then washed with brine (1x). The organic phase was dried (sodium sulfate) and concentrated under high vacuum at 30° C. The residue was dissolved in ethyl acetate and again concentrated under high vacuum at 30° C. This procedure was repeated two more times to remove as much pyridine as possible. The resulting title compound obtained was used directly in the following step.

Example 6

rac-[5R,6S(R)]-3-[[[[6,8-Difluoro-1-(2-fluoroethyl)-1,4-dihydro-7-(4-methyl-1-piperazinyl)-4-oxo-3-quinolonyl] carbonyl]oxy]methyl]-7-oxo-6[1-[(trimethylsilyl)oxy]ethyl] 1-azabicyclo[3.2.0]hept-2-ene-2-carboxylic acid (2,2-dimethyl-1-oxopropoxy)methyl ester

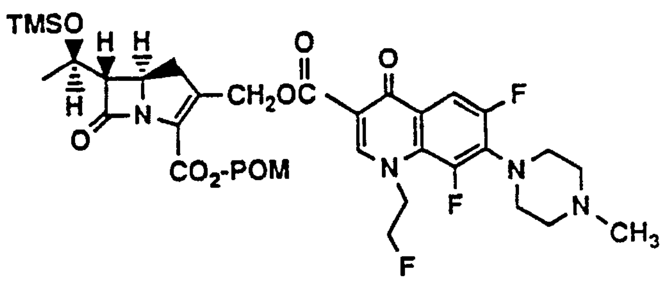

The crude silylated intermediate prepared by the previous procedure was taken up in dry toluene (35 ml) and heated at 105° C for 75 minutes under an argon atmosphere. The reaction was concentrated and the residue was purified on a 2 mm silica gel chromatotron plate (methylene chloride:methanol - 9:1 as eluent) to obtain 0.442 g (80%) of the desired carbapenem as a white solid.

Example 7

rac-[5R,6S(R)]-3-[[[[6,8-Difluoro-1-(2-fluoroethyl)-1,4-dihydro-7-(4-methyl)-1-piperazinyl)-4-oxo-3-quinolonyl]-carbonyl]oxy]methyl]-6-(1-hydroxyethyl)-7-oxo-1-azabicyclo[3.2.0]hept-2-ene-2-carboxylic acid (2,2-dimethyl-1-oxopropoxy)methyl ester

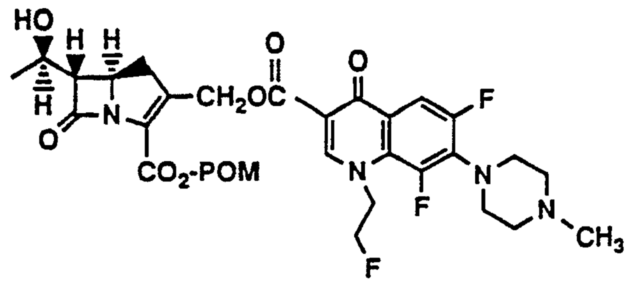

Silylated carbapenem (Example 6) (0.335 g, 0.438 mmole) was dissolved in dry methanol (17 ml) and stirred at ambient temperature under an argon atmosphere. Ammonium fluoride (38 mg, 1.026 mmole) was added in one portion. After 75 minutes an additional portion of ammonium fluoride (5 mg, 0.13 mmole) was added and the solution was stirred for an additional 15 minutes. The mixture was diluted with 125 ml of ethyl acetate and washed with brine (2 x 25 ml). The organic phase was dried (sodium sulfate) and concentrated. The residue was purified on a 2 mm silica gel chromatotron plate using methylene chloride: methanol (15:2) as eluent to afford 0.204g (67%) of the title compound as a white solid.

Example 8

rac-[5R,6S(R)]-3-[[[[6,8-Difluoro-1-(2-fluoroethyl)-1,4-dihydro-4-oxo-7-(4-methyl-1-piperazinyl)-3-quinolonyl]-carbonyl]oxy]methyl]-6-(1-hydroxyethyl)-7-oxo-1-azabicyclo[3.2.0]hept-2-ene-2-carboxylic acid monosodium salt

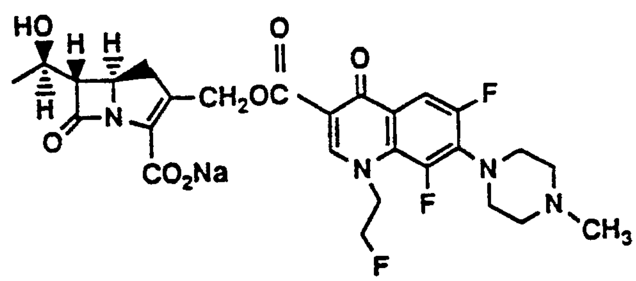

The carbapenem (Example 7) (0.124 g, 0.179 mmole) was dissolved in dimethylsulfoxide (8.0 ml) with stirring at ambient temperature. To this solution, pH 7.0 phosphate buffer (30 ml, 0.025 molar in phosphate) was added which resulted in the precipitation of the carbapenem. At this time, porcine liver esterase (150 μL, EC3.1.1.1) was added followed by an additional portion of the pH 7.0 buffer (10 ml) which brought the pH of the reaction mixture to 7.8. The mixture was stirred at ambient temperature and sodium hydroxide (0.1N) was added as needed to keep the pH of the mixture between 7.2 - 7.4. Two additional portions of the esterase were added during this period as follows: 100 μL after one hour and 125 μL after two hours. As the reaction progressed precipitated material dissolved into solution. After 4 hours, the nearly homogeneous reaction mixture was extracted with ether (2 x 150 ml). The aqueous phase was concentrated under high vacuum to half its original volume. The remaining aqueous solution was chromatographed on a column containing 40 g of $C_{18}$-silica (from Waters). The column was eluted with a stepwise gradient of water to water - acetonitrile (0 to 20% acetonitrile) under pressure. Appropriate fractions were combined, concentrated and freeze-dried to obtain 43.5 mg (41%) of product.

Example 9

rac-[2R,3S(R)]-2-[3-(chlorocarbonyloxy)-2-oxopropyl]-3-[1-[[(2-trichloroethoxy)carbonyl]oxy]ethyl]-4-oxoazetidine-1-[2-(1-triphenylphosphorylidene)acetic acid](2,2-dimethyl-1-oxopropoxy) methyl ester

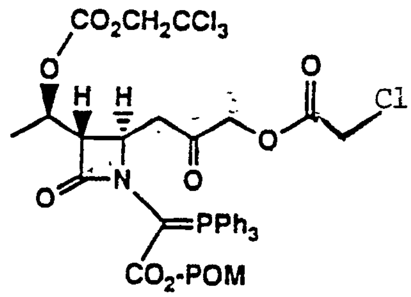

A solution of rac-[2R,3S(R)]-2-(3-bromo-2-oxopropyl)-3-[1-[[2-trichloroethoxy)carbonyl]oxy]ethyl]-4-oxoazetidine-1-triphenylphosphoranylidene)acetic acid (2,2-dimethyl-1-oxopropoxy)methyl ester *(4.060 g, 4.73 mmole) in dry DMF (67 ml) was stirred under an argon atmosphere. Triethylamine (0.70 ml, 5.02 mmole) was added followed by the sodium salt of chloroacetic acid (0.68 g, 5.84 mmole). The mixture was stirred at ambient temperature for 1.5 hours. After this time, the mixture was diluted with 400 ml of ethyl acetate and washed with water (4 x 80 ml) and then with brine (1 x 80ml). The organic extract was dried (sodium sulfate) and concentrated. The residue was purified by flash chromatography (silica gel, ethyl acetate-methylene chloride-hexane (1:1:1) as eluent) to afford 2.725 g (66%) of the title compound.

Example 10

rac-[2R,3S(R)]-2-[3-hydroxy-2-oxopropyl]-3-[1-[[(2-trichloroethoxy)carbonyl]oxy]ethyl]-4-oxoazetidine-1-[2-(1-triphenylphosphorylidene) acetic acid] (2,2-dimethyl-1-oxo-propoxy)methyl ester

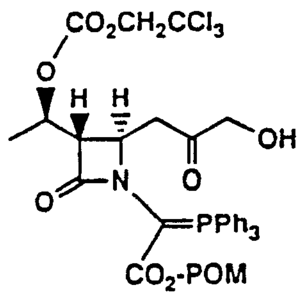

The beta-lactam (Example 9) (2.587 g, 2.98 mmole) was dissolved in methanol (100 ml) and the resulting solution was cooled to 0-5° C. To this solution 0.10N sodium bicarbonate (32 ml, 3.20 mmole) was added and the mixture was stirred for 45 minutes at 0-5° C. After this time, the solution was diluted with 500 ml of ethyl acetate and washed with water (2 x 200 ml) and then brine (1x). The organic extract was dried (sodium sulfate) and concentrated. The crude residue was purified by flash chromatography (silica gel, ethyl acetate-methylene chloride-hexane (1:1:1) as eluent) to yield 2.027 g (88%) of the title compound.

Example 11

rac-[2R,3S(R)]-2-[3-[[[3-carboxy-1-cyclopropyl-6-fluoro-1,4-dihydro-4-oxo-7-quinolonyl)-1-piperazinyl]-carbonyl]oxy]-2-oxopropyl]-3-[[1-[(2-trichloroethoxy)carbonyl]oxy]ethyl]-4-oxoazetidine-1-[2-(1-triphenylphosphorylidene)acetic acid](2,2-dimethyl-1-oxopropoxy)methyl ester

* - E. Götschi, French Patent FR 2,499,081, British Patent GB 2,092,147 and Canadian Patent 1,117,965

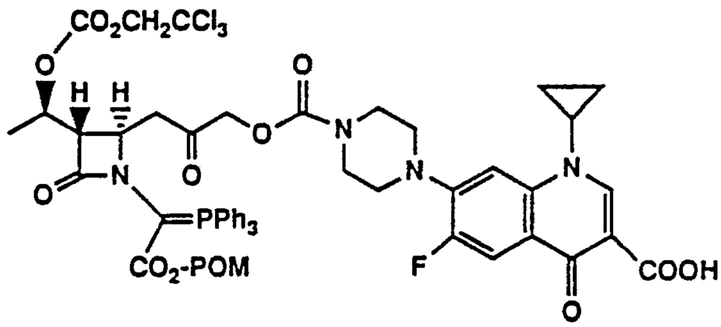

Under an argon atmosphere, a solution of phosgene (0.25 ml of 20% solution in toluene, 0.483 mmole) in 1.5 ml of methylene chloride was cooled to 0° C. A cooled and dry solution of the azetidinone from Example 10 (0.197 g, 0.248 mmole) in 2.5 ml of methylene chloride was then added simultaneously with N,N-diisopropylethylamine (70 µl, 0.409 mmole) to the phosgene solution. The mixture was stirred at 0° C for 30 minutes and then at ambient temperature for 45 minutes. After this time, the reaction mixture was diluted with an additional 10 ml of methylene chloride and then concentrated to the original volume at 5° C. The resultant solution was then added to a solution of ciprofloxacin (0.070 g, 0.211 mmole) in 2.8 ml of methylene chloride which had been pretreated with N-methyl-N-(trimethyl-silyl) trifluoroacetamide (78 µl, 0.420 mmole) for 40 minutes at room temperature and then cooled to 0° C. This mixture was stirred at 0° C for 35 minutes and then for 75 minutes at ambient temperature. After this time, the mixture was concentrated under high vacuum. The filtrate was extracted with pH 4 phosphate buffer (2 x 3 ml) and then with brine solution (1x). The combined organic extract was dried (sodium sulfate) and concentrated. The crude residue was purified on a 2 mm plate silica gel chromatotron plate, using ethyl acetate followed by ethyl acetate-acetonemethanol-water (70:10:5:5) as the eluent, to obtain 112 mg (46%) of the title compound.

Example 12

rac-[2R,3S(R)]-2-[3-[[[(3-carboxy-1-cyclopropyl-6-fluoro-1,4-dihydro-4-oxo-7-quinolonyl)-1-piperazinyl]-carbonyl]oxy]-2-oxopropyl]-3-[1-(hydroxy)ethyl]-4-oxoazetidine-1-[2-(1-triphenylphosphorylidene)acetic acid] (2,2-dimethyl-1-oxopropoxy)methyl ester

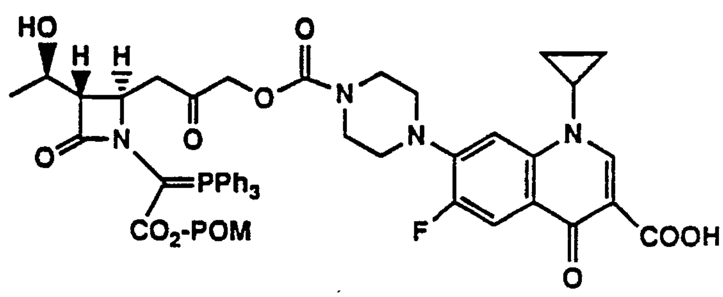

The azetidinone (0.468 g, 0.406 mmole), prepared in the previous step, was taken up in 3 ml of 90% acetic acid and treated with zinc (0.95 g) for 45 minutes at ambient temperature. After this time, the reaction mixture was diluted with 36 ml of methylene chloride and the the mixture was filtered to remove the insoluble material. The filtrate was extracted with saturated sodium carbonate solution (2 x 15 ml) and brine (2 x 25 ml). The aqueous extracts were backwashed twice with methylene chloride because of the thick emulsions that formed. The organic extracts were combined, dried (sodium sulfate), and concentrated. The residue was crystallized from ethyl acetate-ether to give 0.236 g (59%) of the title compound. An additional 0.064 g (16%) of the title compound was obtained by purifying the mother liquor on a 1 mm chromatotron plate using ethyl acetate - acetone - methanol - water (70:10:5:5) as the eluent.

Example 13

rac-[5R,6S(R)]-3-[[[(3-carboxy-1-cyclopropyl-6-fluoro-1,4-dihydro-4-oxo-7-quinolonyl)-1-piperazinyl]carbonyl]-

oxy]methyl-6-[(1-trimethylsilyl)oxy]ethyl-7-oxo-1-azabicyclo[3.2.0]hept-2-ene-2-carboxylic acid (2,2-dimethyl-1-oxopropoxy)methyl ester

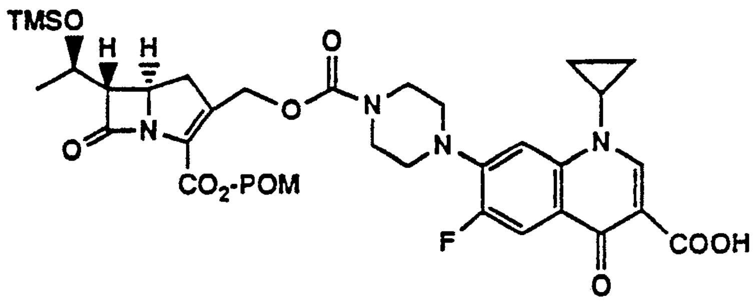

The beta-lactam (Example 12) (0.233 g, 0.237 mmole) was dissolved in 2 ml of dry pyridine and the flask was charged with argon. Trimethylchlorosilane was then added, the flask was sealed and the mixture was stirred at ambient temperature for 5.5 hours. The reaction mixture was then diluted with ethyl acetate and extracted with cold 0.25M sodium bicarbonate solution (2 x 5 ml) and then brine (1x). The organic extract was dried (sodium sulfate) and concentrated under high vacuum. The residue was taken up in ethyl acetate three times and concentrated under high vacuum each time in order to remove the residual pyridine. The residue was then dissolved in toluene (9 ml) and heated to 105 °C for 1 hour. Concentration of the reaction mixture and chromatography of the crude residue on a 1 mm chromatotron plate (ethyl acetate-acetone-methanol-water [70:10:5:5] as eluent) afforded 139 mg (75%) of the title compound.

Example 14

rac-[5R,6S(R)]-3-[[[(3-carboxy-1-cyclopropyl-6-fluoro-1,4-dihydro-4-oxo-7-quinolonyl)-1-piperazinyl]carbonyl]-oxy]methyl-6-[1-hydroxyethyl]-7-oxo-1-azabicyclo[3.2.0]hept-2-ene-2-carboxylicacid-(2,2-dimethyl-1-oxopropoxy)methyl ester

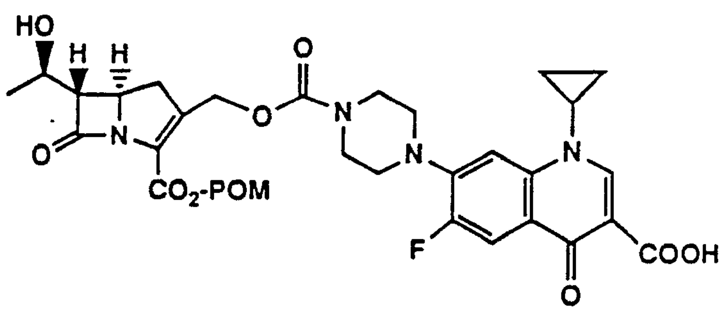

A solution of the silylated carbapenem (Example 13) (110 mg, 0.143 mmole) in 6.5 ml of methanol was treated with ammonium fluoride (38mg). Methylene chloride (2-3 ml) was added to obtain a clear solution. The mixture was stirred at ambient temperature for 80 minutes. After this time, the mixture was diluted with ethyl acetate and extracted with brine (2x). The organic extract was dried (sodium sulfate) and concentrated. The crude residue was chromatographed on a 1 mm chromatotron plate with ethyl acetate-acetone-methanol-water (70:10:5:5) as eluant, giving 70 mg (70%) of the title compound.

Example 15

rac-[5R,6S(R)]-3-[[[(3-carboxylate-1-cyclopropyl-6-fluoro-1,4-dihydro-4-oxo-7-quinolonyl)-1-piperazinyl]-carbonyl]oxy]methyl-6-[1-hydroxyethyl]-7-oxo-1-azabicyclo[3.2.0]hept-2-ene-2-carboxylate disodium salt

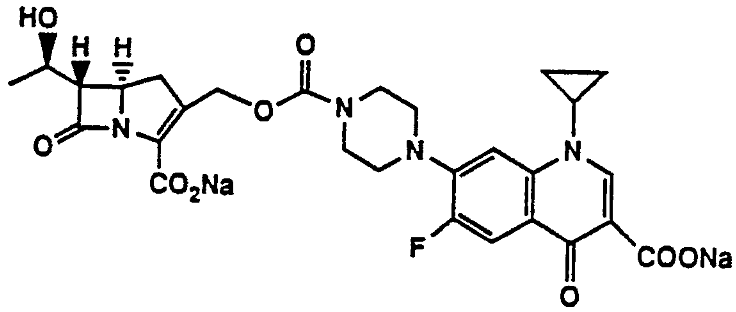

The carbapenem (Example 4) (30 mg, 0.043 mmole) was dissolved in dimethylsulfoxide (1 ml). To this solution, pH 7.0 buffer (2 ml, 0.2M in phosphate) was added which resulted in the precipitation of the carbapenem. Porcine liver esterase (95 µL, EC3.1.1.1) was added which brought the pH of the mixture to 7.70. The mixture was stirred at ambient temperature and sodium hydroxide was added as needed to keep the pH of the mixture between 7.1 - 7.4. Two additional portions of the esterase were added during the early part of the reaction: 50 µL after 20 minutes and another 50 µL after one hour. The reaction mixture was then stirred at ambient temperature for additional 18 hours. The mixture (solid still present) was then extracted with ether (2x). The aqueous extract was chromatographed on a column containing 10 g of $C^{18}$-silica (from Waters). The column was eluted with a stepwise gradient of water to water-acetonitrile (0 to 20% acetonitrile). The appropriate fractions were combined, concentrated and freeze-dried to obtain 2 mg of the final product.

Following the previously described processes in Examples 1-8, the following compounds may be prepared:

rac-[4S,5R,6S(R)]-3[[[6,8-Difluoro-1-(2-fluoroethyl)-1,4-dihydro-4-oxo-7-(4-methyl-1-piperazinyl)-3-quinolonyl]carbonyl]oxy]methyl]-6-(1-hydroxyethyl)-4-methyl-7-oxo-1-azabicyclo-[3.2.0]hept-2-ene-2-carboxylic acid

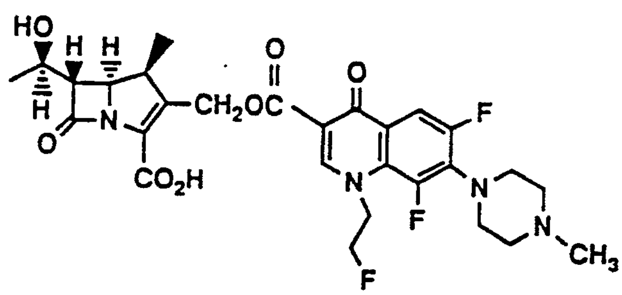

rac-[4S,5S,6R]-3-[[[1-Cyclopropyl-6-fluoro-1,4-dihydro-4-oxo-7-(1-piperazinyl)-3-quinolonyl]carbonyl]oxy]-methyl]-6-ethyl-4-thiomethyl-7-oxo-1-azabicyclo[3.2.0]hept-2-ene-2-carboxylic acid

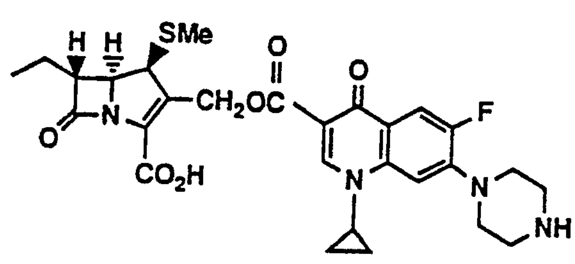

rac-3-[[[7-(3-Amino-1-pyrrolidinyl)-1-cyclopropyl-6,8-difluoro-1,4-dihydro-4-oxo-3-quinolonyl]carbonyl]oxy]-methyl]-4,4-dimethyl-7-oxo-1-azabicyclo[3.2.0]hept-2-ene-2-carboxylic acid

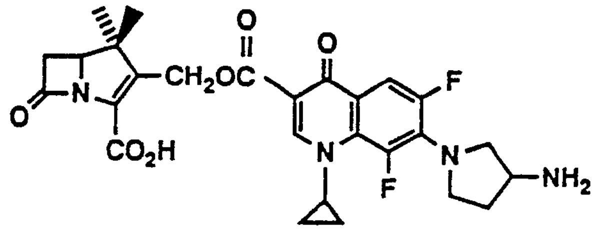

rac-[4S,5R,6R]-3-[[[[1-Cyclopropyl-6,8-difluoro-1,4-dihydro-4-oxo-7-(4-pyridinyl)-3-quinolonyl]carbonyl]oxy]-methyl]-4,6-dimethoxy-7-oxo-1-azabicyclo[3.2.0]hept-2-ene-2-carboxylic acid

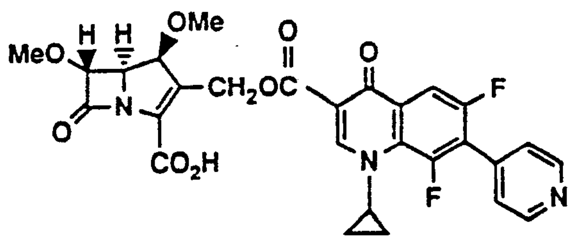

rac-[4S,5R,6S(R)]-3-[[[[5-Amino-7-(3-amino-1-pyrrolidinyl)-1-cyclopropyl-6,8-difluoro-1,4-dihydro-4-oxo-3-quinolonyl]carbonyl]oxy]methyl]-6-(1-hydroxyethyl)-4-methyl-7-oxo-1-azabicyclo[3.2.0]hept-2-ene-2-carboxylic acid

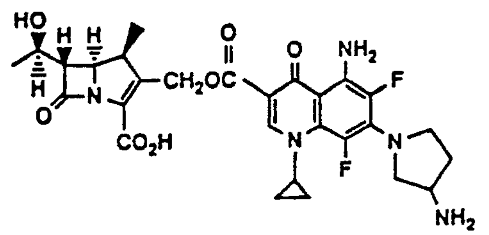

rac-[4S,5R,6S(R)]-3-[[[[10-(3-Amino-1-pyrrolindinyl)-9-fluoro-3,7-dihydro-3-methyl-7-oxo-2H-pyrido[3,2,1-ij]-[4,1,2]benzoxadiazin-6-yl]carbonyl]oxy]methyl]-6-(1-hydroxyethyl)-4-methyl-7-oxo-1-azabicyclo[3.2.0]hept-2-ene-2-carboxylic acid

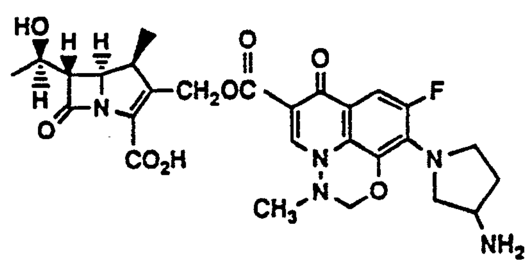

rac-[4S,5R,6S(R)]-3-[[[[1-Cyclopropyl-6-fluoro-1,4-dihydro-4-oxo-7-(1-piperazinyl)-3-quinolonyl]carbonyl]oxy]-methyl]-6-(1-hydroxy-ethyl)-4-methyl-7-oxo-1-azabicyclo[3.2.0]hept-2-ene-2-carboxylic acid

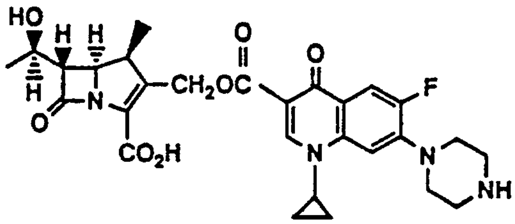

rac-[4S,5R,6S(R)]-3-[[[[6-Fluoro-1-ethyl-1,4-dihydro-4-oxo-7-(1-piperazinyl)-1,8-naphthridin-3-yl]carbonyl]-oxy]methyl]-6-(1-hydroxyethyl)-4-methyl-7-oxo-1-azabicyclo[3.2.0]hept-2-ene-2-carboxylic acid

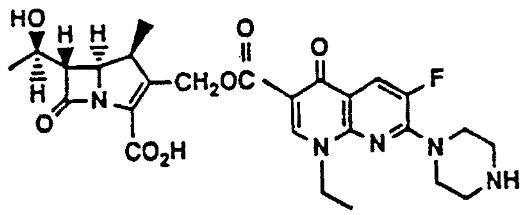

rac-[4S,5R,6S(R)]-3-[[[[9-Fluoro-10-(4-methyl-1-piperazinyl)-3,7-dihydro-3-methyl-7-oxo-2H-pyrido[1,2,3-de]-1,4-benzoxazin-6-yl]carbonyl]oxy]methyl]-6-(1-hydroxyethyl)-4-methyl-7-oxo-1-azabicyclo[3.2.0]hept-2-ene-2-carboxylic acid

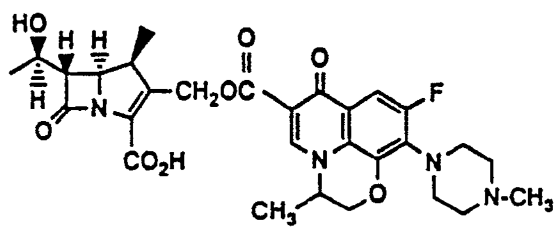

Following the previously described processes in Examples 9-15 the following compounds may be prepared:

rac-[4S,5S,6R]-3-[[[[[(3-Carboxy-1-cyclopropyl-6-fluoro-1,4-dihydro-4-oxo-7-quinolonyl)-3-pyrrolidinyl]amino]-carbonyl]oxy] methyl]-6-ethyl-4-thiomethyl-7-oxo-1-azabicyclo[3.2.0]hept-2-ene-2-carboxylic acid

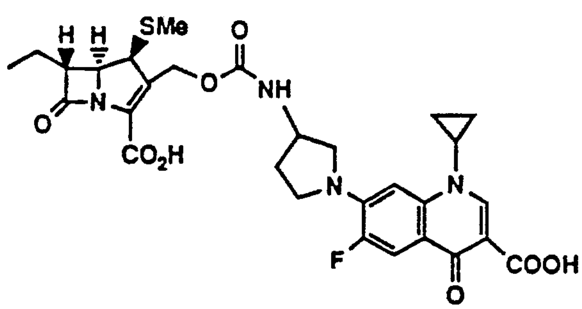

rac-3-[[[[[(3-Carboxy-1-cyclopropyl-6,8-difluoro-1,4-dihydro-4-oxo-7-quinolonyl)-3-pyrrolidinyl]amino]-carbonyl]oxy]methyl]-4-dimethyl-7-oxo-1-azabicyclo[3.2.0]hept-2-ene-2-carboxylic acid

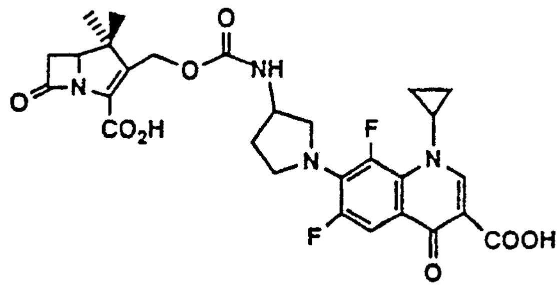

rac-[4S,5R,6R]-3-[[[[(3-Carboxy-1-ethyl-6-fluoro-1,4-dihydro-4-oxo-1,8-naphthyridin-7-yl)-1-piperazinyl]-carbonyl]oxy]methyl]-4,6-dimethoxy-7-oxo-1-azabicyclo[3.2.0]hept-2-ene-2-carboxylic acid

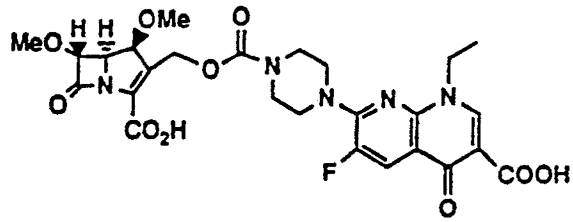

rac-[4S,5R,6S(R)]-3-[[[[(3-Carboxy-1-cyclopropyl-6-fluoro-1,4-dihydro-4-oxo-7-quinolonyl)-1-piperazinyl]-carbonyl]oxy]methyl]-6-(1-hydroxyethyl)-4-methyl-7-oxo-1-azabicyclo-[3.2.0]hept-2-ene-2-carboxylic acid

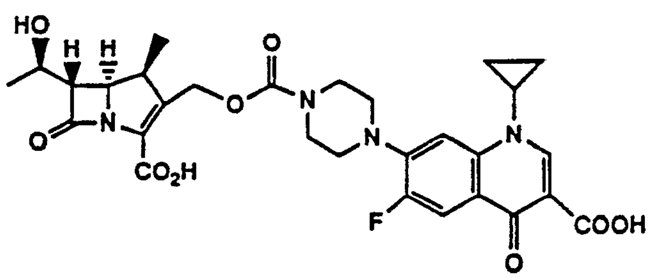

rac-[4S,5R,6S(R)]-3-[[[[(9-Cyclopropyl-6-fluoro-2,3,4,9-tetrahydro-3,4-dioxoisothiazolo[5,4-b]quinolin-7-yl)-1-piperazinyl]carbonyl]oxy]methyl]-6-(1-hydroxyethyl)-4-methyl-7-oxo-1-azabicyclo[3.2.0]hept-2-ene-2-carboxylic acid

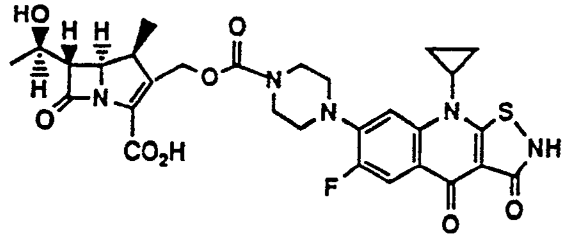

rac-[4S,5R,6S(R)]-3-[[[[[[9-Fluoro-3,7-dihydro-3-methyl-7-oxo-2H-pyrido[3,2,1-ij][4,1,2]benzoxadiazin-10-yl]-3-pyrrolidinyl] amino]carbonyl]oxy]methyl]6-(1-hydroxyethyl)-4-methyl-7-oxo-1-azabicyclo[3.2.0]hept-2-ene-2-carboxylic acid

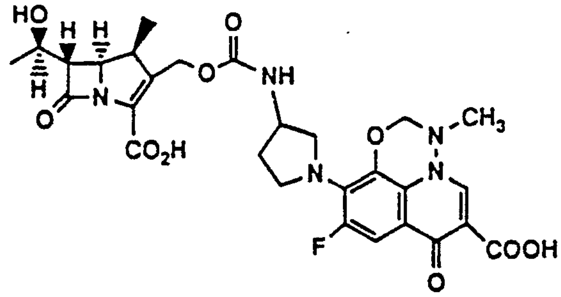

rac-[4S,5R,6S(R)]-3-[[[[[(5-Amino-3-carboxy-1-cyclopropyl-6,8-difluoro-1,4-dihydro-4-oxo-7-quinolonyl)-3-pyrrolidinyl]amino] carbonyl]oxy]methyl]-6-(1-hydroxyethyl)-4-methyl-7-oxo-1-azabicyclo[3.2.O]hept-2-ene-2-carboxylic acid

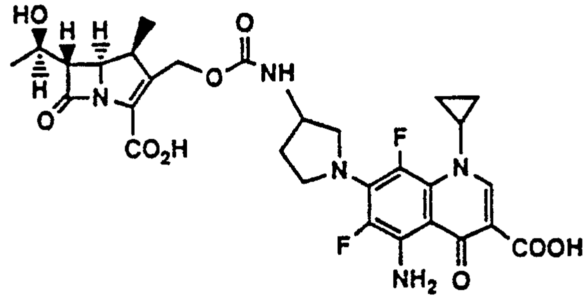

rac-[4S,5R,6S(R)]-3-[[[[[(3-Carboxy-1-cyclopropyl-6,8-di-fluoro-1,4-dihydro-4-oxo-7-quinolonyl)-3-pyrrolidinyl]amino]carbonyl]oxy]methyl]-6-(1-hydroxyethyl)-4-methyl-7-oxo-1-azabicyclo[3.2.0]hept-2-ene-2-carboxylic acid

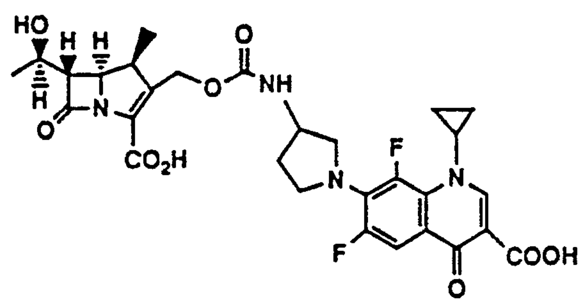

rac-[4S,5R,6S(R)]-3-[[[[[(3-Carboxy-1-(2,4-difluorophenyl)-6-fluoro-1,4-dihydro-4-oxo-1,8-naphthyridin-7-yl)-3-pyrrolidinyl]amino]carbonyl]oxy]methyl]-6-[(1-hydroxyethyl)-4-methyl-7-oxo-1-azabicyclo[3.2.0]hept-2-ene-2-carboxylic acid

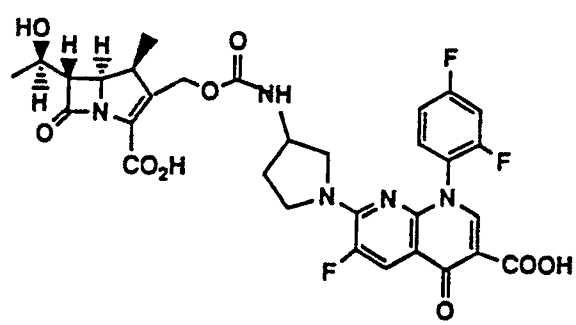

Following the processes previously described in Schemes 3-8, the following compounds may be

prepared:
rac-[4S,5R,6S(R)]-3-[[9-Cyclopropyl-6-fluoro-7-(1-piperazinyl)-2,3,4,9-tetrahydro-3,4-dioxoisothiazolo[5,4-b]-quinolin-2-yl)]methyl]-6-(1-hydroxyethyl)-4-methyl-7-oxo-1-azabicyclo-[3.2.0]hept-2-ene-2-carboxylic acid

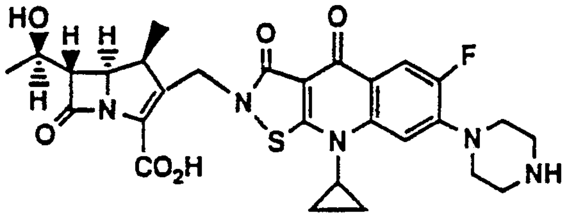

rac-[4S,5R,6S(R)]-3[[[[6,8-Difluoro-1-(2-fluoroethyl)-1,4-dihydro-4-oxo-7-(4-methyl-1-piperazinyl)-3-quinolonyl]carbonyl]thio]methyl]-6-(1-hydroxyethyl)-4-methyl-7-oxo-1-azabicyclo[3.2.0]hept-2-ene-2-carboxylic acid

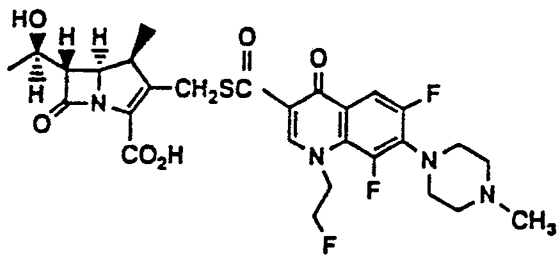

rac-[4S,5R,6S(R)]-3[[(3-Carboxy-1-cyclopropyl-6-fluoro-1,4-dihydro-4-oxo-7-quinolonyl)-1-piperazinyl]-methyl]-6-(1-hydroxyethyl)-4-methyl-7-oxo-1-azabicyclo[3.2.0]hept-2-ene-2-carboxylic acid

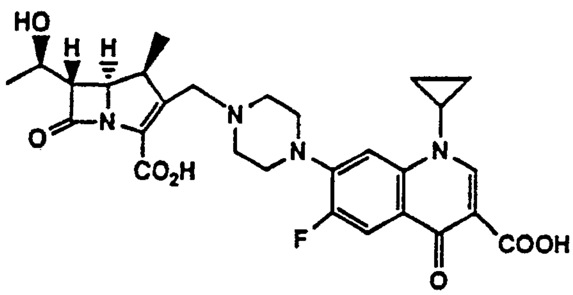

rac-[4S,5R,6S(R)]-4-[3-Carboxy-1-cyclopropyl-6-fluoro-1,4-dihydro-4-oxo-7-quinolonyl]-1-[2-carboxy-6-(1-hydroxyethyl)-4-methyl-7-oxo-1-azabicyclo[3.2.0]hept-2-enyl]methyl-1-methylpiperazinium iodide

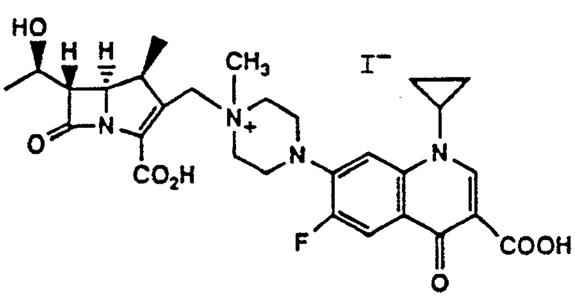

rac-[4S]-3-[[[[3-carboxy-1-cyclopropyl-6-fluoro-1,4,8,9,10,11, 11a,12-octahydro-4-oxo-pyrazino[1',2':4,5][1,4]-oxazino[3,2-h]-quinolin-10-yl]carbonyl]oxy]methyl]-4-methyl-7-oxo-1-azabicyclo[3.2.0]hept-2-ene-2-carboxylic

acid

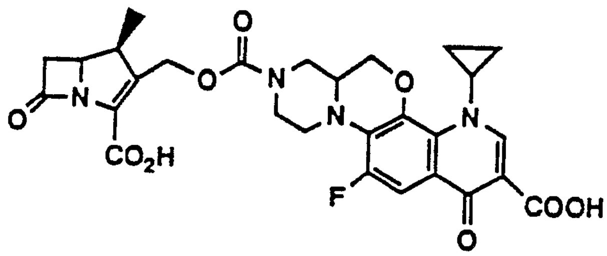

rac-[6R]-3-[[3-carboxy-1-cyclopropyl-6-fluoro-1,4,8,9,10,11, 11a,12-octahydro-4-oxo-pyrazino[1',2':4,5][1,4]-oxazino[3,2-h]-quinolin-10-yl]methyl]-7-oxo-4,4,6-trimethyl-1-azabicyclo-[3.2.0]hept-2-ene-2-carboxylic acid

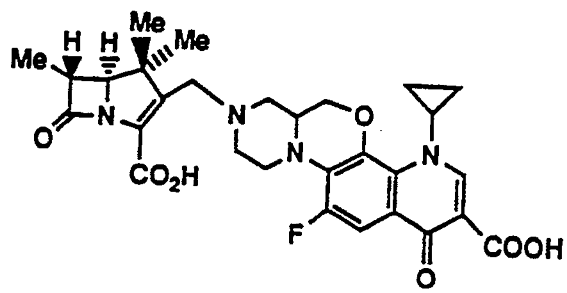

rac-[4S,5R,6S(R)]-10-[2-carboxy-6(1-hydroxyethyl)-4-methyl-7-oxo-1-azabicyclo[3.2.0]hept-2-en-3-yl]methyl-10-methyl-[3-carboxy-1-cyclopropyl-6-fluoro-1,4,8,9,10,11,11a,12-octahydro-4-oxo-pyrazino[1',2':4,5][1,4]-oxazino[3,2-h]quinolinium] iodide

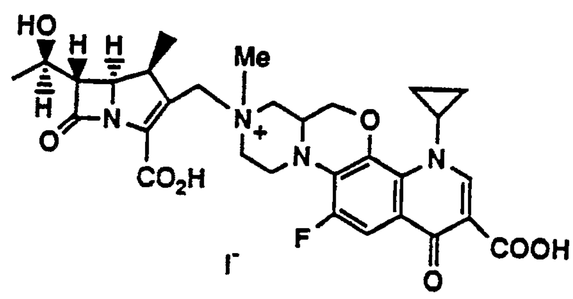

## Claims

1. Carbapenem compounds of the general formula

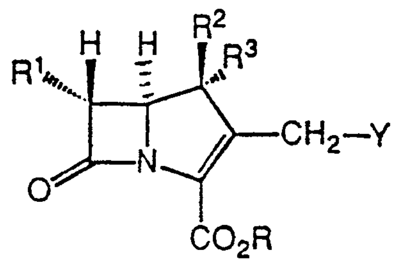

I

wherein R is hydrogen or a carboxylic acid protecting group; $R^1$ is selected from the group

consisting of hydrogen, lower alkyl, lower alkoxy and hydroxyalkyl; $R^2$ and $R^3$ are each independently hydrogen, lower alkyl, lower alkoxy or lower alkylthio; and Y is

(i) an ester group;
(ii) a thioester group;
(iii) a piperazinyl or pyrrolidinyl derived carbamate group;
(iv) a piperazinyl derived amine group;
(v) a piperazinyl derived quaternary ammonium group; or
(vi) a group comprising a covalent bond;

the ester group and the thioester group being bonded to a substituted quinolonyl or naphthyridonyl group; the piperazinyl or pyrrolidinyl derived carbamate group, the piperazinyl derived amine group and the piperazinyl derived quaternary ammonium group containing a piperazinyl nucleus or a pyrrolidinyl nucleus having a nitrogen atom bonded directly to a substituted quinolonyl or naphthyridonyl group, the piperazinyl and the pyrrolidinyl nuclei being unsubstituted or substituted with one or more lower alkyl groups; and the covalent bond of the corresponding group being connected to a substituted isothiazolo or isoxazolo quinolonyl or naphthyridonyl group via a nitrogen atom in an isothiazolo or isoxazolo nucleus; as well as the corresponding readily hydrolyzable esters, pharmaceutically acceptable salts and hydrates of any of the foregoing compounds.

**2.** A compound according to claim 1 wherein Y is an ester of formula

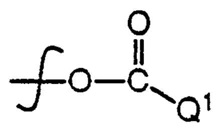

a thioester of formula

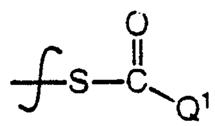

a piperazinyl or pyrrolidinyl derived carbamate of formula

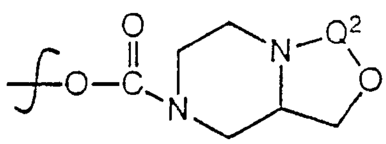 or 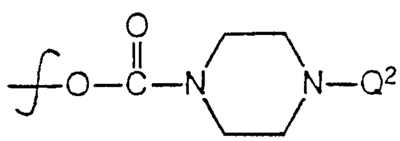

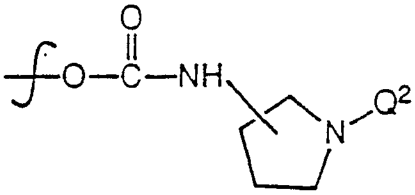 or 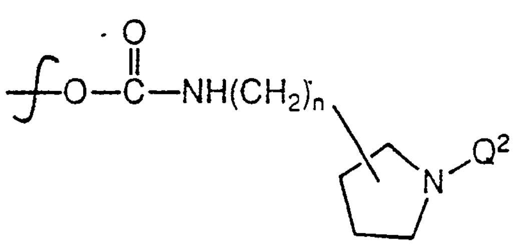

wherein n is 0, 1 or 2.

or a piperazinyl derived amine of formula

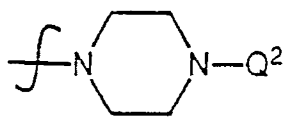

or

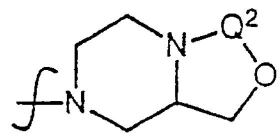

or a piperazinyl derived quaternary ammonium group of formula

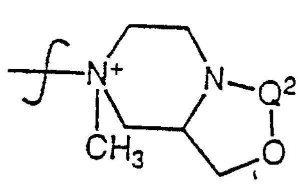

or

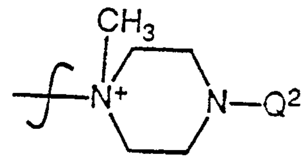

or a group comprising a covalent bond connected to $Q^3$, i.e. a group of formula

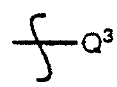

wherein $Q^1$ and $Q^2$ are each independently a substituted quinolonyl or naphthyridonyl group; and $Q^3$ is a substituted isothiazolo or isoxazolo quinolonyl or naphthyridonyl group.

3. A compound according to claim 2 wherein Y is the ester of formula

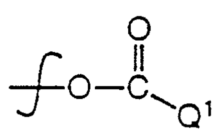

or a piperazinyl derived amine group of formula

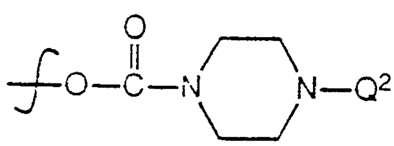

wherein $Q^1$ and $Q^2$ are each independently a substituted quinolonyl or naphthyridonyl group.

4. A compound as in claim 2 wherein $Q^1$ is

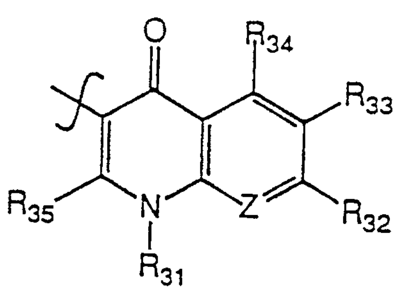

wherein Z represents

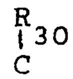

or N, $R_{30}$ is hydrogen or halogen; $R_{31}$ is hydrogen, lower alkyl, lower alkenyl, $C_3$-$C_7$ cycloalkyl, halo-lower-alkyl, phenyl, mono-, di and tri-halophenyl, or

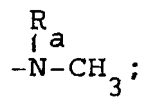

$R_a$ is hydrogen or $R_a$ and $R_{30}$ when taken together represent a lower alkylene mono-oxy group of 1-2 carbon atoms; $R_{30}$ and $R_{31}$ when taken together represent a lower alkylene mono-oxy group of 2-3 carbon atoms; $R_{32}$ represents hydrogen, halogen, lower alkyl, a 5- or 6-membered heterocyclic ring containing one or two heteroatoms selected from the group consisting of O, N and S; or when $R_{32}$ is a piperazine, $R_{30}$ is hydrogen, halogen or a methyleneoxy ($-CH_2O$) bridge to the piperazine of $R_{32}$ to form a fused six-membered ring; $R_{33}$ is hydrogen or halogen; $R_{32}$ and $R_{33}$ when taken together represent a $C_1$-$C_2$ lower alkylene dioxy group; $R_{34}$ is hydrogen or amino; and $R_{35}$ is hydrogen, lower alkoxy or lower alkylthio.

5. A compound as in claim 4 wherein Z is

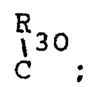

$R_{30}$ is hydrogen or halogen, $R_{31}$ is lower alkyl, $C_3$-$C_7$ cycloalkyl, halo-lower alkyl-or mono-, di- or tri-halophenyl; $R_{32}$ is a piperazinyl or a pyrrolidinyl group; $R_{33}$ is halogen; $R_{34}$ is hydrogen or amino; and $R_{35}$ is hydrogen, lower alkoxy or lower alkylthio.

6. A compound as in claim 5 wherein $R_{30}$ is hydrogen, fluorine or chlorine; $R_{31}$ is ethyl, fluoroethyl, cyclopropyl or fluorophenyl; $R_{32}$ is a piperazinyl, N-methyl piperazinyl or aminopyrrolidinyl group; $R_{33}$ is fluoro; $R_{34}$ is hydrogen; and $R_{35}$ is hydrogen.

7. A compound according to claim 2 wherein $Q^2$ is

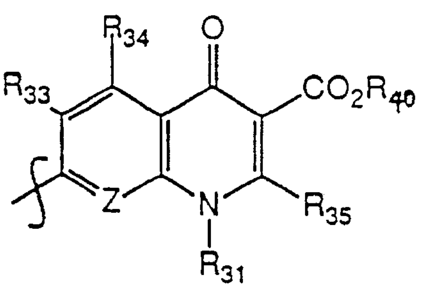

or

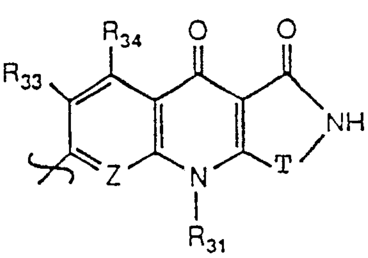

wherein Z represents

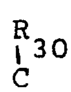

or N;
$R_{30}$ is hydrogen, halogen or a methyleneoxy ($-CH_2O-$) bridge to the piperazine nucleus of Y to form a

fused six-membered ring;
$R_{31}$ represents hydrogen, lower alkyl, lower alkenyl, $C_3$-$C_7$ cycloalkyl, halo-lower-alkyl, phenyl, mono-, di- or tri-halophenyl or

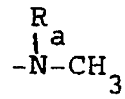 ;

$R_a$ is hydrogen or $R_a$ and $R_{31}$ when taken together represent a lower alkylene mono-oxy group of 1-2 carbon atoms;
$R_{30}$ and $R_{31}$ when taken together represent a lower alkylene mono-oxy group of 2-3 carbon atoms;
$R_{33}$ is hydrogen or halogen;
$R_{34}$ is hydrogen or amino;
$R_{35}$ is hydrogen, lower alkoxy or lower alkylthio;
$R_{40}$ is hydrogen or a carboxylic acid protecting group; and
T is O or S.

**8.** A compound according to claim 7 wherein Z is

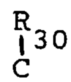 ;

$R_{30}$ is hydrogen or halogen; $R_{31}$ is lower alkyl, $C_3$-$C_7$ cycloalkyl; halo-lower-alkyl, mono-, di- or tri-halophenyl; $R_{32}$ is a piperazinyl or pyrrolidinyl group; $R_{33}$ is halogen; $R_{34}$ is hydrogen or amino; $R_{35}$ is hydrogen; $R_{40}$ is a hydrogen or a carboxylic acid protecting group; and T is O or S.

**9.** A compound according to claim 8 wherein Z is

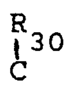 ;

$R_{30}$ is hydrogen, fluoro or chloro; $R_{31}$ is ethyl, fluoroethyl, cyclopropyl or fluorophenyl; $R_{32}$ is a piperazinyl group, a N-methyl piperazinyl group or an aminopyrrolidinyl group; $R_{33}$ is fluoro-; $R_{34}$ is hydrogen; $R_{35}$ is hydrogen; $R_{40}$ is hydrogen; and T is O or S.

**10.** A compound according to claim 2 wherein $Q^3$ is

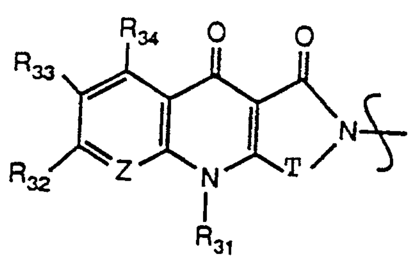

wherein Z represents

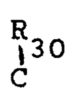

or N;

$R_{30}$ is hydrogen or halogen;
$R_{31}$ is hydrogen, lower alkyl, lower alkenyl, $C_3$-$C_7$ cycloalkyl, halo-lower-alkyl, phenyl, mono-, di- or tri-halophenyl, or

$$-\overset{\displaystyle R_a}{\overset{|}{N}}-CH_3;$$

$R_a$ and $R_{30}$ when taken together represent a lower alkylene mono-oxy group of 1-2 carbon atoms;
$R_{30}$ and $R_{31}$ when taken together represent a lower alkylene mono-oxy group of 2-3 carbon atoms;
$R_{32}$ represents hydrogen, halogen, lower alkyl, a 5- or 6-membered heterocyclic ring containing one or two heteroatoms selected from the group consisting of O, N and S; when $R_{32}$ is a piperazinyl group, $R_{30}$ is hydrogen, halogen or a methyleneoxy ($-CH_2O-$) bridge to a piperazinyl nucleus of $R_{32}$ to form a fused six-membered ring;
$R_{33}$ represents hydrogen and halogen;
$R_{32}$ and $R_{33}$ when taken together represent a lower alkylene dioxy group having 1-2 carbon atoms;
$R_{34}$ is hydrogen or amino; and T is O or S.

**11.** A compound according to claim 10 wherein Z is

$$\overset{\displaystyle R_{30}}{\overset{|}{C}}\;;$$

$R_{30}$ is hydrogen or halogen; $R_{31}$ is lower alkyl, $C_3$-$C_7$ cycloalkyl, halo-lower-alkyl, mono-, di or tri-phenyl; $R_{32}$ is a piperazinyl or a pyrrolidinyl group, $R_{33}$ is halogen; $R_{34}$ is hydrogen; and T is O or S.

**12.** A compound according to claim 11 wherein $R_{30}$ is hydrogen, fluoro or chloro; $R_{31}$ is ethyl, fluoroethyl, cyclopropyl or fluorophenyl; $R_{32}$ is a piperazinyl, N-methyl-piperazinyl or amino pyrrolidinyl group; $R_{33}$ is fluoro; $R_{34}$ is hydrogen; and T is O or S.

**13.** A compound according to claim 1 wherein $R^1$ is hydrogen, lower alkyl, lower alkoxy or hydroxyalkyl.

**14.** A compound according to claim 13 wherein $R^1$ is hydroxyalkyl.

**15.** A compound according to claim 1 wherein $R^2$ and $R^3$ are each independently hydrogen or lower alkyl.

**16.** A compound according to claim 15 wherein $R^2$ and $R^3$ are each hydrogen.

**17.** A compound according to claim 15 wherein $R^2$ is lower alkyl and $R^3$ is hydrogen.

**18.** A compound according to claim 1 having a formula

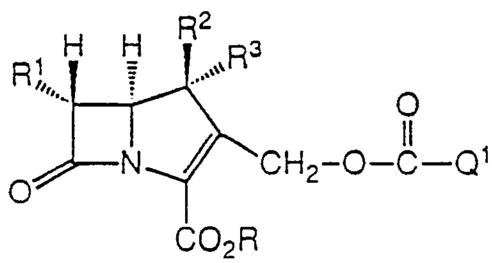

IA

wherein R is hydrogen or a carboxylic acid protecting group; $R^1$ is selected from the group consisting of hydrogen, lower alkyl, lower alkoxy and hydroxyalkyl; $R^2$ and $R^3$ are each independently hydrogen, lower alkyl, lower alkoxy or lower alkylthio and $Q^1$ is formula

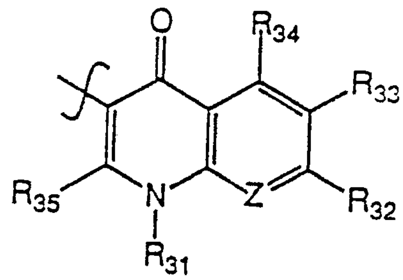

wherein Z represents

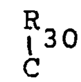

or N, $R_{30}$ is hydrogen or halogen; $R_{31}$ is hydrogen, lower alkyl, lower alkenyl, $c_3$-$C_7$ cycloalkyl, halo-lower-alkyl, phenyl, mono-, di and tri-halophenyl, or

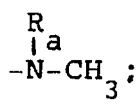

$R_a$ is hydrogen or $R_a$ and $R_{30}$ when taken together represent a lower alkylene mono-oxy group of 1-2 carbon atoms; $R_{30}$ and $R_{31}$ when taken together represent a lower alkylene mono-oxy group of 2-3 carbon atoms; $R_{32}$ represents hydrogen, halogen, lower alkyl, a 5- or 6-membered heterocyclic ring containing one or two heteroatoms selected from the group consisting of O, N and S; or when $R_{32}$ is a piperazine, $R_{30}$ is hydrogen, halogen or a methyleneoxy ($-CH_2O-$) bridge to the piperazine of $R_{32}$ to form a fused six-membered ring; $R_{33}$ is hydrogen or halogen; $R_{32}$ and $R_{33}$ when taken together represent a $C_1$-$C_2$ lower alkylene dioxy group; $R_{34}$ is hydrogen or amino; and $R_{35}$ is hydrogen, lower alkoxy or lower alkylthio.

**19.** A compound according to claim 18 wherein Z is

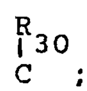

$R_{30}$ is hydrogen or halogen, $R_{31}$ is lower alkyl, $C_3$-$C_7$ cycloalkyl halo-lower-alkyl or halophenyl; $R_{32}$ is a piperazinyl or pyrrolidinyl group; $R_{33}$ is halogen; and $R_{34}$ is hydrogen.

**20.** A compound according to claim 1 of formula

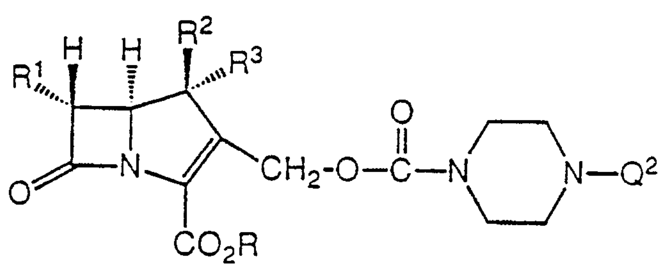

IB

wherein R is hydrogen or a carboxylic acid protecting group; $R^1$ is selected from the group consisting of hydrogen, lower alkyl, lower alkoxy and hydroxyalkyl; $R^2$ and $R^3$ are each independently hydrogen, lower alkyl, lower alkoxy or lower alkylthio and $Q^2$ is of formula

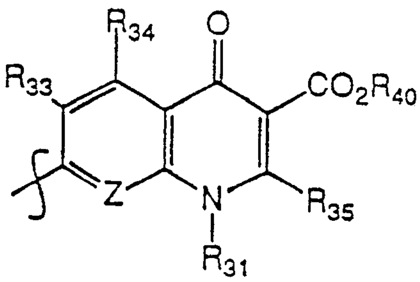

or

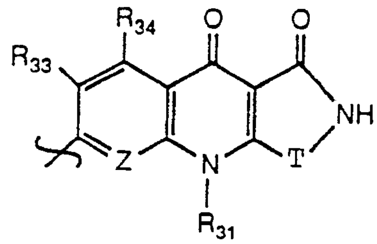

wherein Z represents

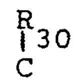

or N;
$R_{30}$ is hydrogen, halogen or a methyleneoxy ($-CH_2O-$) bridge to the piperazine nucleus of Y to form a fused six-membered ring;
$R_{31}$ represents hydrogen, lower alkyl, lower alkenyl, $c_3$-$c_7$ cycloalkyl, halo-lower-alkyl, phenyl, mono-, di-and tri-halophenyl or

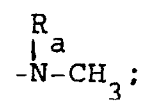

$R_a$ is hydrogen or $R_a$ and $R_{31}$ when taken together represent a lower alkylene mono-oxy group of 1-2 carbon atoms;
$R_{30}$ and $R_{31}$ when taken together represent a lower alkylene mono-oxy group of 2-3 carbon atoms;
$R_{33}$ is hydrogen or halogen;
$R_{34}$ is hydrogen or amino;
$R_{35}$ is hydrogen, lower alkoxy or lower alkylthio;
$R_{40}$ is hydrogen or a carboxylic acid protecting group; and
T is O or S.

**21.** A compound according to claim 1 of formula

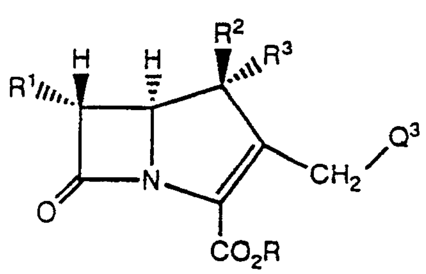

IC

wherein R is hydrogen or a carboxylic acid protecting group; $R^1$ is selected from the group consisting of hydrogen, lower alkyl, lower alkoxy and hydroxyalkyl; $R^2$ and $R^3$ are each independently hydrogen, lower alkyl, lower alkoxy or lower alkylthio and $Q^3$ is of formula

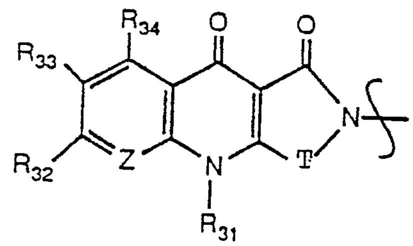

wherein Z represents

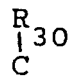

or N;
$R_{30}$ is hydrogen or halogen;
$R_{31}$ is hydrogen, lower alkyl, lower alkenyl, $C_3$-$C_7$ cycloalkyl, halo-lower-alkyl, phenyl, mono-, di- or tri-halophenyl, or

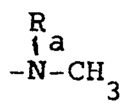 ;

$R_a$ and $R_{30}$ when taken together represent a lower alkylene mono-oxy group of 1-2 carbon atoms;
$R_{30}$ and $R_{31}$ when taken together represent a lower alkylene mono-oxy group of 2-3 carbon atoms;
$R_{32}$ represents hydrogen, halogen, lower alkyl, a 5- or 6-membered heterocyclic ring containing one or two heteroatoms selected from the group consisting of O, N and S; when $R_{32}$ is a piperazinyl group, $R_{30}$ is hydrogen, halogen or a methyleneoxy ($-CH_2O-$) bridge to a piperazinyl nucleus of $R_{32}$ to form a fused six-membered ring;
$R_{33}$ represents hydrogen and halogen;
$R_{32}$ and $R_{33}$ when taken together represent a lower alkylene dioxy group having 1-2 carbon atoms;
$R_{34}$ is hydrogen or amino; and T is O or S.

**22.** A compound according to claim 21 wherein Z is

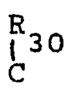 ;

$R_{30}$ is hydrogen or halogen; $R_{31}$ is lower alkyl, $c_3$-$c_7$ cycloalkyl, halo-lower-alkyl or mono-, di- or trihalophenyl; $R_{32}$ is a piperazinyl or a pyrrolidinyl group, $R_{33}$ is halogen; $R_{34}$ is hydrogen; and T is O or S.

**23.** A compound according to claim 22. rac-[4S,5R,6S(R)]-3-([9-cyclopropyl-6-fluoro-7-(1-piperazinyl)-2,3,4,9-tetrahydro-3,4-dioxoisothiazolo[5,4-b]quinolin-2-yl)]methyl]-6-(1-hydroxyethyl)-4-methyl-7-oxo-1-azabicyclo[3.2.0]hept-2-ene-2-carboxylic acid and its salts and hydrates.

**24.** A compound according to claim 1, rac-[5R,6S(R)]-3-[[[[6,8-difluoro-1-(2-fluoroethyl)-1,4-dihydro-4-oxo-7-(4-methyl-1-piperazinyl)-3-quinolonyl]carbonyl]oxy]methyl]-6-(1-hydroxyethyl)-7-oxo-1-azabicyclo-[3.2.0]hept-2-ene-2-carboxylic acid and its salts and hydrates.

**25.** A compound according to claim 1, rac-[5R,6S(R)]-3-[[[[(3-carboxy-1-cyclopropyl-6-fluoro-1,4-dihydro-4-oxo-7-quinolonyl)-1-piperazinyl]carbonyl]oxy]methyl]-6-(1-hydroxyethyl)-7-oxo-1-azabicyclo[3.2.0]hept-2-ene-2-carboxylic acid and its salts and hydrates.

**26.** A compound according to claim 1 selected from the group consisting of

rac-[4S,5S,6R]-3-[[[[1-cyclopropyl-6-fluoro-1,4-dihydro-4-oxo-7-(1-piperazinyl)-3-quinolonyl]carbonyl]-oxy]methyl]-6-ethyl-4-thiomethyl-7-oxo-1-azabicyclo[3.2.0]hept-2-ene-2-carboxylic acid

rac-3-[[[[7-(3-amino-1-pyrrolidinyl)-1-cyclopropyl-6,8-difluoro-1,4-dihydro-4-oxo-3-quinolonyl]carbonyl]-oxy]methyl]-4,4-dimethyl-7-oxo-1-azabicyclo[3.2.0]hept-2-ene-2-carboxylic acid

rac-[4S,5R,6R]-3-[[[[1-cyclopropyl-6,8-difluoro-1,4-dihydro-4-oxo-7-(4-pyridinyl)-3-quinolonyl]carbonyl]-oxy]methyl]-4,6-dimethoxy-7-oxo-1-azabicyclo[3.2.0]hept-2-ene-2-carboxylic acid

rac-[4S,5R,6S(R)]-3-[[[[5-amino-7-(3-amino-1-pyrrolidinyl)-1-cyclopropyl-6,8-difluoro-1,4-dihydro-4-oxo-3-quinolonyl]carbonyl]oxy]methyl]-6-(1-hydroxyethyl)-4-methyl-7-oxo-1-azabicyclo[3.2.O]hept-2-ene-2-carboxylic acid

rac-[4S,5R,6S(R)]-3-[[[[10-(3-amino-1-pyrrolindinyl)-9-fluoro-3,7-dihydro-3-methyl-7-oxo-2H-pyrido[3,2,1-ij][4,1,2]benzoxadiazin-6-yl]carbonyl]oxy]methyl]-6-(1-hydroxyethyl)-4-methyl-7-oxo-1-azabicyclo[3.2.0]-hept-2-ene-2-carboxylic acid

rac-[4S,5R,6S(R)]-3-[[[[6-fluoro-1-ethyl-1,4-dihydro-4-oxo-7-(1-piperazinyl)-1,8-naphthridin-3-yl]carbonyl]-oxy]methyl]-6-(1-hydroxyethyl)-4-methyl-7-oxo-1-azabicyclo[3.2.0]hept-2-ene-2-carboxylic acid

rac-[4S,5R,6S(R)]-3-[[[[9-fluoro-10-(4-methyl-1-piperazinyl)-3,7-dihydro-3-methyl-7-oxo-2H-pyrido[1,2,3-de]-1,4-benzoxazin-6-yl]carbonyl]oxy]methyl]-6-(1-hydroxyethyl)-4-methyl-7-oxo-1-azabicyclo[3.2.0]-hept-2-ene-2-carboxylic acid

rac-[4S,5S,6R]-3-[[[[[(3-carboxy-1-cyclopropyl-6-fluoro-1,4-dihydro-4-oxo-7-quinolonyl)-3-pyrrolidinyl]-amino]carbonyl]oxy] methyl]-6-ethyl-4-thiomethyl-7-oxo-1-azabicyclo[3.2.0]hept-2-ene-2-carboxylic acid

rac-3-[[[[[(3-carboxy-1-cyclopropyl-6,8-difluoro-1,4-dihydro-4-oxo-7-quinolonyl)-3-pyrrolidinyl]amino]-carbonyl]oxy]methyl]-4-dimethyl-7-oxo-1-azabicyclo[3.2.0]hept-2-ene-2-carboxylic acid

rac-[4S,5R,6R]-3-[[[[(3-carboxy-1-ethyl-6-fluoro-1,4-dihydro-4-oxo-1,8-naphthyridin-7-yl)-1-piperazinyl]-carbonyl]oxy]-methyl]-4,6-dimethoxy-7-oxo-1-azabicyclo[3.2.0]hept-2-ene-2-carboxylic acid

rac-[4S,5R,6S(R)]-3-[[[[(3-carboxy-1-cyclopropyl-6-fluoro-1,4-dihydro-4-oxo-7-quinolonyl)-1-piperazinyl]-carbonyl]oxy]-methyl]-6-(1-hydroxyethyl)-4-methyl-7-oxo-1-azabicyclo-[3.2.0]hept-2-ene-2-carboxylic acid

rac-[4S,5R,6S(R)]-3-[[[[(9-cyclopropyl-6-fluoro-2,3,4,9-tetrahydro-3,4-dioxoisothiazolo[5,4-b]quinolin-7-yl)-1-piperazinyl]carbonyl]oxy]methyl]-6-(1-hydroxyethyl)-4-methyl-7-oxo-1-azabicyclo[3.2.0]hept-2-ene-2-carboxylic acid

rac-[4S,5R,6S(R)]-3-[[[[[[9-fluoro-3,7-dihydro-3-methyl-7-oxo-2H-pyrido[3,2,1-ij][4,1,2]benzoxadiazin-10-yl]-3-pyrrolidinyl] amino]carbonyl]oxy]methyl]6-(1-hydroxyethyl)-4-methyl-7-oxo-1-azabicyclo[3.2.0]hept-2-ene-2-carboxylic acid

rac-[4S,5R,6S(R)]-3-[[[[[(5-amino-3-carboxy-1-cyclopropyl-6,8-difluoro-1,4-dihydro-4-oxo-7-quinolonyl)-3-pyrrolidinyl]amino] carbonyl]oxy]methyl]-6-(1-hydroxyethyl)-4-methyl-7-oxo-1-azabicyclo[3.2.0]hept-2-ene-2-carboxylic acid

rac-[4S,5R,6S(R)]-3-[[[[[(3-carboxy-1-(2,4-difluorophenyl)-6-fluoro-1,4-dihydro-4-oxo-1,8-naphthyridin-7-yl)-3-pyrrolidinyl]amino]carbonyl]oxy]methyl]-6-[(1-hydroxyethyl)-4-methyl-7-oxo-1-azabicyclo[3.2.0]-hept-2-ene-2-carboxylic acid

rac-[4S,5R,6S(R)]-3[[[[6,8-difluoro-1-(2-fluoroethyl)-1,4-dihydro-4-oxo-7-(4-methyl-1-piperazinyl)-3-quinolonyl]carbonyl]thio]methyl]-6-(1-hydroxyethyl)-4-methyl-7-oxo-1-azabicyclo[3.2.O]hept-2-ene-2-carboxylic acid

rac-[4S,5R,6S(R)]-3[[(3-carboxy-1-cyclopropyl-6-fluoro-1,4-dihydro-4-oxo-7-quinolonyl)-1-piperazinyl]-methyl]-6-(1-hydroxyethyl)-4-methyl-7-oxo-1-azabicyclo[3.2.0]hept-2-ene-2-carboxylic acid

rac-[4S,5R,6S(R)]-4-[3-carboxy-1-cyclopropyl-6-fluoro-1,4-dihydro-4-oxo-7-quinolonyl]-1-[2-carboxy-6-(1-hydroxyethyl)-4-methyl-7-oxo-1-azabicyclo[3.2.0]hept-2-enyl]methyl-1-methylpiperazinium iodide

rac-[4S]-3-[[[[3-carboxy-1-cyclopropyl-6-fluoro-1,4,8,9,10,11,, 11a,12-octahydro-4-oxo-pyrazino[1',2':4,5]-[1,4]oxazino[3,2-h]-quinolin-10-yl]carbonyl]oxy]methyl]-4-methyl-7-oxo-1-azabicyclo[3.2.0]hept-2-ene-2-carboxylic acid

rac-[6R]-3-[[3-carboxy-1-cyclopropyl-6-fluoro-1,4,8,9,10,11, 11a,12-octahydro-4-oxo-pyrazino[1',2':4,5]-[1,4]oxazino[3,2-h]-quinolin-10-yl]methyl]-7-oxo-4,4,6-trimethyl-1-azabicyclo-[3.2.0]hept-2-ene-2-carboxylic acid

rac-[4S,5R,6S(R)]-10-[2-carboxy-6-(1-hydroxyethyl)-4-methyl-7-oxo-1-azabicyclo[3.2.0]hept-2-en-3-yl]-methyl-10-methyl-[3-carboxy-1-cyclopropyl-6-fluoro-1,4,8,9,10,11,11a,12-octahydro-4-oxo-pyrazino-[1',2':4,5][1,4]oxazino[3,2-h]quinolinium] iodide
and their salts and hydrates.

**27.** A compound according to claim 1 selected from the group consisting of
rac-[4S,5R,6S(R)]-3[[[[6,8-difluoro-1-(2-fluoroethyl)-1,4-dihydro-4-oxo-7-(4-methyl-1-piperazinyl)-3-quinolonyl]carbonyl]oxy]methyl]-6-(1-hydroxyethyl)-4-methyl-7-oxo-1-azabicyclo-[3.2.0]hept-2-ene-2-carboxylic acid

rac-[4S,5R,6S(R)]-3-[[[[1-cyclopropyl-6-fluoro-1,4-dihydro-4-oxo-7-(1-piperazinyl)-3-quinolonyl]carbonyl]-oxy]methyl]-6-(1-hydroxy-ethyl)-4-methyl-7-oxo-1-azabicyclo[3.2.0]hept-2-ene-2-carboxylic acid

rac-[4S,5R,6S(R)]-3-[[[[(3-carboxy-1-cyclopropyl-6-fluoro-1,4-dihydro-4-oxo-7-quinolonyl)-1-piperazinyl]-carbonyl]oxy]methyl]-6-(1-hydroxyethyl)-4-methyl-7-oxo-1-azabicyclo-[3.2.0]hept-2-ene-2-carboxylic acid

rac-[4S,5R,6S(R)]-3-[[[[[(3-carboxy-1-cyclopropyl-6,8-difluoro-1,4-dihydro-4-oxo-7-quinolonyl)-3-pyrrolidinyl]amino]carbonyl]oxy]methyl]-6-(1-hydroxyethyl)-4-methyl-7-oxo-1-azabicyclo[3.2.0]hept-2-ene-2-carboxylic acid
and their salts and hydrates.

**28.** rac-[5R,6S(R)]-3-[[[[6,8-Difluoro-1-(2-fluoroethyl)-1,4-dihydro-7-(4-methyl)-1-piperazinyl)-4-oxo-3-quinolonyl]carbonyl]oxy]methyl]-6-(1-hydroxyethyl)-7-oxo-1-azabicyclo-[3.2.0]hept-2-ene-2-carboxylic acid (2,2-dimethyl-1-oxopropoxy)methyl ester.

**29.** rac-[5R,6S(R)]-3-[[[(3-Carboxy-1-cyclopropyl-6-fluoro-1,4-dihydro-4-oxo-7-quinolonyl)-1-piperazinyl]-carbonyl] oxy]methyl-6-[1-hydroxyethyl]-7-oxo-1-azabicyclo[3.2.0]hept-2-ene-2-carboxylic acid-(2,2-dimethyl-1-oxopropoxy)methyl ester.

**30.** Compounds as set forth in any one of claims 1-29 as pharmaceutically active substances.

**31.** Compounds as set forth in any one of claims 1-29 as pharmaceutically active substances for the treatment and prophylaxis of infectious diseases.

**32.** A pharmaceutical preparation containing an end product according to any one of claims 1-29.

**33.** A pharmaceutical preparation for the treatment and prophylaxis of infectious diseases containing an end product according to any one of claims 1-29.

**34.** A process for the manufacture of the compounds according to any one of claims 1-29, which process comprises
(a) for the manufacture of a readily hydrolysable ester of a carboxylic acid of formula I in which Y is

one of groups (i), (ii), (iv), (v) and (vi) reacting a compound of the formula

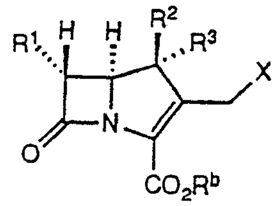

X

wherein $R^1$-$R^3$ are as in claim 1, $R^b$ is the residue of a readily hydrolyzable ester, X is hydrogen and amino, hydroxy and carboxy groups present may be protected;
with a salt of a carboxylic or carbothioic acid bonded to a substituted quinolonyl or naphthyridonyl group;
with a piperazine being unsubstituted or substituted with one or more lower alkyl groups and has a nitrogen atom bonded directly to a substituted quinolonyl or naphthyridonyl group; or
with a salt of a substituted isothiazolo or isoxazolo quinolone or naphthyridone in which the salt formation occurs at a nitrogen atom in the isothiazolo or isoxazolo nucleus;
followed by cleavage of any protecting group present;

or
(b) for the manufacture of a readily hydrolyzable ester of a carboxylic acid of formula I in which Y is group (iii) reacting a compound of the formula

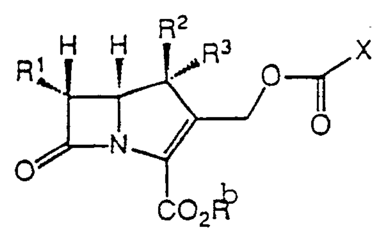

XII

wherein $R^1$-$R^3$, $R^b$ and X are as above and amino, hydroxy and carboxy groups present may be protected,
with a piperazine or pyrrolidine which is unsubstituted or substituted with one or more lower alkyl groups and has a nitrogen atom bonded directly to a substituted quinolonyl or naphthyridonyl group;
followed by cleavage of any protecting group present;

or
(c) for the manufacture of a readily hydrolyzable ester of a carboxylic acid or formula I in which Y is one of groups (i), (ii) and (iii) subjecting a compound of the formula

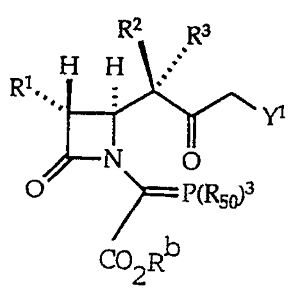

XXII

wherein $R^1$-$R^3$ and $R^b$ are as above. $Y^1$ is an ester or thioester group bonded to a substituted quinolonyl or naphthyridonyl group or a piperazinyl or pyrrolidinyl derived carbamate group which is unsubstituted or substituted with one or more lower alkyl groups and contains a piperazinyl nucleus or a pyrrolidinyl nucleus having a nitrogen atom bonded directly to a substituted quinolonyl or naphthyridonyl group, $R_{50}$ is an aryl or alkyl group and amino, hydroxy and carboxy groups present may be protected,
to thermolysis to effect ring closure;
followed by cleavage of any protecting group present;

or
(d)for the manufacture of a carboxylic acid of formula I converting an ester of the formula

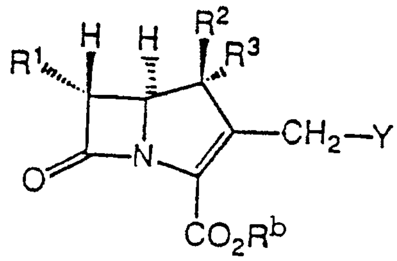

IE

wherein $R^1$-$R^3$ and Y are as above and $R^b$ is a carboxylic acid protecting group,
to the carboxylic acid of formula I,

or
(e) for the manufacture of a readily hydrolyzable ester of a compound of formula I subjecting a carboxylic acid of formula I to a corresponding esterification,

or
(f) for the manufacture of salts or hydrates of a compound of formula I or hydrates of said salts converting a compound of formula I into a salt or hydrate or into a hydrate of said salt.

**35.** The use of the compounds according to any one of claims 1-29 in the treatment or prophylaxis of illnesses.

**36.** The use of the compounds according to any one of claims 1-29 in the treatment or prophylaxis of infectious diseases.

**37.** The use of the compounds according to any one of claims 1-29 for the manufacture of medicaments for the treatment or prophylaxis of infectious diseases.

**Claims for the following Contracting State: ES**

**1.** Process for the manufacture of carbapenem compounds of the general formula

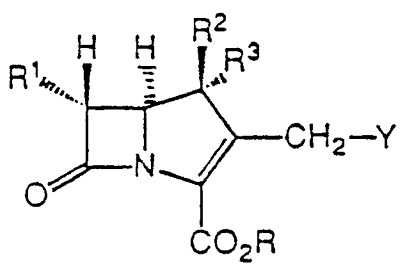

I

wherein R is hydrogen or a carboxylic acid protecting group; $R^1$ is selected from the group

consisting of hydrogen, lower alkyl, lower alkoxy and hydroxyalkyl; $R^2$ and $R^3$ are each independently hydrogen, lower alkyl, lower alkoxy or lower alkylthio; and Y is

(i) an ester group;
(ii) a thioester group;
(iii) a piperazinyl or pyrrolidinyl derived carbamate group;
(iv) a piperazinyl derived amine group;
(v) a piperazinyl derived quaternary ammonium group; or
(vi) a group comprising a covalent bond;

the ester group and the thioester group being bonded to a substituted quinolonyl or naphthyridonyl group; the piperazinyl or pyrrolidinyl derived carbamate group, the piperazinyl derived amine group and the piperazinyl derived quaternary ammonium group containing a piperazinyl nucleus or a pyrrolidinyl nucleus having a nitrogen atom bonded directly to a substituted quinolonyl or naphthyridonyl group; the piperazinyl and the pyrrolidinyl nuclei being unsubstituted or substituted with one or more lower alkyl groups; and the covalent bond of the corresponding group being connected to a substituted isothiazolo or isoxazolo quinolonyl or naphthyridonyl group via a nitrogen atom in an isothiazolo or isoxazolo nucleus;

as well as the corresponding readily hydrolyzable esters, pharmaceutically acceptable salts and hydrates of any of the foregoing compounds, which process comprises

(a) for the manufacture of a readily hydrolyzable ester of a carboxylic acid of formula I in which Y is one of groups (i), (ii), (iv), (v) and (vi) reacting a compound of the formula

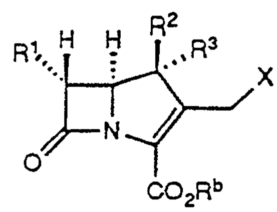

X

wherein $R^1$-$R^3$are as above, $R^b$ is the residue of a readily hydrolysable ester, X is hydrogen, and amino, hydroxy and carboxy groups present may be protected;

with a salt of a carboxylic or carbothioic acid bonded to a substituted quinolonyl or naphthyridonyl group;

with a piperazine being unsubstituted or substituted with one or more lower alkyl groups and has a nitrogen atom bonded directly to a substituted quinolonyl or naphthyridonyl group; or

with a salt of a substituted isothiazolo or isoxazolo quinolone or naphthyridone in which the salt formation occurs at a nitrogen atom in the isothiazolo or isoxazolo nucleus;

followed by cleavage of any protecting group present;

or

(b) for the manufacture of a readily hydrolyzable ester of a carboxylic acid of formula I in which Y is group (iii) reacting a compound of the formula

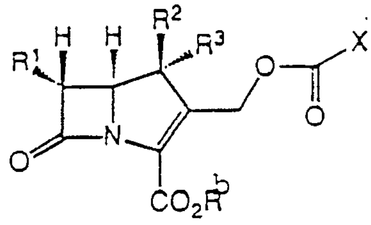

XII

wherein $R^1$-$R^3$, $R^b$ and X are as above and amino, hydroxy and carboxy groups present may be

protected,
with a piperazine or pyrrolidine which is unsubstituted or substituted with one or more lower alkyl groups and has a nitrogen atom bonded directly to a substituted quinolonyl or naphthyridonyl group; followed by cleavage of any protecting group present;

or
(c) for the manufacture of a readily hydrolyzable ester of a carboxylic acid or formula I in which Y is one of groups (i), (ii) and (iii) subjecting a compound of the formula

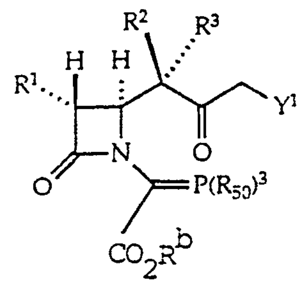

XXII

wherein $R^1$-$R^3$ and $R^b$ are as above, $Y^1$ is an ester or thioester group bonded to a substituted quinolonyl or naphthyridonyl group or a piperazinyl or pyrrolidinyl derived carbamate group which is unsubstituted or substituted with one or more lower alkyl groups and contains a piperazinyl nucleus or a pyrrolidinyl nucleus having a nitrogen atom bonded directly to a substituted quinolonyl or naphthyridonyl groups, $R_{50}$ is an aryl or alkyl group and amino, hydroxy and carboxy groups present may be protected,
to thermolysis to effect ring closure;
followed by cleavage of any protecting group present;

or
(d)for the manufacture of a carboxylic acid of formula I converting an ester of the formula

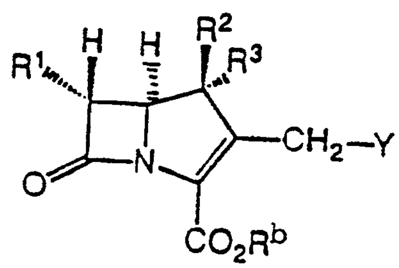

IE

wherein $R^1$-$R^3$ and Y are as above and $R^b$ is a carboxylic acid protecting group,
to the carboxylic acid of formula I,

or
(e) for the manufacture of a readily hydrolysable ester of a compound of formula I subjecting a carboxylic acid of formula I to a corresponding esterification,

or
(f) for the manufacture of salts or hydrates of a compound of formula I or hydrates of said salts converting a compound of formula I into a salt or hydrate or into a hydrate of said salt.

**2.** Process for the manufacture of compounds according to claim 1 wherein Y is an ester of formula

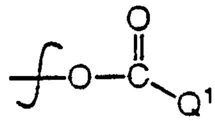

a thioester of formula

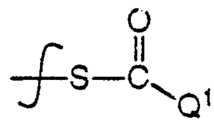

a piperazinyl or pyrrolidinyl derived carbamate of formula

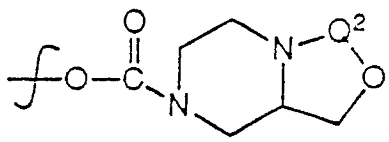

or

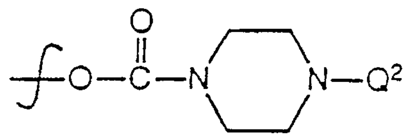

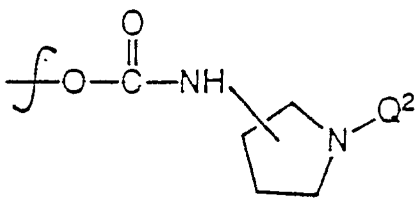

or

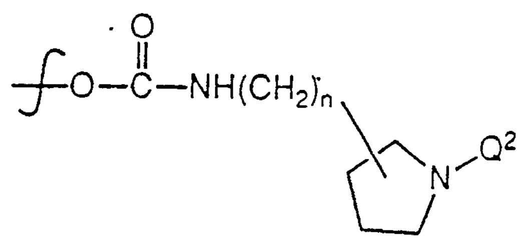

wherein n is 0, 1 or 2.
or a piperazinyl derived amine of formula

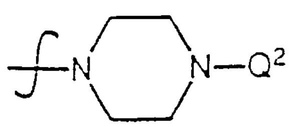

or

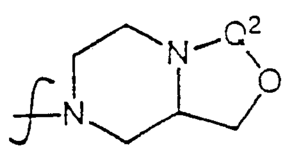

or a piperazinyl derived quaternary ammonium group of formula

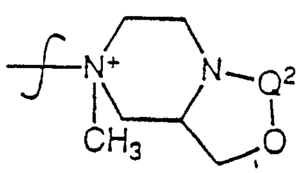

or

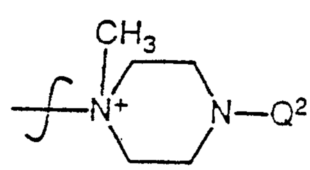

or a group comprising a covalent bond connected to $Q^3$, i.e. a group of formula

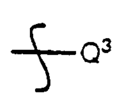

wherein $Q^1$ and $Q^2$ are each independently a substituted quinolonyl or naphthyridonyl group; and $Q^3$ is a substituted isothiazolo or isoxazolo quinolonyl or naphthyridonyl group, characterized in that correspondingly substituted starting materials are used.

3. Process for the manufacture of compounds according to claim 2 wherein Y is the ester of formula

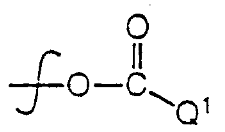

or a piperazinyl derived amine group of formula

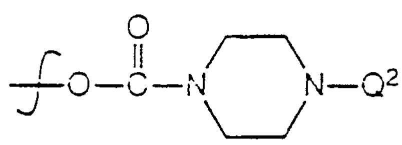

wherein $Q^1$ and $Q^2$ are each independently a substituted quinolonyl or naphthyridonyl group, characterized in that correspondingly substituted starting materials are used.

4. Process for the manufacture of compounds as in claim 2 wherein $Q^1$ is

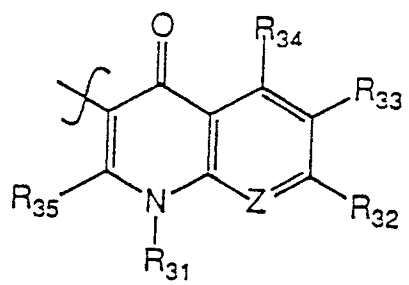

wherein Z represents

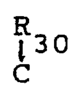

or N, $R_{30}$ is hydrogen or halogen; $R_{31}$ is hydrogen, lower alkyl, lower alkenyl, $C_3$-$C_7$ cycloalkyl, halo-lower-alkyl, phenyl, mono-, di and tri-halophenyl, or

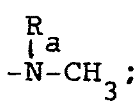

$R_a$ is hydrogen or $R_a$ and $R_{30}$ when taken together represent a lower alkylene mono-oxy group of 1-2 carbon atoms; $R_{30}$ and $R_{31}$ when taken together represent a lower alkylene mono-oxy group of 2-3 carbon atoms; $R_{32}$ represents hydrogen, halogen, lower alkyl, a 5- or 6-membered heterocyclic ring containing one or two heteroatoms selected from the group consisting of O, N and S; or when $R_{32}$ is a piperazine, $R_{30}$ is hydrogen, halogen or a methyleneoxy ($-CH_2O$) bridge to the piperazine of $R_{32}$ to form a fused six-membered ring; $R_{33}$ is hydrogen or halogen; $R_{32}$ and $R_{33}$ when taken together represent a $C_1$-$C_2$ lower alkylene dioxy group; $R_{34}$ is hydrogen or amino; and $R_{35}$ is hydrogen, lower alkoxy or lower alkylthio, characterized in that correspondingly substituted starting materials are used.

**5.** Process for the manufacture of compounds as in claim 4 wherein Z is

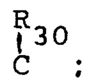

$R_{30}$ is hydrogen or halogen, $R_{31}$ is lower alkyl, $C_3$-$C_7$ cycloalkyl, halo-lower alkyl or mono-, di- or tri-halophenyl; $R_{32}$ is a piperazinyl or a pyrrolidinyl group; $R_{33}$ is halogen; $R_{34}$ is hydrogen or amino; and $R_{35}$ is hydrogen, lower alkoxy or lower alkylthio, characterized in that correspondingly substituted starting materials are used.

**6.** Process for the manufacture of compounds as in claim 5 wherein $R_{30}$ is hydrogen, fluorine or chlorine; $R_{31}$ is ethyl, fluoroethyl, cyclopropyl or fluorophenyl; $R_{32}$ is a piperazinyl, N-methyl piperazinyl or aminopyrrolidinyl group; $R_{33}$ is fluoro; $R_{34}$ is hydrogen; and $R_{35}$ is hydrogen, characterized in that correspondingly substituted starting materials are used.

**7.** Process for the manufacture of compounds according to claim 2 wherein $Q^2$ is

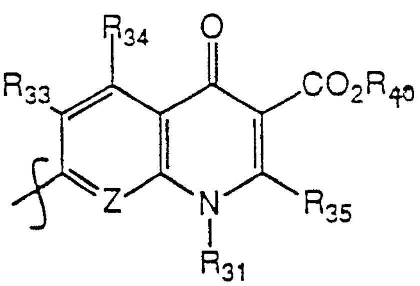

or

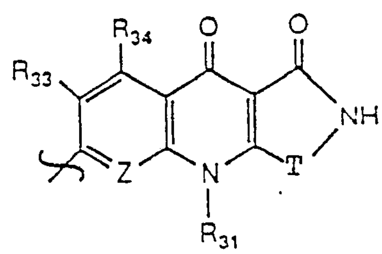

wherein Z represents

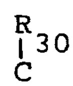

or N;
$R_{30}$ is hydrogen, halogen or a methyleneoxy ($-CH_2O-$) bridge to the piperazine nucleus of Y to form a fused six-membered ring;
$R_{31}$ represents hydrogen, lower alkyl, lower alkenyl, $c_3$-$c_7$ cycloalkyl, halo-lower-alkyl, phenyl, mono-, di- or tri-halophenyl or

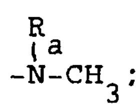

$R_a$ is hydrogen or $R_a$ and $R_{31}$ when taken together represent a lower alkylene mono-oxy group of 1-2 carbon atoms;
$R_{30}$ and $R_{31}$ when taken together represent a lower alkylene mono-oxy group of 2-3 carbon atoms;
$R_{33}$ is hydrogen or halogen;
$R_{34}$ is hydrogen or amino;
$R_{35}$ is hydrogen, lower alkoxy or lower alkylthio;
$R_{40}$ is hydrogen or a carboxylic acid protecting group; and
T is O or S, characterized in that correspondingly substituted starting materials are used.

**8.** Process for the manufacture of compounds according to claim 7 wherein Z is

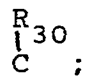

$R_{30}$ is hydrogen or halogen; $R_{31}$ is lower alkyl, $C_3$-$C_7$ cycloalkyl; halo-lower-alkyl, mono-, di- or tri-halophenyl; $R_{32}$ is a piperazinyl or pyrrolidinyl group; $R_{33}$ is halogen; $R_{34}$ is hydrogen or amino; $R_{35}$ is hydrogen; $R_{40}$ is a hydrogen or a carboxylic acid protecting group; and T is O or S, characterized in that correspondingly substituted starting materials are used.

9. Process for the manufacture of compounds according to claim 8 wherein Z is

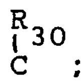

$R_{30}$ is hydrogen, fluoro or chloro; $R_{31}$ is ethyl, fluoroethyl, cyclopropyl or fluorophenyl; $R_{32}$ is a piperazinyl group, a N-methyl piperazinyl group or an aminopyrrolidinyl group; $R_{33}$ is fluoro-; $R_{34}$ is hydrogen; $R_{35}$ is hydrogen; $R_{40}$ is hydrogen; and T is O or S, characterized in that correspondingly substituted starting materials are used.

10. Process for the manufacture of compounds according to claim 2 wherein $Q^3$ is

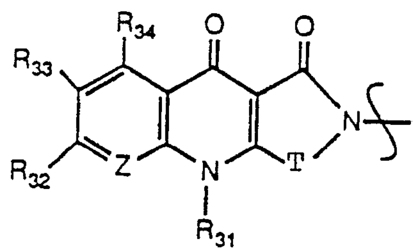

wherein Z represents

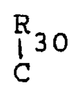

or N;
$R_{30}$ is hydrogen or halogen;
$R_{31}$ is hydrogen, lower alkyl, lower alkenyl, $C_3$-$C_7$ cycloalkyl, halo-lower-alkyl, phenyl, mono-, di- or tri-halophenyl, or

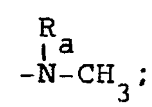

$R_a$ and $R_{30}$ when taken together represent a lower alkylene mono-oxy group of 1-2 carbon atoms;
$R_{30}$ and $R_{31}$ when taken together represent a lower alkylene mono-oxy group of 2-3 carbon atoms;
$R_{32}$ represents hydrogen, halogen, lower alkyl, a 5- or 6-membered heterocyclic ring containing one or two heteroatoms selected from the group consisting of O, N and S; when $R_{32}$ is a piperazinyl group, $R_{30}$ is hydrogen, halogen or a methyleneoxy ($-CH_2O-$) bridge to a piperazinyl nucleus of $R_{32}$ to form a fused six-membered ring;
$R_{33}$ represents hydrogen and halogen;
$R_{32}$ and $R_{33}$ when taken together represent a lower alkylene dioxy group having 1-2 carbon atoms;

$R_{34}$ is hydrogen or amino; and T is O or S, characterized in that correspondingly substituted starting materials are used.

**11.** Process for the manufacture of compounds according to claim 10 wherein Z is

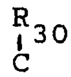 ;

$R_{30}$ is hydrogen or halogen; $R_{31}$ is lower alkyl, $C_3$-$C_7$ cycloalkyl, halo-lower-alkyl, mono-, di or tri-phenyl; $R_{32}$ is a piperazinyl or a pyrrolidinyl group, $R_{33}$ is halogen; $R_{34}$ is hydrogen; and T is O or S, characterized in that correspondingly substituted starting materials are used.

**12.** Process for the manufacture of compounds according to claim 11 wherein $R_{30}$ is hydrogen, fluoro or chloro; $R_{31}$ is ethyl, fluoroethyl, cyclopropyl or fluorophenyl; $R_{32}$ is a piperazinyl, N-methyl-piperazinyl or amino pyrrolidinyl group; $R_{33}$ is fluoro; $R_{34}$ is hydrogen; and T is O or S, characterized in that correspondingly substituted starting materials are used.

**13.** Process for the manufacture of compounds according to claim 1 wherein $R^1$ is hydrogen, lower alkyl, lower alkoxy or hydroxyalkyl, characterized in that correspondingly substituted starting materials are used.

**14.** Process for the manufacture of compounds according to claim 13 wherein $R^1$ is hydroxyalkyl, characterized in that correspondingly substituted starting materials are used.

**15.** Process for the manufacture of compounds according to claim 1 wherein $R^2$ and $R^3$ are each independently hydrogen or lower alkyl, characterized in that correspondingly substituted starting materials are used.

**16.** Process for the manufacture of compounds according to claim 15 wherein $R^2$ and $R^3$ are each hydrogen, characterized in that correspondingly substituted starting materials are used.

**17.** Process for the manufacture of compounds according to claim 15 wherein $R^2$ is lower alkyl and $R^3$ is hydrogen, characterized in that correspondingly substituted starting materials are used.

**18.** Process for the manufacture of compounds according to claim 1 having a formula

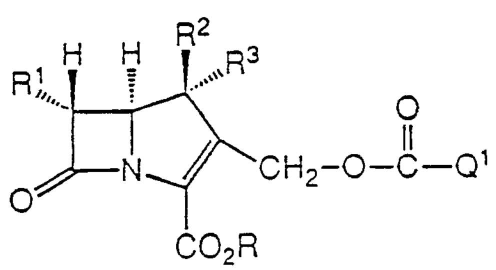

IA

wherein R is hydrogen or a carboxylic acid protecting group; $R^1$ is selected from the group consisting of hydrogen, lower alkyl, lower alkoxy and hydroxyalkyl; $R^2$ and $R^3$ are each independently hydrogen, lower alkyl, lower alkoxy or lower alkylthio and $Q^1$ is formula

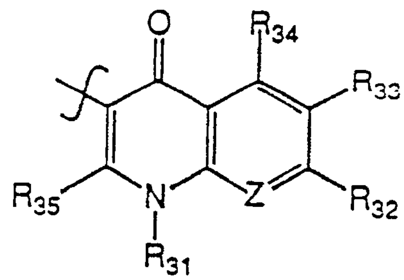

wherein Z represents

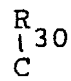

or N, $R_{30}$ is hydrogen or halogen; $R_{31}$ is hydrogen, lower alkyl, lower alkenyl, $C_3$-$C_7$ cycloalkyl, halo-lower-alkyl, phenyl, mono-, di and tri-halophenyl, or

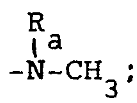

$R_a$ is hydrogen or $R_a$ and $R_{30}$ when taken together represent a lower alkylene mono-oxy group of 1-2 carbon atoms; $R_{30}$ and $R_{31}$ when taken together represent a lower alkylene mono-oxy group of 2-3 carbon atoms; $R_{32}$ represents hydrogen, halogen, lower alkyl, a 5- or 6-membered heterocyclic ring containing one or two heteroatoms selected from the group consisting of O, N and S; or when $R_{32}$ is a piperazine, $R_{30}$ is hydrogen, halogen or a methyleneoxy ($-CH_2O-$) bridge to the piperazine of $R_{32}$ to form a fused six-membered ring; $R_{33}$ is hydrogen or halogen; $R_{32}$ and $R_{33}$ when taken together represent a $C_1$-$C_2$ lower alkylene dioxy group; $R_{34}$ is hydrogen or amino; and $R_{35}$ is hydrogen, lower alkoxy or lower alkylthio, characterized in that correspondingly substituted starting materials are used.

19. Process for the manufacture of compounds according to claim 18 wherein Z is

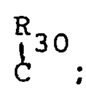

$R_{30}$ is hydrogen or halogen, $R_{31}$ is lower alkyl, $C_3$-$C_7$ cycloalkyl halo-lower-alkyl or halophenyl; $R_{32}$ is a piperazinyl or pyrrolidinyl group; $R_{33}$ is halogen; and $R_{34}$ is hydrogen, characterized in that correspondingly substituted starting materials are used.

20. Process for the manufacture of compounds according to claim 1 of formula

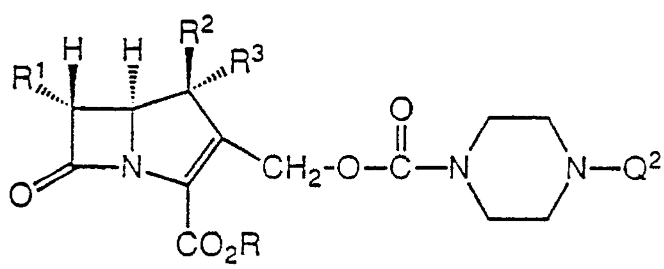

IB

wherein R is hydrogen or a carboxylic acid protecting group; $R^1$ is selected from the group consisting

of hydrogen, lower alkyl, lower alkoxy and hydroxyalkyl; $R^2$ and $R^3$ are each independently hydrogen, lower alkyl, lower alkoxy or lower alkylthio and $Q^2$ is of formula

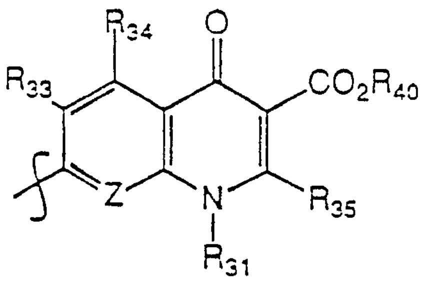

or

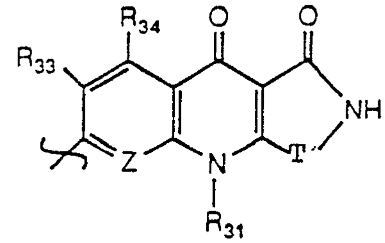

wherein Z represents

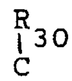

or N;

$R_{30}$ is hydrogen, halogen or a methyleneoxy ($-CH_2O-$) bridge to the piperazine nucleus of Y to form a fused six-membered ring;

$R_{31}$ represents hydrogen, lower alkyl, lower alkenyl, $C_3$-$C_7$ cycloalkyl, halo-lower-alkyl, phenyl, mono-, di-and tri-halophenyl or

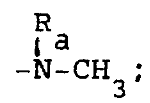

$R_a$ is hydrogen or $R_a$ and $R_{31}$ when taken together represent a lower alkylene mono-oxy group of 1-2 carbon atoms;

$R_{30}$ and $R_{31}$ when taken together represent a lower alkylene mono-oxy group of 2-3 carbon atoms;

$R_{33}$ is hydrogen or halogen;

$R_{34}$ is hydrogen or amino;

$R_{35}$ is hydrogen, lower alkoxy or lower alkylthio;

$R_{40}$ is hydrogen or a carboxylic acid protecting group; and

T is O or S, characterized in that correspondingly substituted starting materials are used.

**21.** Process for the manufacture of compounds according to claim 1 of formula

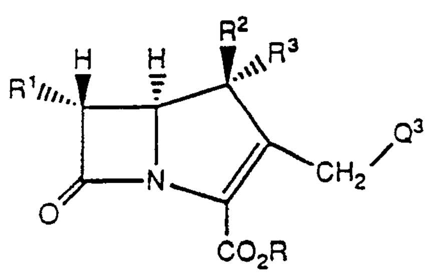

IC

wherein R is hydrogen or a carboxylic acid protecting group; $R^1$ is selected from the group consisting of hydrogen, lower alkyl, lower alkoxy and hydroxyalkyl; $R^2$ and $R^3$ are each independently hydrogen, lower alkyl, lower alkoxy or lower alkylthio and $Q^3$ is of formula

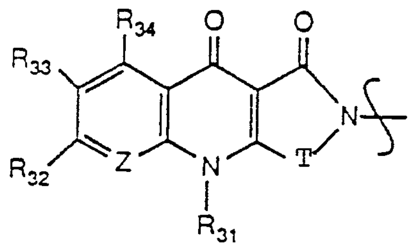

wherein Z represents

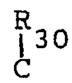

or N;
$R_{30}$ is hydrogen or halogen;
$R_{31}$ is hydrogen, lower alkyl, lower alkenyl, $C_3$ -$C_7$ cycloalkyl, halo-lower-alkyl, phenyl, mono-, di- or trihalophenyl, or

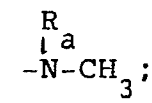

$R_a$ and $R_{30}$ when taken together represent a lower alkylene mono-oxy group of 1-2 carbon atoms;
$R_{30}$ and $R_{31}$ when taken together represent a lower alkylene mono-oxy group of 2-3 carbon atoms;
$R_{32}$ represents hydrogen, halogen, lower alkyl, a 5- or 6-membered heterocyclic ring containing one or two heteroatoms selected from the group consisting of O, N and S; when $R_{32}$ is a piperazinyl group, $R_{30}$ is hydrogen, halogen or a methyleneoxy (-$CH_2O$-) bridge to a piperazinyl nucleus of $R_{32}$ to form a fused six-membered ring;
$R_{33}$ represents hydrogen and halogen;
$R_{32}$ and $R_{33}$ when taken together represent a lower alkylene dioxy group having 1-2 carbon atoms;
$R_{34}$ is hydrogen or amino; and T is O or S, characterized in that correspondingly substituted starting materials are used.

**22.** Process for the manufacture of compounds according to claim 21 wherein Z is

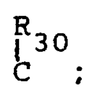

$R_{30}$ is hydrogen or halogen; $R_{31}$ is lower alkyl, $C_3$-$C_7$ cycloalkyl, halo-lower-alkyl or mono-, di- or trihalo-phenyl; $R_{32}$ is a piperazinyl or a pyrrolidinyl group, $R_{33}$ is halogen; $R_{34}$ is hydrogen; and T is O or S, characterized in that correspondingly substituted starting materials are used.

**23.** Process for the manufacture of compounds according to claim 22, rac-[4S,5R,6S(R)]-3-[[9-cyclopropyl-6-fluoro-7-(1-piperazinyl)-2,3,4,9-tetrahydro-3,4-dioxoisothiazolo [5,4-b]quinolin-2-yl)]methyl]-6-(1-hydroxyethyl)-4-methyl-7-oxo-1-azabicyclo[3.2.0]hept-2-ene-2-carboxylic acid and its salts and hydrates, characterized in that correspondingly substituted starting materials are used.

**24.** Process for the manufacture of compounds according to claim 1, rac-[5R,6S(R)]-3-[[[[6,8-difluoro-1-(2-fluoro-ethyl)-1,4-dihydro-4-oxo-7-(4-methyl-1-piperazinyl)-3-quinolonyl]carbonyl]oxy]methyl]-6-(1-hydroxyethyl)-7-oxo-1-azabicyclo-[3.2.0]hept-2-ene-2-carboxylic acid and its salts and hydrates characterized in that correspondingly substituted starting materials are used.

**25.** Process for the manufacture of compounds according to claim 1, rac-[5R,6S(R)]-3-[[[[(3-carboxy-1-cyclopropyl-6-fluoro-1,4-dihydro-4-oxo-7-quinolonyl)-1-piperazinyl]carbonyl] oxy]methyl]-6-(1-hydroxyethyl)-7-oxo-1-azabicyclo[3.2.0]hept-2-ene-2-carboxylic acid and its salts and hydrates characterized in that correspondingly substituted starting materials are used.

**26.** Process for the manufacture of compounds according to claim 1 selected from the group consisting of

rac-[4S,5S,6R]-3-[[[[1-cyclopropyl-6-fluoro-1,4-dihydro-4-oxo-7-(1-piperazinyl)-3-quinolonyl]carbonyl]-oxy]methyl]-6-ethyl-4-thiomethyl-7-oxo-1-azabicyclo[3.2.0]hept-2-ene-2-carboxylic acid

rac-3-[[[[7-(3-amino-1-pyrrolidinyl)-1-cyclopropyl-6,8-difluoro-1,4-dihydro-4-oxo-3-quinolonyl]carbonyl]-oxy]methyl]-4,4-dimethyl-7-oxo-1-azabicyclo[3.2.0]hept-2-ene-2-carboxylic acid

rac-[4S,5R,6R]-3-[[[[1-cyclopropyl-6,8-difluoro-1,4-dihydro-4-oxo-7-(4-pyridinyl)-3-quinolonyl]carbonyl]-oxy]methyl]-4,6-dimethoxy-7-oxo-1-azabicyclo[3.2.0]hept-2-ene-2-carboxylic acid

rac-[4S,5R,6S(R)]-3-[[[[5-amino-7-(3-amino-1-pyrrolidinyl)-1-cyclopropyl-6,8-difluoro-1,4-dihydro-4-oxo-3-quinolonyl]carbonyl]oxy]methyl]-6-(1-hydroxyethyl)-4-methyl-7-oxo-1-azabicyclo[3.2.0]hept-2-ene-2-carboxylic acid

rac-[4S,5R,6S(R)]-3-[[[[10-(3-amino-1-pyrrolindinyl)-9-fluoro-3,7-dihydro-3-methyl-7-oxo-2H-pyrido[3,2,1-ij][4,1,2]benzoxadiazin-6-yl]carbonyl]oxy]methyl]-6-(1-hydroxyethyl)-4-methyl-7-oxo-1-azabicyclo[3.2.0]-hept-2-ene-2-carboxylic acid

rac-[4S,5R,6S(R)]-3-[[[[6-fluoro-1-ethyl-1,4-dihydro-4-oxo-7-(1-piperazinyl)-1,8-naphthridin-3-yl]carbonyl]-oxy]methyl]-6-(1-hydroxyethyl)-4-methyl-7-oxo-1-azabicyclo[3.2.0]hept-2-ene-2-carboxylic acid

rac-[4S,5R,6S(R)]-3-[[[[9-fluoro-10-(4-methyl-1-piperazinyl)-3,7-dihydro-3-methyl-7-oxo-2H-pyrido[1,2,3-de]-1,4-benzoxazin-6-yl]carbonyl]oxy]methyl]-6-(1-hydroxyethyl)-4-methyl-7-oxo-1-azabicyclo[3.2.0]-hept-2-ene-2-carboxylic acid

rac-[4S,5S,6R]-3-[[[[[(3-carboxy-1-cyclopropyl-6-fluoro-1,4-dihydro-4-oxo-7-quinolonyl)-3-pyrrolidinyl]-amino]carbonyl]oxy] methyl]-6-ethyl-4-thiomethyl-7-oxo-1-azabicyclo[3.2.0]hept-2-ene-2-carboxylic acid

rac-3-[[[[[(3-carboxy-1-cyclopropyl-6,8-difluoro-1,4-dihydro-4-oxo-7-quinolonyl)-3-pyrrolidinyl]amino]-carbonyl]oxy]methyl]-4-dimethyl-7-oxo-1-azabicyclo[3.2.0]hept-2-ene-2-carboxylic acid

rac-[4S,5R,6R]-3-[[[[(3-carboxy-1-ethyl-6-fluoro-1,4-dihydro-4-oxo-1,8-naphthyridin-7-yl)-1-piperazinyl]-carbonyl]oxy]methyl]-4,6-dimethoxy-7-oxo-1-azabicyclo[3.2.0]hept-2-ene-2-carboxylic acid

rac-[4S,5R,6S(R)]-3-[[[[(3-carboxy-1-cyclopropyl-6-fluoro-1,4-dihydro-4-oxo-7-quinolonyl)-1-piperazinyl]-carbonyl]oxy]methyl]-6-(1-hydroxyethyl)-4-methyl-7-oxo-1-azabicyclo-[3.2.0]hept-2-ene-2-carboxylic acid

rac-[4S,5R,6S(R)]-3-[[[[(9-cyclopropyl-6-fluoro-2,3,4,9-tetrahydro-3,4-dioxoisothiazolo[5,4-b]quinolin-7-yl)-1-piperazinyl]carbonyl]oxy]methyl]-6-(1-hydroxyethyl)-4-methyl-7-oxo-1-azabicyclo[3.2.0]hept-2-ene-2-carboxylic acid

rac-[4S,5R,6S(R)]-3-[[[[[[9-fluoro-3,7-dihydro-3-methyl-7-oxo-2H-pyrido[3,2,1-ij][4,1,2]benzoxadiazin-10-yl]-3-pyrrolidinyl] amino]carbonyl]oxy]methyl]6-(1-hydroxyethyl)-4-methyl-7-oxo-1-azabicyclo[3.2.0]hept-2-ene-2-carboxylic acid

rac-[4S,5R,6S(R)]-3-[[[[[(5-amino-3-carboxy-1-cyclopropyl-6,8-difluoro-1,4-dihydro-4-oxo-7-quinolonyl)-3-pyrrolidinyl]amino] carbonyl]oxy]methyl]-6-(1-hydroxyethyl)-4-methyl-7-oxo-1-azabicyclo[3.2.0]hept-2-ene-2-carboxylic acid

rac-[4S,5R,6S(R)]-3-[[[[[(3-carboxy-1-(2,4-difluorophenyl)-6-fluoro-1,4-dihydro-4-oxo-1,8-naphthyridin-7-yl)-3-pyrrolidinyl]amino]carbonyl]oxy]methyl]-6-[(1-hydroxyethyl)-4-methyl-7-oxo-1-azabicyclo[3.2.0]-

hept-2-ene-2-carboxylic acid

rac-[4S,5R,6S(R)]-3[[[[6,8-difluoro-1-(2-fluoroethyl)-1,4-dihydro-4-oxo-7-(4-methyl-1-piperazinyl)-3-quinolonyl]carbonyl]thio]methyl]-6-(1-hydroxyethyl)-4-methyl-7-oxo-1-azabicyclo[3.2.0]hept-2-ene-2-carboxylic acid

rac-[4S,5R,6S(R)]-3[[(3-carboxy-1-cyclopropyl-6-fluoro-1,4-dihydro-4-oxo-7-quinolonyl)-1-piperazinyl]-methyl]-6-(1-hydroxyethyl)-4-methyl-7-oxo-1-azabicyclo[3.2.0]hept-2-ene-2-carboxylic acid

rac-[4S,5R,6S(R)]-4-[3-carboxy-1-cyclopropyl-6-fluoro-1,4-dihydro-4-oxo-7-quinolonyl]-1-[2-carboxy-6-(1-hydroxyethyl)-4-methyl-7-oxo-1-azabicyclo[3.2.0]hept-2-enyl]methyl-1-methylpiperazinium iodide

rac-[4S]-3-[[[[3-carboxy-1-cyclopropyl-6-fluoro-1,4,8,9,10,11, 11a,12-octahydro-4-oxo-pyrazino[1',2':4,5]-[1,4]oxazino[3,2-h]-quinolin-10-yl]carbonyl]oxy]methyl]-4-methyl-7-oxo-1-azabicyclo[3.2.0]hept-2-ene-2-carboxylic acid

rac-[6R]-3-[[3-carboxy-1-cyclopropyl-6-fluoro-1,4,8,9,10,11, 11a,12-octahydro-4-oxo-pyrazino[1',2':4,5]-[1,4]oxazino[3,2-h]-quinolin-10-yl]methyl]-7-oxo-4,4,6-trimethyl-1-azabicyclo-[3.2.0]hept-2-ene-2-carboxylic acid

rac-[4S,5R,6S(R)]-10-[2-carboxy-6-(1-hydroxyethyl)-4-methyl-7-oxo-1-azabicyclo[3.2.0]hept-2-en-3-yl]-methyl-10-methyl-[3-carboxy-1-cyclopropyl-6-fluoro-1,4,8,9,10,11,11a,12-octahydro-4-oxo-pyrazino-[1',2':4,5][1,4]oxazino[3,2-h]quinolinium] iodide
and their salts and hydrates, characterized in that correspondingly substituted starting materials are used.

**27.** Process for the manufacture of a compound according to claim 1 selected from the group consisting of rac-[4S,5R,6S(R)]-3[[[[6,8-difluoro-1-(2-fluoroethyl)-1,4-dihydro-4-oxo-7-(4-methyl-1-piperazinyl)-3-quinolonyl]carbonyl]oxy]methyl]-6-(1-hydroxyethyl)-4-methyl-7-oxo-1-azabicyclo-[3.2.0]hept-2-ene-2-carboxylic acid

rac-[4S,5R,6S(R)]-3-[[[[1-cyclopropyl-6-fluoro-1,4-dihydro-4-oxo-7-(1-piperazinyl)-3-quinolonyl]carbonyl]-oxy]methyl]-6-(1-hydroxy-ethyl)-4-methyl-7-oxo-1-azabicyclo[3.2.0]hept-2-ene-2-carboxylic acid

rac-[4S,5R,6S(R)]-3-[[[[(3-carboxy-1-cyclopropyl-6-fluoro-1,4-dihydro-4-oxo-7-quinolonyl)-1-piperazinyl]-carbonyl]oxy]methyl]-6-(1-hydroxyethyl)-4-methyl-7-oxo-1-azabicyclo-[3.2.0]hept-2-ene-2-carboxylic acid

rac-[4S,5R,6S(R)]-3-[[[[[(3-carboxy-1-cyclopropyl-6,8-difluoro-1,4-dihydro-4-oxo-7-quinolonyl)-3-pyrrolidinyl]amino]carbonyl]oxy]methyl]-6-(1-hydroxyethyl)-4-methyl-7-oxo-1-azabicyclo[3.2.0]hept-2-ene-2-carboxylic acid,
and their salts and hydrates, characterized in that correspondingly substituted starting materials are used.

**28.** Process for the manufacture of rac-[5R,6S(R)]-3-[[[[6,8-difluoro-1-(2-fluoroethyl)-1,4-dihydro-7-(4-methyl)-1-piperazinyl)-4-oxo-3-quinolonyl]carbonyl]oxy]methyl]-6-(1-hydroxyethyl)-7-oxo-1-azabicyclo[3.2.0]hept-2-ene-2-carboxylic acid (2,2-dimethyl-1-oxo-propoxy)methyl ester, characterized in that correspondingly substituted starting materials are used.

**29.** Process for the manufacture of rac-[5R,6S(R)]-3-[[[(3-carboxy-1-cyclopropyl-6-fluoro-1,4-dihydro-4-oxo-7-quinolonyl)-1-piperazinyl]-carbonyl] oxy]methyl-6-[1-hydroxyethyl]-7-oxo-1-azabicyclo[3.2.0]hept-2-ene-2-carboxylic acid-(2,2-dimethyl-1-oxopropoxy)methyl ester, characterized in that correspondingly substituted starting materials are used.

**30.** The use of the end products defined in any one of claims 1-29 in the treatment or prophylaxis of illnesses.

31. The use of the end products defined in any one of claims 1-29 in the treatment or prophylaxis of infectious diseases.

32. The use of the end products defined in any one of claims 1-29 for the manufacture of medicaments for the treatment or prophylaxis of infectious diseases.

**Claims for the following Contracting State: GR**

1. Process for the manufacture of carbapenem compounds of the general formula

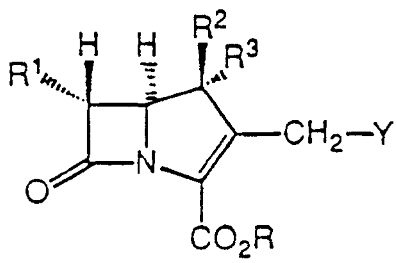

I

wherein R is hydrogen or a carboxylic acid protecting group; $R^1$ is selected from the group consisting of hydrogen, lower alkyl, lower alkoxy and hydroxyalkyl; $R^2$ and $R^3$ are each independently hydrogen, lower alkyl, lower alkoxy or lower alkylthio; and Y is
(i) an ester group;
(ii) a thioester group;
(iii) a piperazinyl or pyrrolidinyl derived carbamate group;
(iv) a piperazinyl derived amine group;
(v) a piperazinyl derived quaternary ammonium group; or
(vi) a group comprising a covalent bond;
the ester group and the thioester group being bonded to a substituted quinolonyl or naphthyridonyl group; the piperazinyl or pyrrolidinyl derived carbamate group, the piperazinyl derived amine group and the piperazinyl derived quaternary ammonium group containing a piperazinyl nucleus or a pyrrolidinyl nucleus having a nitrogen atom bonded directly to a substituted quinolonyl or naphthyridonyl group; the piperazinyl and the pyrrolidinyl nuclei being unsubstituted or substituted with one or more lower alkyl groups; and the covalent bond of the corresponding group being connected to a substituted isothiazolo or isoxazolo quinolonyl or naphthyridonyl group via a nitrogen atom in an isothiazolo or isoxazolo nucleus;
as well as the corresponding readily hydrolyzable esters, pharmaceutically acceptable salts and hydrates of any of the foregoing compounds, which process comprises
(a) for the manufacture of a readily hydrolyzable ester of a carboxylic acid of formula I in which Y is one of groups (i), (ii), (iv), (v) and (vi) reacting a compound of the formula

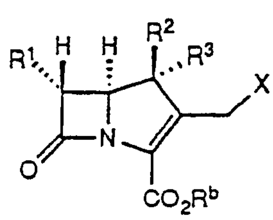

X

wherein $R^1$-$R^3$ are as above, $R^b$ is the residue of a readily hydrolyzable ester, X is hydrogen, and amino, hydroxy and carboxy groups present may be protected;
with a salt of a carboxylic or carbothioic acid bonded to a substituted quinolonyl or naphthyridonyl group;
with a piperazine being unsubstituted or substituted with one or more lower alkyl groups and has a

nitrogen atom bonded directly to a substituted quinolonyl or naphthyridonyl group; or with a salt of a substituted isothiazolo or isoxazolo quinolone or naphthyridone in which the salt formation occurs at a nitrogen atom in the isothiazolo or isoxazolo nucleus; followed by cleavage of any protecting group present;

or

(b) for the manufacture of a readily hydrolyzable ester of a carboxylic acid of formula I in which Y is group (iii) reacting a compound of the formula

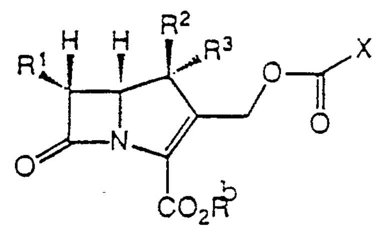

XII

wherein $R^1$-$R^3$, $R^b$ and X are as above and amino, hydroxy and carboxy groups present may be protected, with a piperazine or pyrrolidine which is unsubstituted or substituted with one or more lower alkyl groups and has a nitrogen atom bonded directly to a substituted quinolonyl or naphthyridonyl group; followed by cleavage of any protecting group present;

or

(c) for the manufacture of a readily hydrolyzable ester of a carboxylic acid or formula I in which Y is one of groups (i), (ii) and (iii) subjecting a compound of the formula

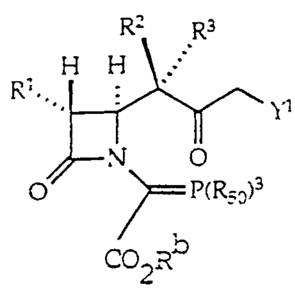

XXII

wherein $R^1$-$R^3$ and $R^b$ are as above, $Y^1$ is an ester or thioester group bonded to a substituted quinolonyl or naphthyridonyl group or a piperazinyl or pyrrolidinyl derived carbamate group which is unsubstituted or substituted with one or more lower alkyl groups and contains a piperazinyl nucleus or a pyrrolidinyl nucleus having a nitrogen atom bonded directly to a substituted quinolonyl or naphthyridonyl groups, $R_{50}$ is an aryl or alkyl group and amino, hydroxy and carboxy groups present may be protected, to thermolysis to effect ring closure; followed by cleavage of any protecting group present;

or

(d)for the manufacture of a carboxylic acid of formula I converting an ester of the formula

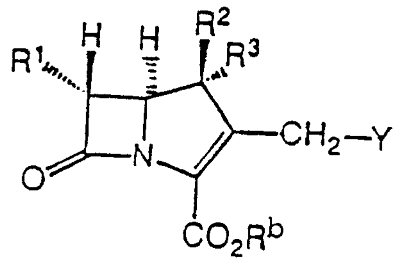

IE

wherein $R^1$-$R^3$ and Y are as above and $R^b$ is a carboxylic acid protecting group, to the carboxylic acid of formula I,

or

(e) for the manufacture of a readily hydrolyzable ester of a compound of formula I subjecting a carboxylic acid of formula I to a corresponding esterification,

or

(f) for the manufacture of salts or hydrates of a compound of formula I or hydrates of said salts converting a compound of formula I into a salt or hydrate or into a hydrate of said salt.

**2.** Process for the manufacture of compounds according to claim 1 wherein Y is an ester of formula

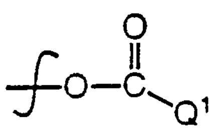

a thioester of formula

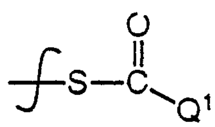

a piperazinyl or pyrrolidinyl derived carbamate of formula

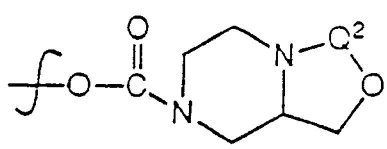

or

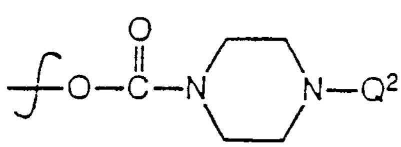

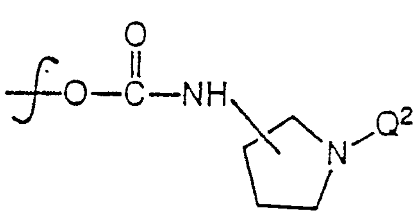

or

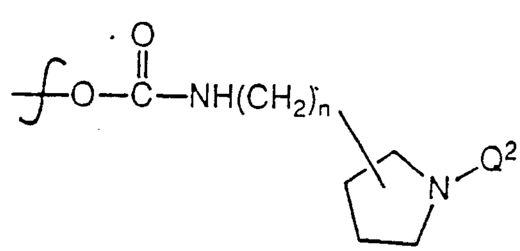

wherein n is 0, 1 or 2.
or a piperazinyl derived amine of formula

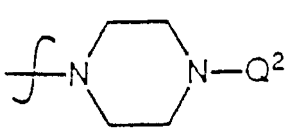

or

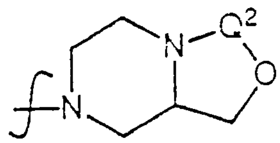

or a piperazinyl derived quaternary ammonium group of formula

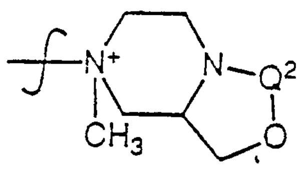

or

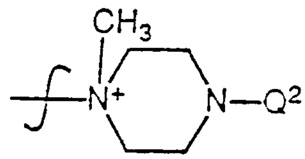

or a group comprising a covalent bond connected to $Q^3$, i.e. a group of formula

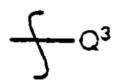

wherein $Q^1$ and $Q^2$ are each independently a substituted quinolonyl or naphthyridonyl group; and $Q^3$ is a substituted isothiazolo or isoxazolo quinolonyl or naphthyridonyl group, characterized in that correspondingly substituted starting materials are used.

3. Process for the manufacture of compounds according to claim 2 wherein Y is the ester of formula

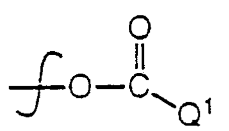

or a piperazinyl derived amine group of formula

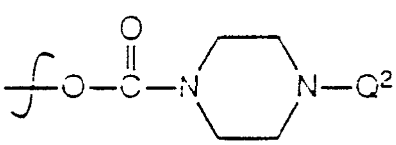

wherein $Q^1$ and $Q^2$ are each independently a substituted quinolonyl or naphthyridonyl group, characterized in that correspondingly substituted starting materials are used.

4. Process for the manufacture of compounds as in claim 2 wherein $Q^1$ is

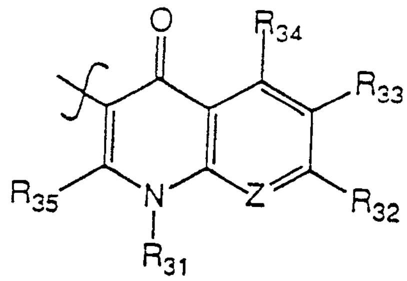

wherein Z represents

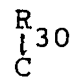

or N, $R_{30}$ is hydrogen or halogen; $R_{31}$ is hydrogen, lower alkyl, lower alkenyl, $C_3$-$C_7$ cycloalkyl, halo-lower-alkyl, phenyl, mono-, di and tri-halophenyl, or

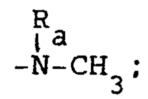

$R_a$ is hydrogen or $R_a$ and $R_{30}$ when taken together represent a lower alkylene mono-oxy group of 1-2 carbon atoms; $R_{30}$ and $R_{31}$ when taken together represent a lower alkylene mono-oxy group of 2-3 carbon atoms; $R_{32}$ represents hydrogen, halogen, lower alkyl, a 5- or 6-membered heterocyclic ring containing one or two heteroatoms selected from the group consisting of O, N and S; or when $R_{32}$ is a piperazine, $R_{30}$ is hydrogen, halogen or a methyleneoxy ($-CH_2O$) bridge to the piperazine of $R_{32}$ to form a fused six-membered ring; $R_{33}$ is hydrogen or halogen; $R_{32}$ and $R_{33}$ when taken together represent a $C_1$-$C_2$ lower alkylene dioxy group; $R_{34}$ is hydrogen or amino; and $R_{35}$ is hydrogen, lower alkoxy or lower alkylthio, characterized in that correspondingly substituted starting materials are used.

5. Process for the manufacture of compounds as in claim 4 wherein Z is

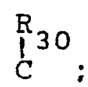

$R_{30}$ is hydrogen or halogen, $R_{31}$ is lower alkyl, $C_3$-$C_7$ cycloalkyl, halo-lower alkyl or mono-, di- or tri-halophenyl; $R_{32}$ is a piperazinyl or a pyrrolidinyl group; $R_{33}$ is halogen; $R_{34}$ is hydrogen or amino; and $R_{35}$ is hydrogen, lower alkoxy or lower alkylthio, characterized in that correspondingly substituted starting materials are used.

6. Process for the manufacture of compounds as in claim 5 wherein $R_{30}$ is hydrogen, fluorine or chlorine; $R_{31}$ is ethyl, fluoroethyl, cyclopropyl or fluorophenyl; $R_{32}$ is a piperazinyl, N-methyl piperazinyl or aminopyrrolidinyl group; $R_{33}$ is fluoro; $R_{34}$ is hydrogen; and $R_{35}$ is hydrogen, characterized in that correspondingly substituted starting materials are used.

7. Process for the manufacture of compounds according to claim 2 wherein $Q^2$ is

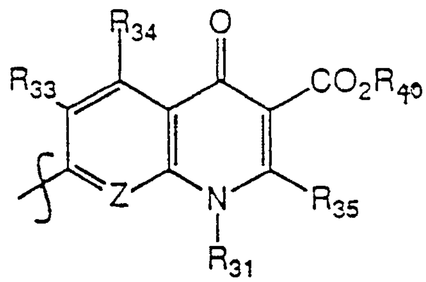

or

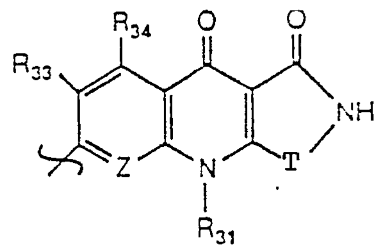

wherein Z represents

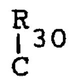

or N;

$R_{30}$ is hydrogen, halogen or a methyleneoxy ($-CH_2O-$) bridge to the piperazine nucleus of Y to form a fused six-membered ring;

$R_{31}$ represents hydrogen, lower alkyl, lower alkenyl, $C_3$-$C_7$ cycloalkyl, halo-lower-alkyl, phenyl, mono-, di- or tri-halophenyl or

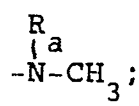

$R_a$ is hydrogen or $R_a$ and $R_{31}$ when taken together represent a lower alkylene mono-oxy group of 1-2 carbon atoms;

$R_{30}$ and $R_{31}$ when taken together represent a lower alkylene mono-oxy group of 2-3 carbon atoms;

$R_{33}$ is hydrogen or halogen;

$R_{34}$ is hydrogen or amino;

$R_{35}$ is hydrogen, lower alkoxy or lower alkylthio;

$R_{40}$ is hydrogen or a carboxylic acid protecting group; and

T is O or S, characterized in that correspondingly substituted starting materials are used.

**8.** Process for the manufacture of compounds according to claim 7 wherein Z is

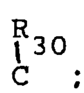

$R_{30}$ is hydrogen or halogen; $R_{31}$ is lower alkyl, $C_3$-$C_7$ cycloalkyl; halo-lower-alkyl, mono-, di- or tri-halophenyl; $R_{32}$ is a piperazinyl or pyrrolidinyl group; $R_{33}$ is halogen; $R_{34}$ is hydrogen or amino; $R_{35}$ is hydrogen; $R_{40}$ is a hydrogen or a carboxylic acid protecting group; and T is O or S, characterized in that correspondingly substituted starting materials are used.

**9.** Process for the manufacture of compounds according to claim 8 wherein Z is

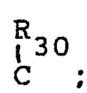

$R_{30}$ is hydrogen, fluoro or chloro; $R_{31}$ is ethyl, fluoroethyl, cyclopropyl or fluorophenyl; $R_{32}$ is a piperazinyl group, a N-methyl piperazinyl group or an aminopyrrolidinyl group; $R_{33}$ is fluoro-; $R_{34}$ is

hydrogen; $R_{35}$ is hydrogen; $R_{40}$ is hydrogen; and T is O or S, characterized in that correspondingly substituted starting materials are used.

**10.** Process for the manufacture of compounds according to claim 2 wherein $Q^3$ is

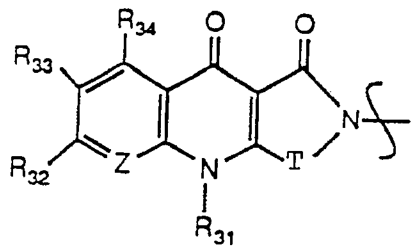

wherein Z represents

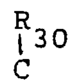

or N;
$R_{30}$ is hydrogen or halogen;
$R_{31}$ is hydrogen, lower alkyl, lower alkenyl, $C_3$-$C_7$ cycloalkyl, halo-lower-alkyl, phenyl, mono-, di- or tri-halophenyl, or

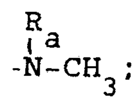

$R_a$ and $R_{30}$ when taken together represent a lower alkylene mono-oxy group of 1-2 carbon atoms;
$R_{30}$ and $R_{31}$ when taken together represent a lower alkylene mono-oxy group of 2-3 carbon atoms;
$R_{32}$ represents hydrogen, halogen, lower alkyl, a 5- or 6-membered heterocyclic ring containing one or two heteroatoms selected from the group consisting of O, N and S; when $R_{32}$ is a piperazinyl group, $R_{30}$ is hydrogen, halogen or a methyleneoxy ($-CH_2O-$) bridge to a piperazinyl nucleus of $R_{32}$ to form a fused six-membered ring;
$R_{33}$ represents hydrogen and halogen;
$R_{32}$ and $R_{33}$ when taken together represent a lower alkylene dioxy group having 1-2 carbon atoms;
$R_{34}$ is hydrogen or amino; and T is O or S, characterized in that correspondingly substituted starting materials are used.

**11.** Process for the manufacture of compounds according to claim 10 wherein Z is

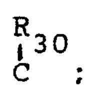

$R_{30}$ is hydrogen or halogen; $R_{31}$ is lower alkyl, $C_3$-$C_7$ cycloalkyl, halo-lower-alkyl, mono-, di or tri-phenyl; $R_{32}$ is a piperazinyl or a pyrrolidinyl group, $R_{33}$ is halogen; $R_{34}$ is hydrogen; and T is O or S, characterized in that correspondingly substituted starting materials are used.

**12.** Process for the manufacture of compounds according to claim 11 wherein $R_{30}$ is hydrogen, fluoro or chloro;
$R_{31}$ is ethyl, fluoroethyl, cyclopropyl or fluorophenyl;
$R_{32}$ is a piperazinyl, N-methyl-piperazinyl or amino pyrrolidinyl group; $R_{33}$ is fluoro; $R_{34}$ is hydrogen; and
T is O or S, characterized in that correspondingly substituted starting materials are used.

13. Process for the manufacture of compounds according to claim 1 wherein $R^1$ is hydrogen, lower alkyl, lower alkoxy or hydroxyalkyl, characterized in that correspondingly substituted starting materials are used.

14. Process for the manufacture of compounds according to claim 13 wherein $R^1$ is hydroxyalkyl, characterized in that correspondingly substituted starting materials are used.

15. Process for the manufacture of compounds according to claim 1 wherein $R^2$ and $R^3$ are each independently hydrogen or lower alkyl, characterized in that correspondingly substituted starting materials are used.

16. Process for the manufacture of compounds according to claim 15 wherein $R^2$ and $R^3$ are each hydrogen, characterized in that correspondingly substituted starting materials are used.

17. Process for the manufacture of compounds according to claim 15 wherein $R^2$ is lower alkyl and $R^3$ is hydrogen, characterized in that correspondingly substituted starting materials are used.

18. Process for the manufacture of compounds according to claim 1 having a formula

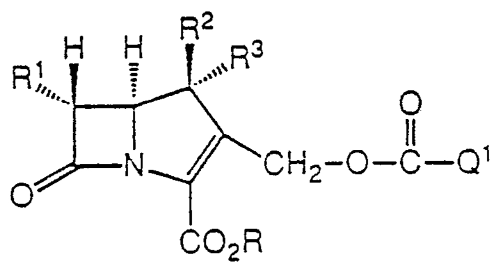

IA

wherein R is hydrogen or a carboxylic acid protecting group; $R^1$ is selected from the group consisting of hydrogen, lower alkyl, lower alkoxy and hydroxyalkyl; $R^2$ and $R^3$ are each independently hydrogen, lower alkyl, lower alkoxy or lower alkylthio and $Q^1$ is formula

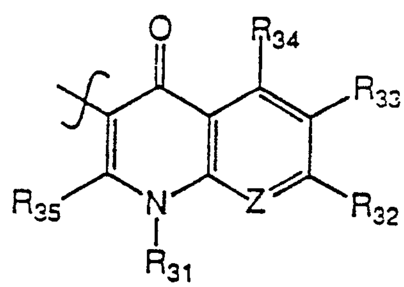

wherein Z represents

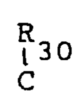

or N, $R_{30}$ is hydrogen or halogen; $R_{31}$ is hydrogen, lower alkyl, lower alkenyl, $C_3$ -$C_7$ cycloalkyl, halo-lower-alkyl, phenyl, mono-, di and tri-halophenyl, or

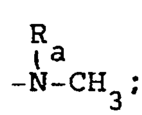

$R_a$ is hydrogen or $R_a$ and $R_{30}$ when taken together represent a lower alkylene mono-oxy group of 1-2 carbon atoms; $R_{30}$ and $R_{31}$ when taken together represent a lower alkylene mono-oxy group of 2-3 carbon atoms; $R_{32}$ represents hydrogen, halogen, lower alkyl, a 5- or 6-membered heterocyclic ring containing one or two heteroatoms selected from the group consisting of O, N and S; or when $R_{32}$ is a piperazine, $R_{30}$ is hydrogen, halogen or a methyleneoxy ($-CH_2O-$) bridge to the piperazine of $R_{32}$ to form a fused six-membered ring; $R_{33}$ is hydrogen or halogen; $R_{32}$ and $R_{33}$ when taken together represent a $C_1$-$C_2$ lower alkylene dioxy group; $R_{34}$ is hydrogen or amino; and $R_{35}$ is hydrogen, lower alkoxy or lower alkylthio, characterized in that correspondingly substituted starting materials are used.

**19.** Process for the manufacture of compounds according to claim 18 wherein Z is

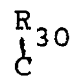 ;

$R_{30}$ is hydrogen or halogen, $R_{31}$ is lower alkyl, $C_3$-$C_7$ cycloalkyl halo-lower-alkyl or halophenyl; $R_{32}$ is a piperazinyl or pyrrolidinyl group; $R_{33}$ is halogen; and $R_{34}$ is hydrogen, characterized in that correspondingly substituted starting materials are used.

**20.** Process for the manufacture of compounds according to claim 1 of formula

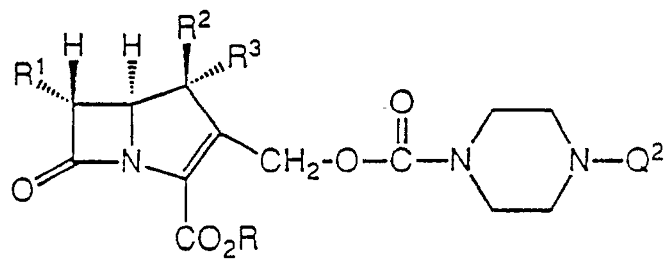

IB

wherein R is hydrogen or a carboxylic acid protecting group; $R^1$ is selected from the group consisting of hydrogen, lower alkyl, lower alkoxy and hydroxyalkyl; $R^2$ and $R^3$ are each independently hydrogen, lower alkyl, lower alkoxy or lower alkylthio and $Q^2$ is of formula

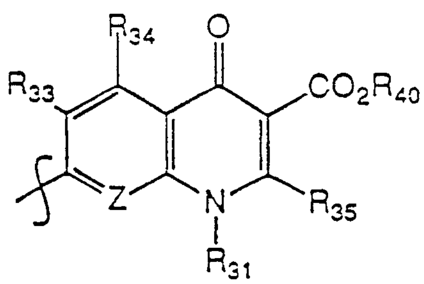

or

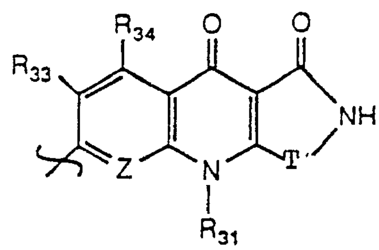

wherein Z represents

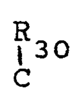

or N;

$R_{30}$ is hydrogen, halogen or a methyleneoxy ($-CH_2O-$) bridge to the piperazine nucleus of Y to form a fused six-membered ring;

$R_{31}$ represents hydrogen, lower alkyl, lower alkenyl, $C_3$-$C_7$ cycloalkyl, halo-lower-alkyl, phenyl, mono-, di-and

tri-halophenyl or

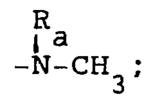

$R_a$ is hydrogen or $R_a$ and $R_{31}$ when taken together represent a lower alkylene mono-oxy group of 1-2 carbon atoms;
$R_{30}$ and $R_{31}$ when taken together represent a lower alkylene mono-oxy group of 2-3 carbon atoms;
$R_{33}$ is hydrogen or halogen;
$R_{34}$ is hydrogen or amino;
$R_{35}$ is hydrogen, lower alkoxy or lower alkylthio;
$R_{40}$ is hydrogen or a carboxylic acid protecting group; and
T is O or S, characterized in that correspondingly substituted starting materials are used.

**21.** Process for the manufacture of compounds according to claim 1 of formula

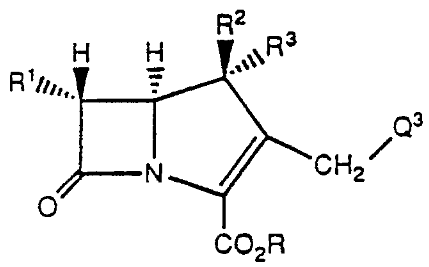

IC

wherein R is hydrogen or a carboxylic acid protecting group; $R^1$ is selected from the group consisting of hydrogen, lower alkyl, lower alkoxy and hydroxyalkyl; $R^2$ and $R^3$ are each independently hydrogen, lower alkyl, lower alkoxy or lower alkylthio and $Q^3$ is of formula

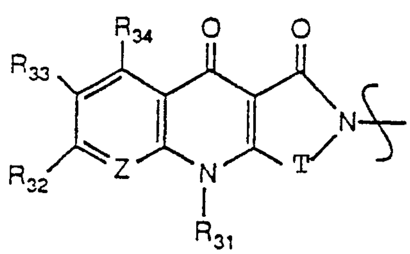

wherein Z represents

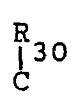

or N;
$R_{30}$ is hydrogen or halogen;
$R_{31}$ is hydrogen, lower alkyl, lower alkenyl, $C_3$-$C_7$ cycloalkyl, halo-lower-alkyl, phenyl, mono-, di- or trihalophenyl, or

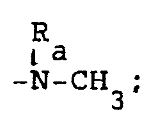

$R_a$ and $R_{30}$ when taken together represent a lower alkylene mono-oxy group of 1-2 carbon atoms;
$R_{30}$ and $R_{31}$ when taken together represent a lower alkylene mono-oxy group of 2-3 carbon atoms;

$R_{32}$ represents hydrogen, halogen, lower alkyl, a 5- or 6-membered heterocyclic ring containing one or two heteroatoms selected from the group consisting of O, N and S;
when $R_{32}$ is a piperazinyl group, $R_{30}$ is hydrogen, halogen or a methyleneoxy ($-CH_2O-$) bridge to a piperazinyl nucleus of $R_{32}$ to form a fused six-membered ring;
$R_{33}$ represents hydrogen and halogen;
$R_{32}$ and $R_{33}$ when taken together represent a lower alkylene dioxy group having 1-2 carbon atoms;
$R_{34}$ is hydrogen or amino; and T is O or S, characterized in that correspondingly substituted starting materials are used.

**22.** Process for the manufacture of compounds according to claim 21 wherein Z is

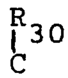 ;

$R_{30}$ is hydrogen or halogen; $R_{31}$ is lower alkyl, $C_3$-$C_7$ cycloalkyl, halo-lower-alkyl or mono-, di- or trihalo-phenyl; $R_{32}$ is a piperazinyl or a pyrrolidinyl group, $R_{33}$ is halogen; $R_{34}$ is hydrogen; and T is O or S, characterized in that correspondingly substituted starting materials are used.

**23.** Process for the manufacture of compounds according to claim 22, rac-[4S,5R,6S(R)]-3-[[9-cyclopropyl-6-fluoro-7-(1-piperazinyl)-2,3,4,9-tetrahydro-3,4-dioxoisothiazolo [5,4-b]quinolin-2-yl)]methyl]-6-(1-hydroxyethyl)-4-methyl-7-oxo-1-azabicyclo[3.2.0]hept-2-ene-2-carboxylic acid and its salts and hydrates, characterized in that correspondingly substituted starting materials are used.

**24.** Process for the manufacture of compounds according to claim 1, rac-[5R,6S(R)]-3-[[[[6,8-difluoro-1-(2-fluoroethyl)-1,4-dihydro-4-oxo-7-(4-methyl-1-piperazinyl)-3-quinolonyl]carbonyl]oxy]methyl]-6-(1-hydroxyethyl)-7-oxo-1-azabicyclo-[3.2.0]hept-2-ene-2-carboxylic acid and its salts and hydrates characterized in that correspondingly substituted starting materials are used.

**25.** Process for the manufacture of compounds according to claim 1, rac-[5R,6S(R)]-3-[[[[(3-carboxy-1-cyclopropyl-6-fluoro-1,4-dihydro-4-oxo-7-quinolonyl)-1-piperazinyl]carbonyl] oxy]methyl]-6-(1-hydroxyethyl)-7-oxo-1-azabicyclo[3.2.0]hept-2-ene-2-carboxylic acid and its salts and hydrates characterized in that correspondingly substituted starting materials are used.

**26.** Process for the manufacture of compounds according to claim 1 selected from the group consisting of

rac-[4S,5S,6R]-3-[[[[1-cyclopropyl-6-fluoro-1,4-dihydro-4-oxo-7-(1-piperazinyl)-3-quinolonyl]carbonyl]-oxy]methyl]-6-ethyl-4-thiomethyl-7-oxo-1-azabicyclo[3.2.0]hept-2-ene-2-carboxylic acid

rac-3-[[[[7-(3-amino-1-pyrrolidinyl)-1-cyclopropyl-6,8-difluoro-1,4-dihydro-4-oxo-3-quinolonyl]carbonyl]-oxy]methyl]-4,4-dimethyl-7-oxo-1-azabicyclo[3.2.0]hept-2-ene-2-carboxylic acid

rac-[4S,5R,6R]-3-[[[[1-cyclopropyl-6,8-difluoro-1,4-dihydro-4-oxo-7-(4-pyridinyl)-3-quinolonyl]carbonyl]-oxy]methyl]-4,6-dimethoxy-7-oxo-1-azabicyclo[3.2.0]hept-2-ene-2-carboxylic acid

rac-[4S,5R,6S(R)]-3-[[[[5-amino-7-(3-amino-1-pyrrolidinyl)-1-cyclopropyl-6,8-difluoro-1,4-dihydro-4-oxo-3-quinolonyl]carbonyl]oxy]methyl]-6-(1-hydroxyethyl)-4-methyl-7-oxo-1-azabicyclo[3.2.0]hept-2-ene-2-carboxylic acid

rac-[4S,5R,6S(R)]-3-[[[[10-(3-amino-1-pyrrolindinyl)-9-fluoro-3,7-dihydro-3-methyl-7-oxo-2H-pyrido[3,2,1-ij][4,1,2]benzoxadiazin-6-yl]carbonyl]oxy]methyl]-6-(1-hydroxyethyl)-4-methyl-7-oxo-1-azabicyclo[3.2.0]-hept-2-ene-2-carboxylic acid

rac-[4S,5R,6S(R)]-3-[[[[6-fluoro-1-ethyl-1,4-dihydro-4-oxo-7-(1-piperazinyl)-1,8-naphthridin-3-yl]carbonyl]-oxy]methyl]-6-(1-hydroxyethyl)-4-methyl-7-oxo-1-azabicyclo[3.2.0]hept-2-ene-2-carboxylic acid

rac-[4S,5R,6S(R)]-3-[[[[9-fluoro-10-(4-methyl-1-piperazinyl)-3,7-dihydro-3-methyl-7-oxo-2H-pyrido[1,2,3-de]-1,4-benzoxazin-6-yl]carbonyl]oxy]methyl]-6-(1-hydroxyethyl)-4-methyl-7-oxo-1-azabicyclo[3.2.0]-

hept-2-ene-2-carboxylic acid

rac-[4S,5S,6R]-3-[[[[[(3-carboxy-1-cyclopropyl-6-fluoro-1,4-dihydro-4-oxo-7-quinolonyl)-3-pyrrolidinyl]-amino]carbonyl]oxy] methyl]-6-ethyl-4-thiomethyl-7-oxo-1-azabicyclo[3.2.0]hept-2-ene-2-carboxylic acid

rac-3-[[[[[(3-carboxy-1-cyclopropyl-6,8-difluoro-1,4-dihydro-4-oxo-7-quinolonyl)-3-pyrrolidinyl]amino]-carbonyl]oxy]methyl]-4-dimethyl-7-oxo-1-azabicyclo[3.2.0]hept-2-ene-2-carboxylic acid

rac-[4S,5R,6R]-3-[[[[(3-carboxy-1-ethyl-6-fluoro-1,4-dihydro-4-oxo-1,8-naphthyridin-7-yl)-1-piperazinyl]-carbonyl]oxy]-methyl]-4,6-dimethoxy-7-oxo-1-azabicyclo[3.2.0]hept-2-ene-2-carboxylic acid

rac-[4S,5R,6S(R)]-3-[[[[(3-carboxy-1-cyclopropyl-6-fluoro-1,4-dihydro-4-oxo-7-quinolonyl)-1-piperazinyl]-carbonyl]oxy]methyl]-6-(1-hydroxyethyl)-4-methyl-7-oxo-1-azabicyclo-[3.2.0]hept-2-ene-2-carboxylic acid

rac-[4S,5R,6S(R)]-3-[[[[(9-cyclopropyl-6-fluoro-2,3,4,9-tetrahydro-3,4-dioxoisothiazolo[5,4-b]quinolin-7-yl)-1-piperazinyl]carbonyl]oxy]methyl]-6-(1-hydroxyethyl)-4-methyl-7-oxo-1-azabicyclo[3.2.0]hept-2-ene-2-carboxylic acid

rac-[4S,5R,6S(R)]-3-[[[[[[9-fluoro-3,7-dihydro-3-methyl-7-oxo-2H-pyrido[3,2,1-ij][4,1,2]benzoxadiazin-10-yl]-3-pyrrolidinyl] amino]carbonyl]oxy]methyl]6-(1-hydroxyethyl)-4-methyl-7-oxo-1-azabicyclo[3.2.0]hept-2-ene-2-carboxylic acid

rac-[4S,5R,6S(R)]-3-[[[[[(5-amino-3-carboxy-1-cyclopropyl-6,8-difluoro-1,4-dihydro-4-oxo-7-quinolonyl)-3-pyrrolidinyl]amino] carbonyl]oxy]methyl]-6-(1-hydroxyethyl)-4-methyl-7-oxo-1-azabicyclo[3.2.0]hept-2-ene-2-carboxylic acid

rac-[4S,5R,6S(R)]-3-[[[[[(3-carboxy-1-(2,4-difluorophenyl)-6-fluoro-1,4-dihydro-4-oxo-1,8-naphthyridin-7-yl)-3-pyrrolidinyl]amino]carbonyl]oxy]methyl]-6-[(1-hydroxyethyl)-4-methyl-7-oxo-1-azabicyclo[3.2.0]-hept-2-ene-2-carboxylic acid

rac-[4S,5R,6S(R)]-3[[[[6,8-difluoro-1-(2-fluoroethyl)-1,4-dihydro-4-oxo-7-(4-methyl-1-piperazinyl)-3-quinolonyl]carbonyl]thio]methyl]-6-(1-hydroxyethyl)-4-methyl-7-oxo-1-azabicyclo[3.2.0]hept-2-ene-2-carboxylic acid

rac-[4S,5R,6S(R)]-3[[(3-carboxy-1-cyclopropyl-6-fluoro-1,4-dihydro-4-oxo-7-quinolonyl)-1-piperazinyl]-methyl]-6-(1-hydroxyethyl)-4-methyl-7-oxo-1-azabicyclo[3.2.0]hept-2-ene-2-carboxylic acid

rac-[4S,5R,6S(R)]-4-[3-carboxy-1-cyclopropyl-6-fluoro-1,4-dihydro-4-oxo-7-quinolonyl]-1-[2-carboxy-6-(1-hydroxyethyl)-4-methyl-7-oxo-1-azabicyclo[3.2.0]hept-2-enyl]methyl-1-methylpiperazinium iodide

rac-[4S]-3-[[[[3-carboxy-1-cyclopropyl-6-fluoro-1,4,8,9,10,11, 11a,12-octahydro-4-oxo-pyrazino[1',2':4,5]-[1,4]oxazino[3,2-h]-quinolin-10-yl]carbonyl]oxy]methyl]-4-methyl-7-oxo-1-azabicyclo[3.2.0]hept-2-ene-2-carboxylic acid

rac-[6R]-3-[[3-carboxy-1-cyclopropyl-6-fluoro-1,4,8,9,10,11, 11a,12-octahydro-4-oxo-pyrazino[1',2':4,5]-[1,4]oxazino[3,2-h]-quinolin-10-yl]methyl]-7-oxo-4,4,6-trimethyl-1-azabicyclo-[3.2.0]hept-2-ene-2-carboxylic acid

rac-[4S,5R,6S(R)]-10-[2-carboxy-6-(1-hydroxyethyl)-4-methyl-7-oxo-1-azabicyclo[3.2.0]hept-2-en-3-yl]-methyl-10-methyl-[3-carboxy-1-cyclopropyl-6-fluoro-1,4,8,9,10,11,11a,12-octahydro-4-oxo-pyrazino-[1',2':4,5][1,4]oxazino[3,2-h]quinolinium] iodide
and their salts and hydrates, characterized in that correspondingly substituted starting materials are used.

**27.** Process for the manufacture of a compound according to claim 1 selected from the group consisting of rac-[4S,5R,6S(R)]-3[[[[6,8-difluoro-1-(2-fluoroethyl)-1,4-dihydro-4-oxo-7-(4-methyl-1-piperazinyl)-3-quinolonyl]carbonyl]oxy]methyl]-6-(1-hydroxyethyl)-4-methyl-7-oxo-1-azabicyclo-[3.2.0]hept-2-ene-2-carboxylic acid

rac-[4S,5R,6S(R)]-3-[[[[1-cyclopropyl-6-fluoro-1,4-dihydro-4-oxo-7-(1-piperazinyl)-3-quinolonyl]carbonyl]-oxy]methyl]-6-(1-hydroxy-ethyl)-4-methyl-7-oxo-1-azabicyclo[3.2.0]hept-2-ene-2-carboxylic acid

rac-[4S,5R,6S(R)]-3-[[[[(3-carboxy-1-cyclopropyl-6-fluoro-1,4-dihydro-4-oxo-7-quinolonyl)-1-piperazinyl]-carbonyl]oxy]methyl]-6-(1-hydroxyethyl)-4-methyl-7-oxo-1-azabioyclo-[3.2.0]hept-2-ene-2-carboxylic acid

rac-[4S,5R,6S(R)]-3-[[[[[(3-carboxy-1-cyclopropyl-6,8-difluoro-1,4-dihydro-4-oxo-7-quinolonyl)-3-pyrrolidinyl]amino]carbonyl]oxy]methyl]-6-(1-hydroxyethyl)-4-methyl-7-oxo-1-azabicyclo[3.2.0]hept-2-ene-2-carboxylic acid,
and their salts and hydrates, characterized in that correspondingly substituted starting materials are used.

**28.** Process for the manufacture of
rac-[5R,6S(R)]-3-[[[[6,8-difluoro-1-(2-fluoroethyl)-1,4-dihydro-7-(4-methyl)-1-piperazinyl)-4-oxo-3-quinolonyl]carbonyl]oxy]methyl]-6-(1-hydroxyethyl)-7-oxo-1-azabicyclo-[3.2.0]hept-2-ene-2-carboxylic acid (2,2-dimethyl-1-oxopropoxy)methyl ester, characterized in that correspondingly substituted starting materials are used.

**29.** Process for the manufacture of
rac-[5R,6S(R)]-3-[[[(3-carboxy-1-cyclopropyl-6-fluoro-1,4-dihydro-4-oxo-7-quinolonyl)-1-piperazinyl]-carbonyl] oxy]methyl-6-[1-hydroxyethyl]-7-oxo-1-azabicyclo[3.2.0]hept-2-ene-2-carboxylic acid-(2,2-dimethyl-1-oxopropoxy)methyl ester, characterized in that correspondingly substituted starting materials are used.

**30.** A process for the manufacture of pharmaceutical preparations, which process comprises mixing a compound of formula I as defined in claim 1 or a readily hydrolyzable ester or salt of such a compound or a hydrate of a compound of formula I or or an ester or salt thereof, as active ingredient, with nontoxic, inert, therapeutically compatible solid or liquid carriers and/or excipients commonly used in such preparations.

**31.** The use of the end products defined in any one of claims 1-29 in the treatment or prophylaxis of illnesses.

**32.** The use of the end products defined in any one of claims 1-29 in the treatment or prophylaxis of infectious diseases.

**33.** The use of the end products defined in any one of claims 1-29 for the manufacture of medicaments for the treatment or prophylaxis of infectious diseases.

European Patent Office

# PARTIAL EUROPEAN SEARCH REPORT

which under Rule 45 of the European Patent Convention shall be considered, for the purposes of subsequent proceedings, as the European search report

Application number
EP 91105550
- page 1 -

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP-A-0366641 (NORWICH EATON PHARMACEUTICALS, INC.) * page 52, line 49 - page 54, line 14; claims 1-3,11-13 * | 1-6, 18,19, 30-33, 37 | C07D519/00 C07D477/00 A61K31/435 //(C07D519/00, 498:00,477:00) (C07D519/00, 477:00,471:00) (C07D519/00, 495:00,477:00) (C07D519/00, 513:00,477:00) |
| P,X | EP-A-0366193 (NORWICH EATON PHARMACEUTICALS, INC.) * page 69, lines 40-46; page 112, lines 46-55; claims 1-3,12-14 * | 1,2, 4-6, 18,19, 30-33 37 | |
| P,X | EP-A-0366189 (NORWICH EATON PHARMACEUTICALS, INC.) * page 77, lines 24-45; page 148, line 34 - page 149, line 39 * | 1-3, 7-9, 20, 30-33 37 | |
| | ... | | TECHNICAL FIELDS SEARCHED (Int. Cl.5) C07D477/00 C07D519/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-34,37
Claims searched incompletely: -
Claims not searched: 35,36
Reason for the limitation of the search:

Article 52(4) EPC.

| Place of search | Date of completion of the search | Examiner |
|---|---|---|
| Berlin | 04.07.1991 | C.V.F. HASS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# PARTIAL EUROPEAN SEARCH REPORT

Application number
EP 91105550
- page 2 -

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | GB-A-2092147 (F. HOFFMANN-LA ROCHE AND CO. AG)<br>* pages 24-30; claims 1,15-19 * | 1, 30-34, 37 | |
| A | EP-A-0185315 (MERCK AND CO., INC.)<br>* claims 1,2,6; examples 4-6 * | 1, 30-33, 37 | |
| A,D | US-A-4767762 (D.T. CHU)<br>* claims 1,8,9 * | 1,7,10, 18, 30-33, 37 | |

TECHNICAL FIELDS SEARCHED (Int. Cl.5)